(19) **Europäisches Patentamt / European Patent Office / Office européen des brevets**

(11) **EP 3 600 668 B1**

(12) **EUROPEAN PATENT SPECIFICATION**

(45) Date of publication and mention
of the grant of the patent:
**26.01.2022 Bulletin 2022/04**

(21) Application number: **18732169.0**

(22) Date of filing: **20.03.2018**

(51) International Patent Classification (IPC):
***C12M 1/12*** *(2006.01)*

(52) Cooperative Patent Classification (CPC):
**B01L 3/502715; B01L 3/50273; B01L 3/502738;
C12M 23/04; C12M 23/16; C12M 23/42;
C12M 25/04; F04B 43/043; F04B 43/14;
G01F 23/263;** B01J 2219/00306; B01J 2219/00355;
B01J 2219/00479; B01L 2200/0621;
B01L 2300/0829; (Cont.)

(86) International application number:
**PCT/US2018/023411**

(87) International publication number:
**WO 2018/175478 (27.09.2018 Gazette 2018/39)**

(54) **MODULAR ORGAN MICROPHYSIOLOGICAL SYSTEM WITH MICROBIOME**

MODULARES MIKROPHYSIOLOGISCHES ORGANSYSTEM MIT MIKROBIOM

SYSTÈME MICROPHYSIOLOGIQUE D'ORGANE MODULAIRE AVEC MICROBIOME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.03.2017 US 201762474337 P**
**11.09.2017 US 201762556595 P**

(43) Date of publication of application:
**05.02.2020 Bulletin 2020/06**

(73) Proprietor: **Massachusetts Institute of Technology**
**Cambridge, MA 02139 (US)**

(72) Inventors:
• **GRIFFITH, Linda, G.**
**Cambridge, MA 02139 (US)**
• **TRUMPER, David, L.**
**Plaistow, NH 03865 (US)**
• **EDINGTON, Collin**
**San Mateo, CA 94401 (US)**
• **ROHATGI, Gaurav**
**Boston, MA 02210 (US)**
• **FREAKE, Duncan**
**Boston, MA 02210 (US)**
• **SOENKSEN, Luis**
**Boston, MA 02215 (US)**
• **KASSIS, Timothy**
**Malden, MA 02148 (US)**
• **BHUSHAN, Brij, Mohan**
**Cambridge, MA 02139 (US)**

(74) Representative: **Hoffmann Eitle**
**Patent- und Rechtsanwälte PartmbB**
**Arabellastraße 30**
**81925 München (DE)**

(56) References cited:
**US-A1- 2005 260 745     US-A1- 2010 230 613
US-A1- 2013 068 310**

• **KAREL DOMANSKY ET AL: "Perfused multiwell
plate for 3D liver tissue engineering", LAB ON A
CHIP, vol. 10, no. 1, 1 January 2010 (2010-01-01),
pages 51-58, XP055174411, ISSN: 1473-0197,
DOI: 10.1039/B913221J**

**(Cont. next page)**

(52) Cooperative Patent Classification (CPC): (Cont.)
B01L 2300/0887; B01L 2400/0406;
B01L 2400/0457; B01L 2400/0487;
B01L 2400/0655; B01L 2400/086; F04B 43/12

## Description

This invention was made with government support under Contracts W911NF-12-2-0039 and UH3TR000496 awarded by the Defense Advanced Research Projects Agency Microphysiological Systems Program and National Institutes of Health, respectively. The government has certain rights in the invention.

## BACKGROUND OF THE INVENTION

The content of each of US 2005/260745 A1, US 2010/230613 A1 and US 2013/068310 A1 forms part of the state of the art.

Improving the effectiveness of preclinical predictions of human drug responses is critical to reducing costly failures in clinical trials. Complex diseases often arise from dysregulation of systemic regulatory networks, including across multiple organs, resulting from integration of local and systemic perturbations. Incomplete understanding of inter-tissue communication can undermine the accurate diagnosis and treatment of disease conditions. Although the study of human pathophysiology has relied on genetically tractable animal models such as murine models, these animal models may be inadequate for recapitulating polygenic and multifactorial human diseases with diverse clinical phenotypes.

Recent advances in cell biology, microfabrication and microfluidics have enabled the development of micro engineered models of the functional units of human organsknown as organs -on- a-chip (OCC)- that could provide the basis for preclinical assays with greater predictive power. For example, U.S. Patent No. 6,197,575 to Griffith, et al., describes a micromatrix and a perfusion assembly suitable for seeding, attachment, and culture of complex hierarchical tissue or organ structures. U.S. Patent No. 8,318,479 to Inman, et al., describes a system that facilitates perfusion at the length scale of a capillary bed suitable for culture and assaying in a multiwell plate format.

These platforms, termed microphysiological systems (MPSs), are designed to mimic physiological functions by integrating tissue engineering principles with microfabrication or micromachining techniques for recapitulating 3D multicellular interactions and dynamic regulation of nutrient transport and/or mechanical stimulation (Huh D, et al., Lab Chip, 12(12):2156-2164 (2012); Sung JH, et al. Lab Chip 13(7):1201-1212 (2013); Wikswo JP, et al., Exp Biol Med (Maywood) 239(9):1061-1072 (2014); Livingston CA, et al., Computational and Structural Biotechnology Journal 14:207-210 (2016); Yu J, et al., Drug Discovery Today, 19(10):1587-1594 (2014); Zhu L, et al. Lab Chip, 16(20):3898-3908 (2016)). While significant advances have been made in the development of individual MPS (e.g., cardiac, lung, liver, brain) (Roth A, et al., Adv Drug Deliver Rev, 69-70:179-189 (2014); Huebsch N, et al. Scientific Reports, 6:24726 (2016); Domansky K, et al. Lab Chip 10(1):51-58 (2010)), efforts towards the interconnection of MPS are still in their infancy, with most studies primarily focused on basic viability and toxicity demonstrations (Oleaga C, et al. Sci Rep 6:20030 (2016); Esch MB, et al., Lab Chip 14(16):3081-3092 (2014); Maschmeyer I, et al., Lab Chip 15(12):2688-2699 (2015); Materne EM, et al. J Biotechnol 205:36-46 (2015); Loskill P, et al., Plos One 10(10):e0139587 (2015)). However, lack of clinical efficacy, rather than toxicity, was identified as the leading cause of drug attrition in Phase II and III clinical trials (the most costly stage) (Kubinyi H, Nat Rev Drug Discov 2(8):665-668 (2003); Cook D, et al. Nat Rev Drug Discov 13(6):419-431 (2014); Denayer T, et al., New Horizons in Translational Medicine, 2(1):5-11 (2014)). Major contributing factors include incomplete understanding of disease mechanisms, the lack of predictive biomarkers, and interspecies differences. There is an urgent unmet need in drug development due to the need for humanized model systems for target identification/validation and biomarker discovery.

The increasing need for more predictive *in vitro* systems is not limited to single MPS technologies. The complexity of the human physiology can be better recapitulated at a systemic level in multi-MPS platforms, where multi-organ crosstalk and the physiological responses to therapeutic agents and toxins occur via surrogate signals (e.g. chemokines, cytokines, growth factors) and circulating cells (e.g. immune cells). Shuler et al. demonstrated pharmacological applications of multi-compartmental bioreactor systems (Sweeney LM, et al., Toxicol. Vitr. 9, 307-316 (1995)). Sung et al. showed a micro cell culture analog ($\mu$CCA), where cells were embedded in 3D hydrogels in separate chambers, could be used for interacting MPS systems (Sung JH, et al., Lab Chip 9, 1385 (2009)). Some prototypes use gravitational flow for inter-MPS communication (Sung JH, et al., Lab Chip 10, 446-455 (2010)). Some prototypes of the three-MPS system use off-platform pumping with a bubble trap (Sung JH, et al., Lab Chip 9, 1385 (2009); Esch MB, et al. Lab Chip 14, 3081 (2014)).

While toxicology and pharmacodynamic studies are common applications, pharmacokinetic studies have been limited in multi-MPS platforms. Moreover, current multi-MPS systems generally employ a closed format associated with traditional microfluidic chips for operating with very small fluid volumes (Anna SL, Annu. Rev. Fluid Mech. 48, 285-309 (2016)). Current fabrication processes for these systems require the use of castable elastomeric polymers like PDMS mainly for desirable optical properties, but due to fluid-surface interactions such as drug and growth factor adsorption are commonly present (Halldorsson S, et al., Biosens. Bioelectron. 63, 218-231 (2015)).

Other practical limitations in the design and fabrication of the hardware also significantly reduce the robustness, long-term reliability, and compatibility of customization in existing multi-MPS devices. Poor hardware designs and constructs often result in a poor of lack of control on the directionality of fluid among wells (inter-well directionality) and within-well recirculation, leaving some wells dry due to breakage of fluid flow, the syphoning effect, and/or evaporation. Media depletion and waste removal at near-physiological scales often require single-pass media flow, making it difficult or impossible to study slow-clearing drugs, effects of drug metabolites, and inter-MPS communications. Removable inserts to fit into the wells of multi-MPS devices may be desirable in culturing some tissues, but their compatibility with fluid in-flow to support perfusion of cultures has been difficult to achieve.

Non-contact fluid level-sensing techniques have been reported in monitoring large volumes of hazardous or sensitive fluids in industrial applications. These non-contact fluid level sensors measure height changes in macrofluidic applications in the order of centimeters, using techniques such as direct visualization of fluid height (e.g. optical monitoring through translucent window), time-of-flight (TOF) measurements (e.g. ultrasonic, laser, and radar transmitters), and capacitive fluid sensing (U.S. Patent No. 5,042,299). However, accurately and continuously measuring small fluid height changes in microfluidic and mesofluidic systems is still very challenging, particularly to capture height changes in the order of millimeters or micrometers over an extended period of hours, days, and weeks. This limitation in fluid sensing at the small scale, in turn, has hindered the development of closed-loop micro- and mesoscale fluidic systems.

One application of these systems is in creating an *in vitro* organmicrobiome co-culture for testing of compounds not just on organs but on organ systems and integrated multi-organ systems.

It is therefore an object of the present invention to provide organ systems for co-culture of an *in vitro* microbiome component for testing of compounds not just on organs but on integrated organ systems with microbial populations.

It is another object of the present invention to provide a robust, sensitive, and scalable sensor for non-contact fluid level detection for smallscale changes for an extended period of time.

## SUMMARY OF THE INVENTION

The invention is as defined in the claims hereof.

Multi-well cell culture systems (or organs-on-a-chip devices, microphysiosome bioreactors) are provided with integrated pumping, spontaneous liquid leveling, and programmable drug/media dosing. A multiwell culture system, i.e., a chip or a bioreactor, contains at least three layers of constructs, which from top to bottom are (1) a multi-well cell culture plate construct with built-in fluid channels (e.g., fluid paths) below and connected to the wells, (2) a barrier membrane as a pump actuator, and (3) a pneumatic plate to apply pressure and vacuum. The multi-well culture system also includes (4) an apical flow module. The apical flow module, when combined with the multi-well culture system that provides basal recirculation, may be used to establish gradients for oxygen, nutrients, and therapeutic, prophylatic and diagnostic agents available to the multiple modular organ models. The apical module may also be used to introduce and maintain a gradient of microbial metabolites or microbes themselves. These microbes can be a single strain of interest or complex populations, for example, obtained from human patients or from established cell cultures or microbiomes such as large or small intestine, vagina, nasal or oral cavities. The module interfaces with the basal flow system through integrated fixing features, such as screws, magnets, clasps, or other reversible fixing methods. Flow to the apical module can be provided by commercial pumps or by an isolated flow loop built into the base system, taking advantage of the existing pumping architecture. Real-time monitoring of optical microbial density in the effluent can be used to adjust the flow rate, maintaining a desired concentration despite continuous growth.

In different embodiments, the membrane layer is bonded on either the fluidic or the pneumatic side, or is a separate component. Bonding the membrane layer to the pneumatic or fluidic side enhances reliability and reduces manufacture time and cost. In a preferred embodiment, the membrane is bonded to the pneumatic side, and the fluidic layer is open faced, making cleaning and sterilization easier. In some embodiments, no bonding on the fluidic side eliminates delamination.

Pneumatic control of vacuum or pressure causes the membrane to actuate, which acts like a valve to control the passage or blockade on the fluid channel, and thus the fluid flow, on the fluidic side of the system. Fluid such as cell culture media is flowed in to fill at least one of the wells, and passive self-leveling spillways connecting two or more wells in the upper space allow for transfer of excess fluid from one well to another. Recirculation within a well or between two wells is allowed actively, through additional pumps.

The system combines one or more of the following features to improve the operability and performance of modeled organs on a chip, such as spillways having defined geometric arrangements to promote unidirectional flow and anti-siphon capability. One or more features in the entry, the conduit, and/or the exit of the spillway are provided to ensure spontaneous capillary flow across the spillway for unidirectional self-leveling of fluid amount in MPS chambers. Some embodiments provide entry geometry that eliminates a step or V-cut to minimize fluid film disruption; and includes a radial meniscus pinning groove around the source well, the groove being able to "pin" the fluid meniscus, making a specified fluid height energetically favorable.

Some embodiments provide a spillway conduit that has a small-width (e.g., less than 3 mm), high aspect ratio groove at the bottom along the conduit to permit spontaneous capillary flow, thus leveling of excess fluid from the source well to the destination well. Some embodiments provide exit geometry where the groove at the end of the spillway conduit encounters an enlarged, curved area, to thin the fluid film, thereby breaking it into drops which coalesce and fall due to gravity. In another embodiment, at the exit of spillway there is a vertical groove along the wall and toward the bottom of the destination well. Some embodiments additionally provide an undercut into the wall of the destination well, where the cut is at some distance below the exit of the conduit, to prevent back flow due to siphoning effect. These features allow a self-leveling spillway in a unidirectional flow and prevent breakage of flow and over accumulation in the source well or the conduit.

Optionally coupled with an internal humidity reservoir or an evaporation-combatting moat, the multi-organ MPS platforms allow for long-term culture of functional organ-like tissues, e.g., for at least 1, 2, 3, 4, 5, 6 weeks or at least 1, 2, 3 months.

The on-board pumping system (e.g., built-in fluid pumping channels) eliminates the need for tubing. Modular pumping can be configured to drive external flows. Ferrule connections may be used to interface the built-in pump with external tubing, allowing for a pumping manifold to drive a large number of flows simultaneously in a compact package.

A dual pumping system in addition to single multi-chamber unit pumping system permits not only pulsating flow but also a smooth flow volume profile. A triple pumping system or more parallel channels may further increase the smoothness of the flow.

A removable yet perfusion-enabled scaffold to fit into the wells on the platform is provided. Unlike conventional removable inserts that do not allow integrable features to participate in the perfusion process in a bioreactor, the scaffold enables cell culture to be perfused on-platform and processed off-platform. The scaffold may optionally contain a fluid aggregation lid for non-contact oxygen ($O_2$) sensing.

One or more means for non-contact fluid leveling sensing are provided. Capacitors with a symmetrical, front-and-back electrode design provides accurate measurement of fluid level in a well from within the wall of the well, avoiding direct contact, electrochemical reactions, and potential contamination.

A closed-loop feedback control system for micro and/or mesofluidic systems through automated fluid height sensing is described. The system permits fully controllable gravity-driven pumps including at least one supply reservoir and a sensor to accurately measure fluid height and its changes (e.g., as small as in the centimeter, millimeter, or micron scale) without direct fluid contact. As fluid flows from the outlet of the supply reservoir to a connecting microfluidic channel, well, or device, the fluid level in the reservoir decreases. The sensor detects the fluid height changes in the reservoir and the signal is processed through a computer processing unit (CPU) or a microcontroller unit (MCU) to direct replenishment (or in some circumstances reduction) of fluid in the supply reservoir to a previous level or a user input level. The fluid to replenish the supply reservoir may be withdrawn from an auxiliary, additional reservoir (as opposed to the gravity-driven, fluid supply reservoir above). The fluid through the microfluidic channel, well, or device may be exposed to open air pressure or recirculated to the auxiliary reservoir for the system. Generally, the fluid to be handled by the closed-loop feedback controlled system refers to a liquid or a mixture of liquids such as cell culture fluid. Air can act as a fluid in some cases. The fluid height sensor for accurate measurement of fluid height changes at the meso- or microscale is applicable to both open reservoirs and closed reservoirs, as long as there is an interface between fluids, such as a liquid/air interface or a liquid/liquid interface, where one of the fluids has a dielectric constant at least one order of magnitude higher than that of the other fluid.

In some embodiments, the gravity-driven pump in the system contains one fluid supply reservoir, which generally supports unidirectional fluid flow from the reservoir into a fluidic channel or well that is connected to the outlet of the supply reservoir. In other embodiments, the gravity-driven pump in the system contains two fluid supply reservoirs, and a fluidic channel, well, or device situated in between the two fluid supply reservoirs and in fluidic communication therewith. With two fluid supply reservoirs, a bidirectional fluid flow is permitted from a first reservoir through the microfluidic channel, well, or device to a second reservoir, as well as from the second reservoir through the microfluidic channel, well, or device to the first reservoir. In another embodiment, multiple (e.g., more than two) fluid supply reservoirs may be fluidically connected (e.g., in tandem) to support fluid flow through one or more microfluidic channel, well, or device. In yet another embodiment, two or more gravity-driven pumps are included in one system (e.g., in parallel or in tandem), each pump containing one or more fluid supply reservoirs, to direct fluid flow through two or more microfluidic channels, wells, devices, or a combination thereof.

The system allows for independent control of the hydrostatic pressure of the fluid supply reservoir and the flow rate of fluid dispensed from the fluid supply reservoir. In other words, the flow rate of fluid into a connected microfluidic channel, well, or device can be maintained at a controllable level, without the risk of unwanted reduced flow rate due to the decrease of fluid level in the supply reservoir. The system supports unidirectional or bidirectional, adjustable, pulseless, continuous, and steady-state flow, which can be further connected to a microfluidic channel, well, or device of choice, for various fluidic applications.

Exemplary microfluidic applications of the system include *in vitro* controlled fluid/medium supply in biological research to mimic physiological or pathological environment *in vivo.* For example, cell or tissue culture vehicles are connected to the system supplying controlled medium flow rates to mimic angiogenesis *in vivo.* It also can be used to direct fluid flow in a device of choice for *in vitro* testing of pharmaceuticals, organ-on-chip applications, as well as cell sorting.

Preferably, the sensor for accurate measurement of fluid height and its changes is a capacitive fluid level sensor. The capacitive fluid level sensor contains a specific configuration of electrodes, having a primary sensing arrangement of three electrodes and a parallel, self-shielding arrangement of three electrodes. In the primary sensing arrangement, three inter-digitating electrodes, designated for excitation, for sensing, and for excitation, are arranged to be coplanar with the sensing electrode in the center and the two excitation electrodes on the sides, and facing (electromagnetic fringe penetrating) the fluid to be measured. In the parallel, back arrangement, three electrodes are also coplanar, forming an excitation-sensing-excitation (ESE) arrangement, which mirrors and symmetrically aligns with the three electrodes in the primary sensing arrangement. The electromagnetic fringing of this electrode configuration supports a self-shielding capability where parasitic capacitances running through the thin inter-electrode dielectric are minimized or entirely eliminated. Preferably, the two excitation electrodes on the sides are of the same width, and the width of the sensing electrode is about two times the width of a side, excitation electrode. The capacitive fluid level sensor for accurate measurement of fluid height and its changes in the micro- or meso-fluidic scale is also applicable for other pumping mechanisms besides gravity-driven pump systems. For example, the sensor is used in quantifying fluid height changes in an open reservoir with fluid driven by a peristaltic pump, which is related to and can be used to compute the stroke volume and overall flow rate provided by the peristaltic pump. Preferably, microfluidic gravity driven pumps are coupled with a capacitive fluid level sensor with the parallel arrangements of primary sensing and back self-shielding/guarding electrodes, to support a closed-loop feedback controlled fluidic supply system for applications in numerous microfluidic channels, wells, or devices.

Two or more multi-organ bioreactors may be daisy chained due to the pass-through design of internal channels (e.g., air actuation lines) passing through the body of the pneumatic plate of the bioreactor. Two or more bioreactors may also be stacked to save space. Pneumatic line and fluid connection layouts for stacked configuration are provided.

The platform is preferably fabricated from materials that minimize loss of biochemical factors due to adsorption. In some embodiments, the top fluidic plate is fabricated from polysulfone. In some embodiments, the top fluidic plate is fabricated from polystyrene. In some embodiments, the pneumatic plate is fabricated from acrylic material. In some embodiments, the actuation membrane is fabricated from polyurethane; alternatively elastomers are placed on the multi-chamber pumping unit in sections to replace the polyurethane membrane.

The organ-on-chip has on-board pneumatic microfluidic pumping in order to achieve extended 3D culture of functional tissue such as liver tissue. The on-board pumping technology minimizes space, auxiliary equipment, and dead volumes associated with excess tubing. This multi-organ platform features deterministic pumping for precise flow rate control over a wide range of flow rates from 0 to several hundreds of milliliters per day with controlled volume flux such as between 0.1 and 10 microliter per stroke, at frequencies between about 0.01 Hz and 20 Hz, to provide controlled recirculation of medium within each MPS as well as controlled "systemic" circulation.

The platform has a similar footprint to a typical multi-well plate with chambers designed to house different types of micro-tissues. The individual tissue compartments are equipped with their own intra-MPS pumps to provide nutrient recirculation and are fluidically connected to the mixer via passive spillways for level control. Although one-organ culture is feasible with the platform (e.g., with benefits of perfusion and drug addition coming from other wells), the hardware can be reconfigured to accommodate multiple applications including 2-way, 3-way, 4-way and N-way interactions (N>=2), with user-defined control of flow rates and flow partitioning from the mixing chamber to the different tissues, recapitulating physiologically relevant circulation.

Validations of multi-way MPS interactomes are also provided. "M - W MPS" refers to a configuration whereby each individual micro physiological system has its own internal circulation to control oxygenation and mixing and mechanical stimulation independent of other MPS units on the platform. Each MPS is connected fluidically to other MPS units in a controlled manner via the central circulatory flow circuit, or via direct connections. For example, the gut module has an internal circulation to mix the fluid beneath the transwell membrane and receives flow from the central circulatory flow, then its effluent goes directly to the liver. The liver module has its own internal circulatory flow, and receives flow from the gut, the pancreas, and the central circulatory flow.

**BRIEF DESCRIPTION OF THE DRAWINGS**

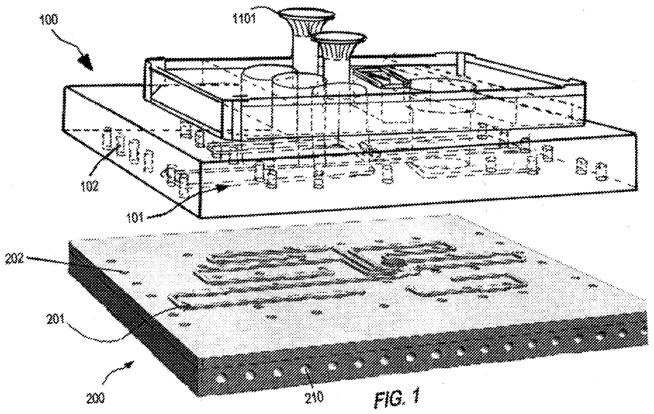

Figure 1 is an exemplary diagram of components in a multiwell device with on-board pumping system. A fluidic plate **100** contains two or more wells, which can be fitted with inserts such as a transwell **1101,** fluid paths **101** providing fluid connectivity between at least two of the wells, and pin holes or slots **102** for attachment with a second plate **200**. The second plate **200** (e.g., a pneumatic plate) contains a number of internal channels (i.e., air actuation lines), each with openings **210** (an inlet opening and an outlet opening) on opposing sides of the second plate **200.** On the surface of the second plate is one or more protruding features **201** corresponding to the shape, totuosity, and length of the fluid paths **101** of the fluidic plate **100.** These protruding features have holes in connection to each of the internal channel, such that compressed air or vacuum is distributed through the internal channels to holes on the surface of the pneumatic plate. The pneumatic plate also has slots **20**2 for attachment with the fluidic plate. Stainless steel screws fasten the layers together into a single unit that can be handled like a traditional N-well plate.

Figure 2 is a schematic showing two devices daisy-chained at the openings **210** of the internal channels (i.e., air actuation lines) of the pneumatic plate **200.** The fluidic plate **100** (with a plate lid **1200)** is assembled with the pneumatic plate **200.**

Figure 3A shows a schematic of an assembled 7-way device, having wells **103** for cell culture and/or mixing medium where a transwell insert **1101** is fitted into a well. Two ports **105** in fluid connectivity with the fluid paths of the fluidic plate may be used to connect with external fluid containers for import and/or export of fluid.

Figure 3B is a map showing the organs to be placed and flow directionality between organs on a 7-way platform corresponding to Figure 3A.

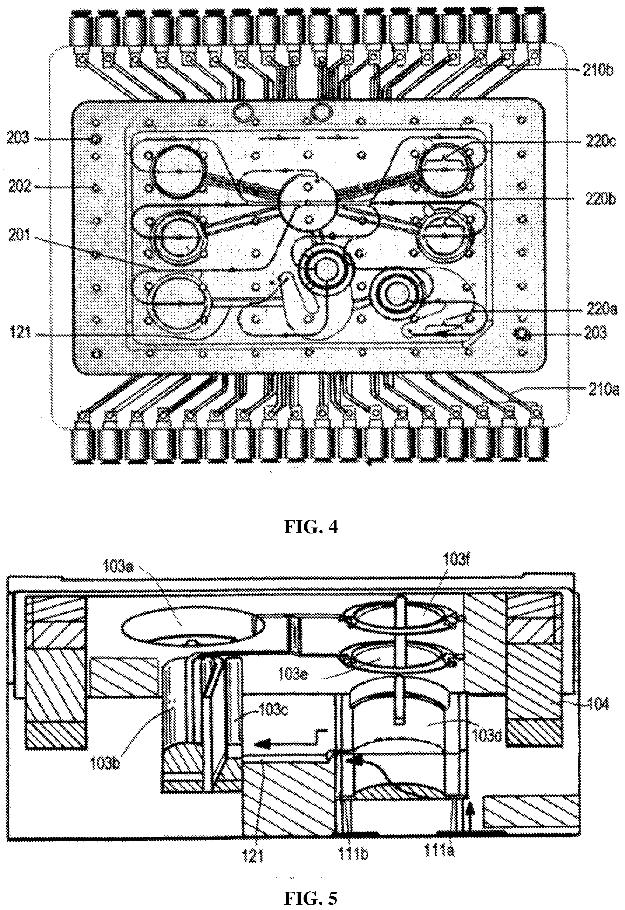

Figure 4 is a schematic of a top view of a pneumatic plate of a 7-way device. The plate has alignment pins **203** for alignment and slots **202** for attachment with a fluidic plate. The plate has protruding features **201** on the surface which in multiple locations has a set of three holes, representing a set of three-chamber units **220a, 220b,** and **220c.** These three sets of three-chamber units are in air/pressure connection with three internal channels (i.e., air actuation lines) with inlet and outlet openings **210a** and **210b** on opposing sides of the pneumatic plate. The middle hole/chamber of each of these three sets of three-chamber units is positioned to share a same internal channel (i.e., air actuation line). The hole/chamber on the same (i.e., left- or right-hand side) of the middle hole/chamber of each of these three sets of three-chamber units is positioned to share another same internal channel (i.e., air actuation line), reducing the complexity of pneumatically actuated flow controls of the device. Corresponding positions of a fluidic plate's wells and spillway conduit **121** are also shown on the pneumatic plate here.

Figure 5 is a schematic showing a cross-sectional side view of a gutliver-lung-endometrium 4-way platform. Arrows represent the direction of fluid flow, where fluid is pumped into a gut **well 103d** via an inlet **111a** in the well, and excess fluid above a height is spilled through a spillway conduit **121** to a liver well **103b** that contains an oxygenation tail **103c.** The gut well also has an outlet **111b** in the well for potential same-well recirculation of fluid with inlet **111a.** Fluid from a mixer/mixing **well 103a** flows through fluid paths to cell culture wells including an endometrium **well 103e** and a lung well **103f.** The plate also has a moat **104** to combat evaporation.

Figure 6 is a diagram showing the flow directionality and cell culture type of each well on a 4-way platform operating in a two-way configuration.

Figure 7 is a diagram showing of the flow directionality and function of each well on a 4-way platform operating in a one-organ configuration.

Figure 8 is a diagram showing the flow directionality, flow partitioning, and cell culture type of each well in a 2-way configuration.

Figure 9 is a diagram showing the flow directionality, flow partitioning, and cell culture type of each well on another 4-way platform.

Figure 10 is a diagram showing the flow directionality, flow partitioning, and cell culture type of each well on a 7-way platform.

Figure 11 is a diagram of a different configurations of well orientations for drug additions to a 2-way interactome.

Figure 12 is a schematic showing a top view of a spillway (containing a spillway conduit **121)** providing unidirectional fluid connectivity from a source well **103i** to a sink well (or destination well) **103j.** The inlet **111a** and outlet **111b** of the source well **103i** are also shown.

Figure 13 is a side view of the entry geometry for a spillway from a source well **103i** (containing an outlet hole **111b,** e.g., for active pumpinginduced recirculation). Radial meniscus pinning groove **122** aligns with a curved entry geometry **124** of the spillway, and the curved entry geometry aligns with the bottom of a conduit groove **125** of the spillway conduit. Transwell height is set by the vertical location of a step shelf **123** on which the outer rim of the transwell rests.

Figure 14 is a side view of the exit geometry for a spillway **121** into a destination well **103j.** The exit geometry of the spillway includes an undercut **130** in the wall of the destination well, below the edge of the spillway conduit, and a vertical groove **131** to guide along the wall of the destination well.

Figure 15 is a cross-sectional side view of a perfusable scaffold in a perfused well of a device showing the apical volume **1102** in the scaffold and the basal volume **1103** in the well.

Figure 16A, Figure 16B, and Figure 16C illustrate a successive time-course, potential development of a spillway V-shaped entry geometry of (cross-sectional side view), from initial continuous fluid film across the spillway (Figure 16A), to breakage of fluid film (Figure 16B), and finally drying in the sink well and over accumulation in the source (Figure 16C).

Figure 17 is a schematic of a cross-sectional side view of another spillway entry geometry without the V-shape in Figure 16A, for continuous fluid film across the spillway.

Figure 18 is a schematic of an enlarged cross-sectional side view of the spillway entry geometry corresponding to Figure 17, i.e., U-shaped conduit with a groove at the bottom.

Figure 19 is a schematic of a cross-sectional side view of another embodiment of a spillway geometry. This spillway has a conduit **127** that permits open fluid flow (space above the conduit **126**) with a tower conduit **128a** entry, and an upward conduit exit **129a.**

Figure 20 is a schematic of a top view of the spillway shown in Figure 19. The tower conduit has an opening, i.e., a hole **128b,** on the surface of a step in the wall of the source well, which connects to the spillway conduit **127** in an open-fluid configuration **126.**

Figure 21 is a schematic of a cross-sectional side view of the spillway shown in Figure 19, where a screw **140** plugs the tower conduit **128a,** preventing spillout flow from a source well.

Figure 22A, Figure 22B, and Figure 22C illustrate a successive time-course development of a spillway with a V-shaped entry geometry of (cross-sectional side view), from initial fluid front into the conduit (Figure 22A), to migration of fluid front along the conduit (Figure 22B), and fluid accumulation in conduit (Figure 22C).

Figure 23 is a schematic of the dimension of conduit geometry for calculation to determine spontaneous capillary flow (SCF). $W_F$ symbols the dimension of liquid-air interface.

Figure 24 is a schematic of the dimension of a rectangle conduit for calculation to determine SCF. The conduit has a depth of b and a width of a, totaling a cross-sectional conduit perimeter of Pw, whereas the liquid-air interface has a perimeter of $P_F$.

Figure 25 is a schematic of a cross-sectional side of a spillway without a V-shaped entry geometry to support SCF.

Figures 26A-26D show different views of a rounded bottom spillway conduit at the inlet (Figure 26A), a diagonal view (Figure 26B), a section view (Figure 26C), and at the outlet (Figure 26D).

Figures 27A-27D show different views of a spillway conduit with a knife edge geometry at the inlet (Figure 27A), a diagonal view (Figure 27B), a section view (Figure 27C), and at the outlet (Figure 27D).

Figures 28A-28D show different views of a spillway conduit with a V-cut geometry at the inlet (Figure 28A), a diagonal view (Figure 28B), a section view (Figure 28C), and at the outlet (Figure 28D).

Figure 29 is a schematic of the cross-sectional side view of a spillway conduit geometry, i.e., U-shaped with a bottom-located rectangle groove of a high depth-to-width ratio (e.g., greater than 3).

Figure 30A, Figure 30B, and Figure 30C illustrate another successive time-course development of a spillway with a V-shaped entry geometry (cross-sectional side view), from initial continuous fluid film across the spillway (Figure 30A), to fluid accumulation in the conduit (Figure 30B), and syphon effect (Figure 30C).

Figure 31 is a schematic of a cross-sectional side view of a spillway exit geometry, where the spillway conduit 127 ends with a slope 132, and a distance of d below the conduit there is an undercut **130** in the wall of the destination well. A vertical groove **131** below the slope **132** and interrupted by the undercut **130** is present along the wall of the destination well.

Figure 32 is a schematic of a top view of a spillway exit geometry where fluid flowing from a small-width groove **127** encounters an enlarged curved area **132** for exit.

Figure 33 is a schematic of a top view of an oxygenation tail **150a** with guiding grooves **151** on the bottom surface of the well.

Figure 34 is a schematic of a top view of a well **103** connecting to a zig-zag oxygenation tail **150b.**

Figure 35 is a diagram showing the geometry features of the zig-zag oxygenation tail shown in Figure 34, for a phase-guiding purpose. The tail has a maximum width of $W_1$ and a minimum width of $W_2$, appearing in an alternating order for a length of $L_1$ and $L_2$, respectively. The angle $\alpha$ symbols the direction of an increasing width with respect to the fluid flow direction in the oxygenation tail.

Figure 36 is a schematic of the cross-sectional side view of a removable, perfused scaffold **160** inserted into a well on platform, which shows a ramp area **159** for securing (e.g., turn by screw thread) the scaffold, radial seals **161a** and **161b** (e.g., O-rings), a cell culture region **162** in the scaffold, and a fluid aggregation lid **163** useful for non-contact oxygen sensing.

Figure 37A is a schematic showing the top view of a three-chamber unit on the surface of a pneumatic plate.

Figure 37B is a schematic showing the side view of a three-chamber unit corresponding to Figure 37A. A barrier membrane **300** separates a fluidic plate (containing a fluid path **101)** and a pneumatic plate. The pneumatic plate has protruding features **201** on which holes create chamber spaces that are connected to internal channels (air actuation lines) of the pneumatic plate (not shown in this Figure). Here the chamber **221** serves as a valve, chamber **222** as a pump, and chamber **223** as another valve.

Figure 38 is a schematic of a top view of split fluid flow on top of dual three-chamber units that are controlled by four air actuation lines.

Figure 39 is a cross-sectional side view schematic of an in-wall fluid level sensing capacitor **1200,** including front electrodes **1201** and back electrodes **1203** that are on opposing sides of a board **1202** (e.g., polychlorinated biphenyl (PCB) board).

Figure 40 is a top view schematic of the electrodes of the in-wall fluid level capacitive sensor shown in Figure 39, showing a front sensing electrode **1201b** with a front reference electrode **1201a** coplanar on one side and another front reference electrode **1201c** coplanar on the other side, as well as a back sensing electrode **1203b** with a back reference electrode **1203a** coplanar on one side and another back reference electrode **1203c** coplanar on the other side.

Figure 41 is a schematic of three layers of pneumatic lines for stacked platform.

Figure 42 is a cross-sectional side view schematic of a top plate **150** and a bottom plate **250,** with geometries supporting sintering between the two plates. The bottom plate **250** has protruding pillars **251a** and **251b** with narrowed vertices **252a** and **252b,** respectively, and flat surfaced protrusion **253** lower than the protruding pillars by a height of $d_2$.

Figure 43 is a cross-sectional side view schematic of a fused, onepiece construct **350,** sintered from the top plate **150** and bottom plate **250** of Figure 42. The vertices of protruding pillars in Figure 42, after sintering (forced compression between the top plate and the bottom plate under heat), have deformed into sintered surfaces **252c** and **252d** and attached with the top plate. Space between protrusions of a bottom plate before sintering has become space (e.g., channel) for fluid **351.**

Figure 44A is a diagram showing an apical insert assembly **3000** for use with standardized transwells and a fluidic plate. The apical insert assembly includes an inlet point **375** and an outlet point **377.** Figure 44B is a diagram showing the apical insert assembly **3000** with compression fittings **400** and **402** for 1/16" OD tubing, four 0-80 screws **405a, 405b, 405c,** and **405d,** an apical insert **407,** an o-ring **409,** a standard 12-well transwell **412,** and a lower ring **410.**

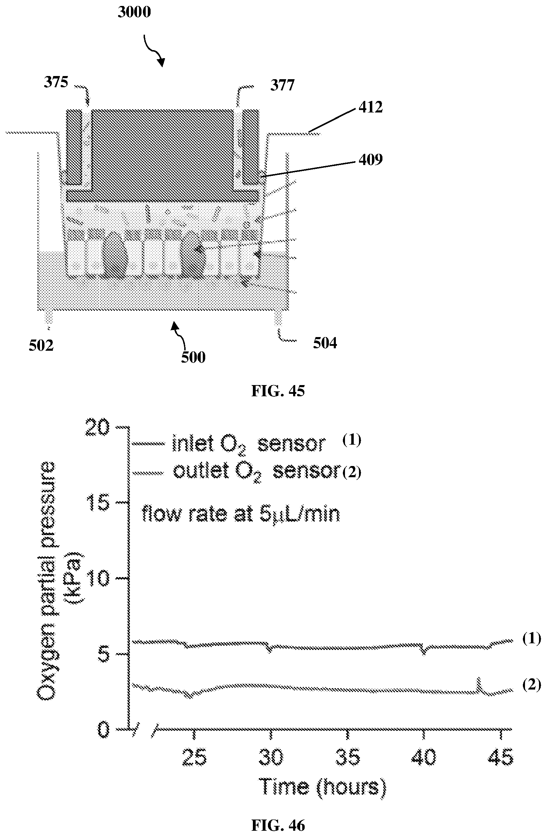

Figure 45 is a diagram showing a simplified version of an apical insert assembly **3000** with a transwell **412** seeded with a monolayer of 9:1 of Caco2/HT-29 cells receiving a feeding medium inoculated with commensal bacteria with different oxygen tolerability. The feeding medium for the apical insert assembly **3000** is supplied through the inlet point **375** (apical feed), and removed through the outlet point **377** (apical effluent), and is sealed in with the o-ring **409.** The apical insert assembly **3000** is suspended in a basolateral compartment **500** containing a medium with immune cells. The medium at the basolateral compartment **500** is supplied through a basal feed port **502** and removed through a basal effluent port **504.**

Figure 46 is a line graph showing the oxygen consumption rate as oxygen partial pressure (kPa) over time (hours) for Caco2-HT29 mixtures seeded on a membrane exposed to physioxia on apical side and normoxia on basal side. The oxygen consumption was measured at inlet oxygen sensor ((1), top line) and outlet oxygen sensor ((2), lower line) over time.

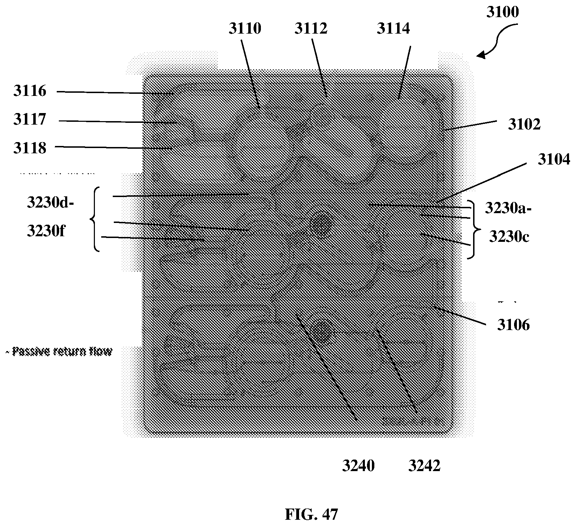

Figure 47 is a floor plan view of a three-organ culture system. The system includes a pneumatic control plate (not shown) positioned below the fluidic plate **3100.** The fluidic plate **3100** includes three parallel lanes **3102, 3104,** and **3106,** each lane containing MPS transwell **3110,** MPS perfused scaffold **3112,** MPS transwell **3114,** and utility wells **3116, 3117,** and **3118** for feeding, drug dosing, and waste removal. The transwells, scaffold, and utility wells are interconnected with fluidic channels. Micropumps **3230a-3230f** pump fluid between the transwells, scaffold, and utility wells. The micropumps are bi-directional, with a wide range of flow rates, and high precision. The fluid level in each lane is maintained through spillways **3240** and **3242.** The three-organ culture system is a flexible system for 1-3 organ co-culture, it accommodates 12- and 24-well transwell models, includes a perfused scaffold for tissues with high $O_2$ demand, and programmable reservoirs for automated feeding, drug dosing, sampling, and waste removal.

Figure 48 is a schematic of an exemplary closed-loop gravity-driven pump having two hydrostatic pressure chambers (also denoted fluid supply reservoirs) and a fluid-level sensor.

Figure 49 is a schematic illustrating an exemplary microfluidic capacitive fluid level sensing containing a specific electrode arrangement for non-contact fluid level measurement for a reservoir.

Figure 50 is a schematic showing the capacitive fluid level sensor, shown in Figure 49, connected to a standard capacitance-to-digital-converter terminals.

Figure 51 is a schematic illustrating a cross-sectional detail of an excitation-sensing-excitation (ESE) electrode design in both the primary sensing arrangement and the back arrangement of the capacitive fluid level sensor of Figure 49, with respect to the measured fluid and surrounding environment.

Figure 52 is a schematic illustrating exemplary physical details of the capacitive fluid-level sensor of Figure 49, adapted for fabrication via traditional or flexible printed circuit board manufacturing.

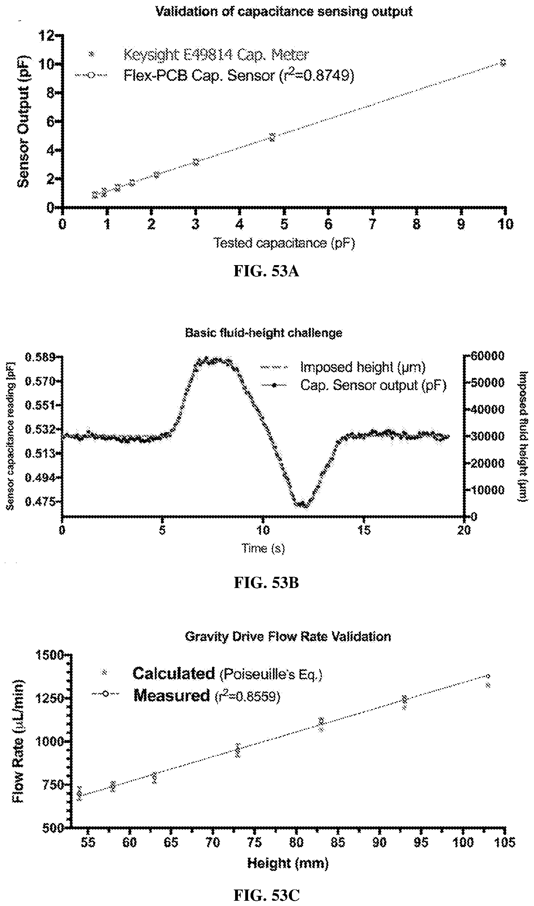

Figures 53A-53C are graphs showing results of the initial characterization experiments used to assess the accuracy of capacitance readings and basic fluid level tracking capabilities of the sensing circuit. Figure 53A is a graph showing the correlation between capacitance sensor output (pF) measured with Keysight E49814 Capacitance Meter and the tested capacitance (pF) measured with the Flex-PCB Capacitance Sensor ($r^2 = 0.8749$). Capacitances measurements with the developed sensing circuit matched real values within <0.1 pF error. Circles denote average of experiment conducted in triplicate at a single capacitance value with the error bars shown. A linear fit of the measured values is also shown. Figure 53B is a graph showing results of basic fluid-level tracking using capacitive sensing. The dashed profile is the expected fluid-height ($\mu$m) as imposed by the calibrated syringe pump. Solid lines refer to the averaged capacitive sensor output (Sensor capacitance reading [pF]) for the three replicates at each time point (s). Figure 53C is a graph showing the measured and calculated flow rates ($\mu$L/min) as a function of total fluid height (mm) in gravity-driven pump. The measured values (circles) with error bars, closely approximate the theoretical values calculated using Poiseuille's equation (asterisks). A linear fit of the measured values is also shown.

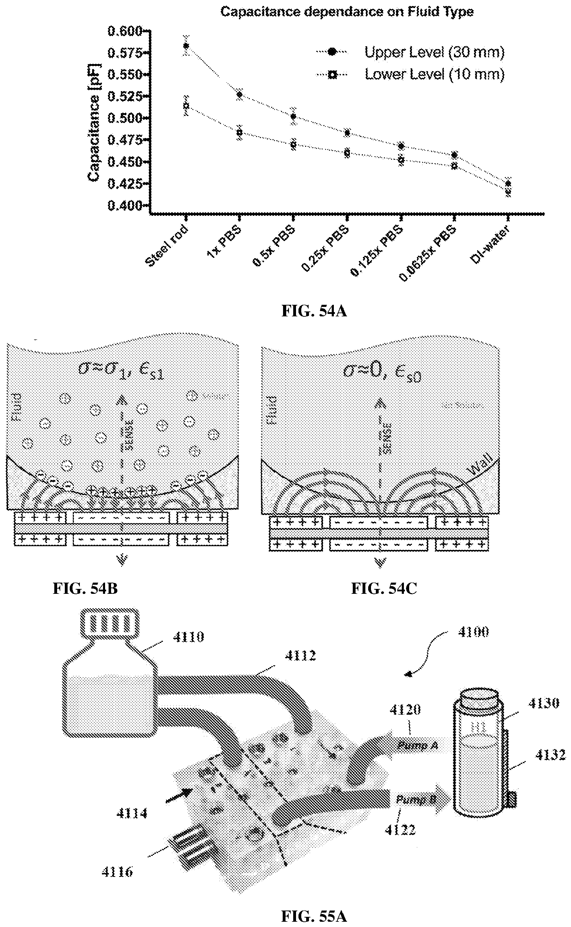

Figure 54A is a graph and Figures 54B and 54C are diagrams showing the dependence of capacitive sensing on fluid type. Figure 54A is a graph showing change in capacitance (pF) with the change in fluid type. Use of fluids with high concentration of solutes forming free ions lead to higher capacitance measurements and ranges. Error bars representing standard deviation of upper measurements (h=30mm) and lower measurements (h=10mm) are shown. Figure 54B is a diagram showing capacitive fringing for electrolytic fluids with free ions. Figure 54C is a diagram showing capacitive fringing for non-conductive fluids with high dielectric constant. Fringes at the fluid-wall interface exhibit a change in angle towards the symmetry axis (not shown) depending on properties of wall material and fluid. Finite element analysis (FEA) of the electric displacement flux density for 1xPBS target fluid and FEA simulation of electric flux density for DIwater as target were performed.

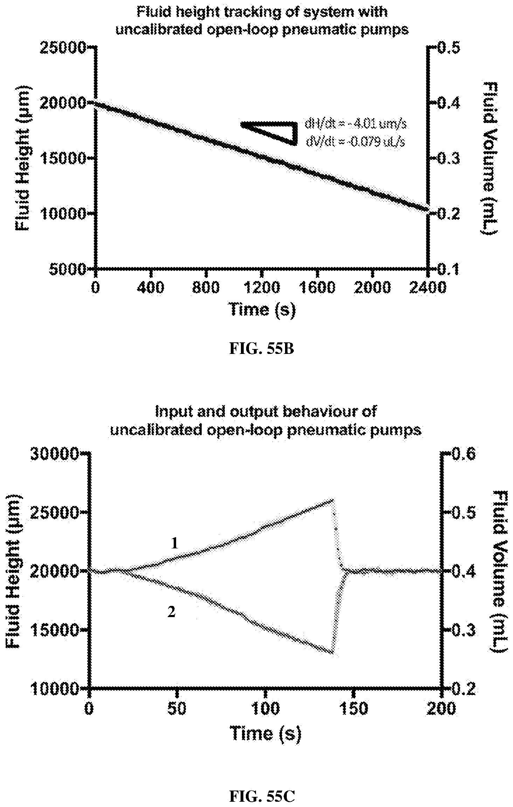

Figure 55A is a diagram of a test block setup **4100** and Figures 55B and 55C are graphs showing fluid height ($\mu$m) change over time (s) with the test block setup. Figure 55A shows a testing block **4114** with six parallel pneumatic diaphragm micropumps, connected to a supply reservoir **4110** and monitored reservoir **4130** with a capacitive sensor **4132**. Two pumps, **4120** and **4122**, were used to drive fluid in and out the monitored fluid reservoir. Figure 55B is a graph showing fluid-height change over time (40 min) as measured by the capacitive sensor in the system driven by unbalanced input/output micropumps. Figure 55C is a graph showing an overlay of isolated experimental input and output behavior of each pneumatic diaphragm micropump. Profile **1** denotes fluid-height over time produced by pump **4120** (input) alone, while profile **2** shows the same for pump **4122** (output). Points denote average for the three replicates at each time point. Standard deviation is shown.

Figures 56A and 56B are graphs showing results of closed-loop feedback control for a constant set point ($\Delta$H=30mm, Figure 56A) and for a dynamic set point using constant, sine, triangular, saw tooth and step waveforms. (Figure 56B). Black points refer to the averaged capacitive sensor output for the three replicates at each time point. Error bars are also shown.

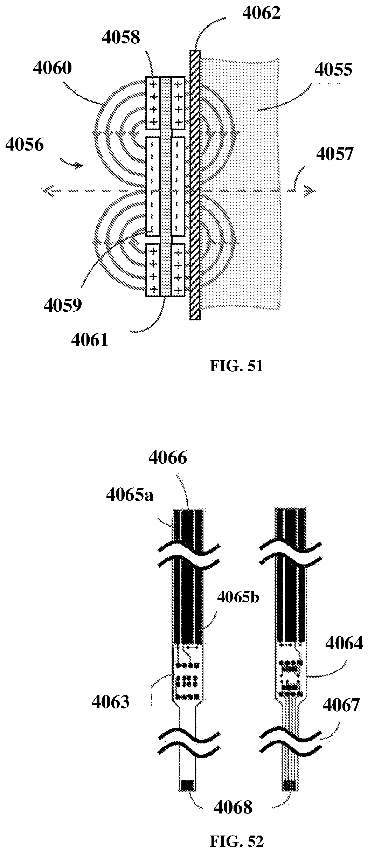

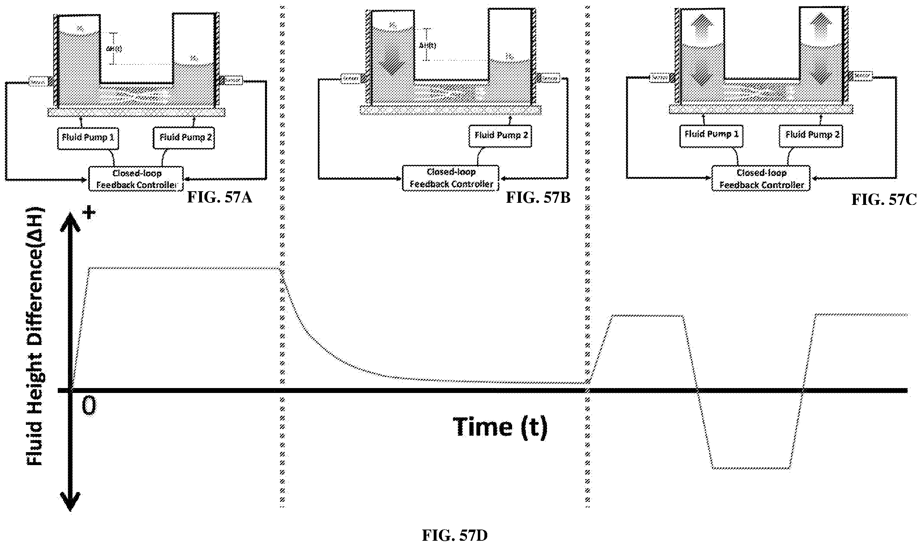

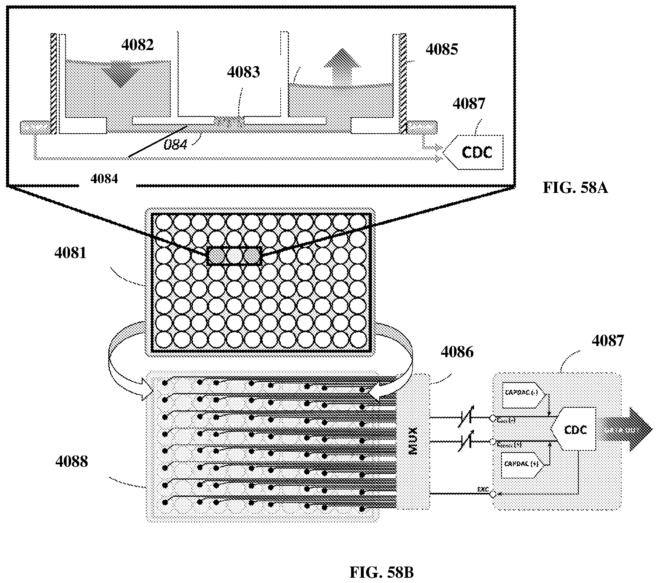

Figures 57A-57C show the schematics of various setups of a closed-loop gravity-driven pump system. Figure 57D is a line graph exemplifying different fluid height profiles according the three variations shown in Figures 57A-57C. Figures 58A and 58B are schematics illustrating the use of three consecutive wells in a modifier microtiter plate for the integration of a closed-loop gravity-driven pump system containing a sensor of Figure 52 for the assessment of the hydraulic permeability of a porous material (Figure 58B); the plate is modified with multiplexed electrodes allowing for closed-loop feedback control. Figure 58A provides details that a first well as a fluid supply reservoir imposes gravity-driven flow through a second well containing the porous material, and at least one non-contact fluid-level sensor monitors the decrease rate of fluid height in the reservoir.

## DETAILED DESCRIPTION OF THE INVENTION

### *I. Definition*

The terms "organ-on-chip (OOC)", "bioreactor", and "microphysiological system (MPS)", used interchangeably, refer to the platform providing for interactions among single or multiple organ or other tissue types on an *in vitro* platform which provides for the maintenance of growth of these tissues.

The term "pneumatic" refers to a system which uses air or vacuum pressure for operation.

The term "manifold" refers to an interconnection device for pneumatic or fluid connections.

The term "spillway" refers to a system of fluidic connections between a source well and a destination well to automatically maintain fluid levels in the source well.

The term "leveling" refers to maintaining fluid level.

The term "self-leveling", refers to maintaining level using passive means, i.e., without active means.

The term "undercut" refers to a mechanical detail associated with an overhanging feature.

The term "wetting" refers to the wetting of a solid surface by a liquid in a gas environment, which is determined by the minimum in Gibbs energy of the system. Wetting of a solid surface by a liquid in a gas environment results in an equilibrium contact angle $\theta$ across the liquid phase between the solid/liquid (SL) and liquid/gas (LG) interfaces as they emanate from the contact line. Generally the terms "wetting" and "nonwetting" surface refer to cases of $\theta < 90°$ and $\theta > 90°$, respectively. The relationship between the contact angle and the interfacial energies involved is expressed by Young's equation $\gamma_{SV} = \gamma_{SL} - \gamma_{cos}\theta$, where $\gamma_{SV}$, $\gamma_{SL}$, and $\gamma$ are the Gibbs interfacial energies between solid and gas, solid and liquid, and liquid and vapor, respectively, and where the last quantity is addressed as surface tension. To satisfy the thermodynamic equilibrium requirement, the gas phase is saturated with vapor.

The term "meniscus" refers to the fluid boundary at the intersection of fluid with a solid material and a vapor phase.

The term "meniscus pinning" herein refers to, in a situation of raising the level of a wetting liquid in a vertical well to the top edge, the end of the wetting line with a contact angle $\theta$ stays (or "is pinned") at the top edge of the well while the contact angle $\theta$ to rise from < 90° to > 90° at the top edge of the well side wall during further increase of the liquid level, until accumulation of liquid results in spilling over the edge of the well, thus releasing the contact line ("unpinned"). For nonwetting liquid, meniscus pinning occurs at the base edge and the top edge of the side face of a vertical well, and at the top edge the angle for the liquid orientation at the contact line changes from the value $\theta$ to the value $\theta+90°$. Details of the term is described in Wijs et al., Separations: Materials, Devices and Processes, 62(12):4453-4465 (2016).

The term "capillary length" refers to a characteristic length scale for an interface between two fluids which is subject both to gravitational acceleration and to a surface force due to surface tension at the interface.

The term "insert" refers to an element which can be mechanically assembled in a well of an MPS.

The term "scaffold" in the relevant sections is an insert or component of the wells which provides support for tissue constructs.

The term "whippletree" refers to a mechanism to distribute force or pressure evenly through linkages. As used herein, it refers to force or pressure applied from one direction at or near the center and distributes to the tips (generally two tips), where each serves as the center for distribution to further tips.

The terms "program" or "software" refer to any type of computer code or set of computer-executable instructions that can be employed to program a computer or other processor to implement various aspects of embodiments as discussed above. Additionally, it should be appreciated that one or more computer programs that when executed perform methods of the present invention need not reside on a single computer or processor, but may be distributed in a modular fashion amongst a number of different computers or processors to implement various aspects of the present invention.

An open loop control system acts entirely on the basis of input, and the output has no effect on the control action. A closed loop control system considers the current output (feedback) and alters one or more parameters in the system to a desired condition; that is, the control action is based on the output. A closed loop feedback control system may in some instances allows for external inputs to initiate, alter, or terminate the control.

The term "gravity-dominated" or "gravity-driven" refers to a pump or reservoir whose main force to drive a fluid flow is hydrostatically generated due to the vertical weight of the fluid tower within the reservoir. Other effects such as capillary forces is mostly neglible. For example, the ratio of the dominant gravitational force to the surface effects (or any other force present) exceeds 10:1. Generally fluid reservoirs containing fluid of a height >10mm satisfy this force ratio requirement using common water-like fluids (such as water, cell culture media, buffered saline), even in the presence of solutes which may alter surface tension. Fluid reservoirs with gravity-toother force ratios smaller than 10:1 in some embodiments are also treated as gravity-driven when gravity remains greater than any other acting force and the force ratios have been characterized to account for their effects in the system.

*II. Apparatus and Operation of Apparatus*

Each multiwell device is generally a three-component construct with an on-board pumping system. A fluidic plate **100** contains multiple wells, some be fitted with inserts such as a TRANSWELL® **1101** (Corning, distributed also by Sigma-Aldrich), and built-in micromachined fluid paths **101** for distribution of culture medium (Figure 1). A pneumatic plate **200** distributes compressed air and vacuum to the surface of the pneumatic plate through small holes. A barrier membrane **300** (generally translucent) is situated between the fluidic plate **100** and the pneumatic plate **200,** which under pressure may flex to expand or contract, thereby obstructing or clearing corresponding portions of the fluid paths of the fluidic plate. This barrier membrane also provides a sterile barrier, acting as the actuation layer of the pumps and valves.

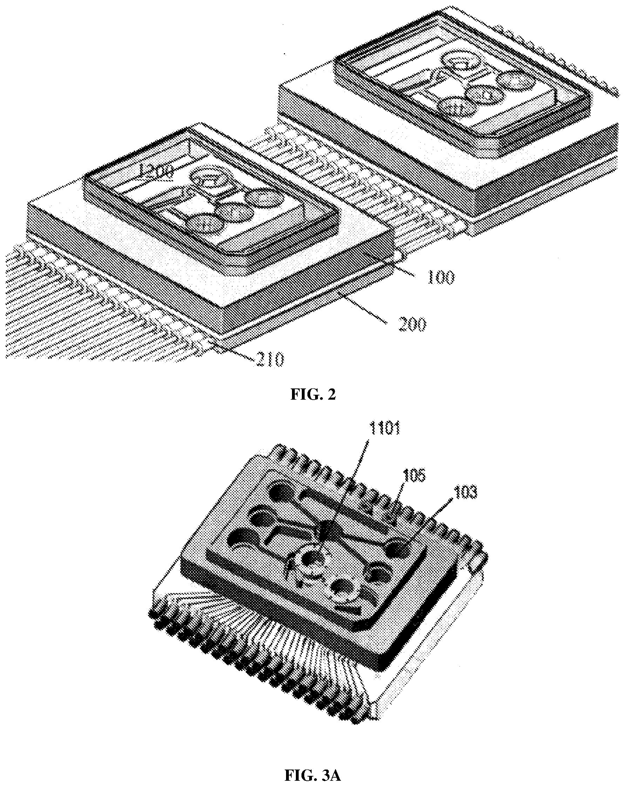

Multiple devices can be chained for simultaneous in-phase operation/actuation (Figure 2). Each device is a bioreactor, which as a platform supports the culture of multiple MPSs mimicking different organs, their interconnections, and interactions as *in vivo*. The open wells and channels allow users easy access to the cells and culture media to perform measurements requiring direct fluid contact. Up to seven of these MPS have been coupled together, as demonstrated in the examples, although it is understood that the system allows for mixing of more than one of the same type of MPS as well as mixing and integration of a variety of different types, not limited to a total of seven.

The system uniquely incorporates a high degree-of-freedom (DOF) on-board pumping system, effectively configured to support multiple organ culture. While existing devices have compartments linked linearly by a single pump to drive flow through a loop (Materne EM, et al., J. Vis. Exp. 1-11 (2015). doi: 10.3791/52526) or linked in parallel with channel diameters imposing predefined passive flow rates (Oleaga C., et al., Sci. Rep. 6, 20030 (2016)), a high DOF control makes it easy to reconfigure the platform for addition of new MPSs or exclusion of certain compartments.

In some embodiments of 4-way MPS bioreactors, the platform may operate with 18 degrees of freedom ("DOF"), or 18 individual channels of tubing. For example, in a liver-gut-lung-endometrium 4-way MPS, an individually addressable pump requires 3 DOF, while multiple pumps can be run at the same rate by sharing inlets on the pneumatic manifold across multiple pumps. A 4-way MPS platform may have 6 independently programmable flow rates which are used to drive 9 pumps. All four pumps providing mixer-to-MPS flow can be individually addressable. Recirculation pump rates are shared: mixer/liver recirculation are linked, as are gut/lung/endometrium recirculation. It is economically advantageous to link pump rates, as this reduces the number of pneumatic valves and tubing connections required for a platform.

In some embodiments of 7-way MPS bioreactors, the platform has 36 DOFs which operate the functional equivalent of 17 syringe pumps per platform, and can dynamically control intra- and inter-MPS mixing. In this instance, only 12 flow rates can be independently specified, as each requires 3 pneumatic lines.

**A. Multi-well Bioreactor**

(1) Overview of Directions of Fluid Flow

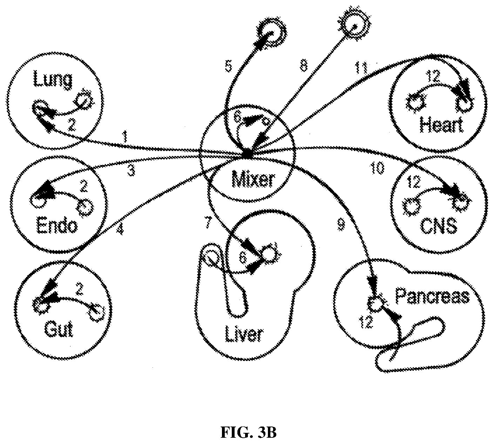

Figure 3A shows a schematic of a 7-organ interactive bioreactor, for which Figure 3B shows an exemplary map of tissues to be cultured in each well and directions of fluid flow. In an exemplary 7-way bioreactor containing lung, endometrium, gut, liver, heart, central nervous system (CNS), and pancreas, generally active flow of fluid is conducted via built-in fluid channels from the mixer well (Mixer) to lung (arrow 1 in Figure 3B), from Mixer to endometrium (Endo; arrow 3 in Figure 3B), from Mixer to gut (arrow 4 in Figure 3B), from Mixer to liver (arrow 7 in Figure 3B), from Mixer to pancreas (arrow 9 in Figure 3B), from Mixer to CNS (arrow 10 in Figure 3B), from Mixer to heart (arrow 11 in Figure 3B); and via within-well pumping to recirculate within each of lung, endometrium, gut, heart, CNS, liver, pancreas, and Mixer (arrows 2, 6, and 12 in Figure 3B). External supply may be imported to Mixer (arrow 8 in Figure 3B), which through the fluid flow gets distributed to each organ well. Waste from Mixer may be exported to an external collector (arrow 5 in Figure 3B). In some embodiments, each out-flow from Mixer to an organ has a designated pump for individually controlled flow rates, as well as the external supply import to Mixer and the export of waste to external collector from Mixer. To reduce complexity in some embodiments, the recirculation within each of lung, endometrium, and gut may share one pump control for an identical recirculation flow rate; the recirculation within each of heart, CNS, and pancreas may share another pump control for an identical recirculation flow rate; and the recirculation within Mixer and within liver may share yet another pump control for an identical recirculation flow rate.

Spillways are generally designed between at least one pair of wells, and in one embodiment of the 7-organ platform between lung and Mixer, between endometrium and Mixer, between gut and liver, between liver and Mixer, between heart and Mixer, between CNS and Mixer, and between pancreas and liver, to automatically transfer excess fluid from the former well to the latter.

Figure 4 shows a schematic of the pneumatic bottom plate corresponding to the exemplary 7-way apparatus shown in Figure 3A for multi-organ culture as mapped out in Figure 3B. A pneumatic plate may have alignment pins 203, in some embodiments two pins at symmetrical positions about the center, on the side of the pneumatic plate for mating/aligning with corresponding features (e.g., pin holes or slots) on the bottom of the top plate. A pneumatic plate may also have a number of holes 202 throughout the depth of the plate, on multiple locations (not obstructing the air-conducting actuation lines), for corresponding protruding pin features on the bottom of the top fluidic plate to align with. On the pneumatic plate shown in Figure 4, there are 18 internal channels as air-conducting actuation lines spanning horizontally across the inside of the pneumatic plate. For example, a set of three air-conducting actuation lines with air inlets and air outlets 210a and 210b (entry and exit being relative to the orientation of the plate) controls multiple three-chamber units 220a, 220b, and 220c that are located on the surface of the actuation-side (i.e., the side that through an actuation membrane assembles with the bottom of the fluidic plate) of the pneumatic plate. Each three-chamber unit (e.g., bracketed as 220a, 220b, and 220c) has three chambers, each having an air-conducting hole to the surface connecting with a horizontal air-conducting line below, and three chambers as a whole controls, via pneumatic actuation causing plus and minus deflection of a membrane, the stroke or the peristaltic fluid flow in the fluid channel of a top plate once assembled. The pneumatic plate may also have protruding curved line raised features 201 connecting one or more three-chamber units. These raised features provide the matching sealing surface for the corresponding fluidic channels in the bottom surface of the fluidic plate which conduct fluid in defined fluidic circuits interconnecting the various fluidic MPS modules. These raised features 201 can be seen outlining the positions of fluidic paths in a fluidic plate once the pneumatic plate is assembled with a fluidic plate. Element 121 shows the position of the spillways which carry fluid between the MPS modules in a fluidic plate, once the pneumatic plate is assembled with a fluidic plate.

Figure 5 shows a cross-section of an exemplary 4-way platform showing a built-in channel for fluid flow from mixer to gut, and a general spillway position from gut to liver. The disclosed wells for cell culture on the multi-organ MPS platform generally follow this "flow-in/spill-out" principle of operation.

Operation of the directions of active flow and passive spillover of fluid generally mimic circulation paths in *in*

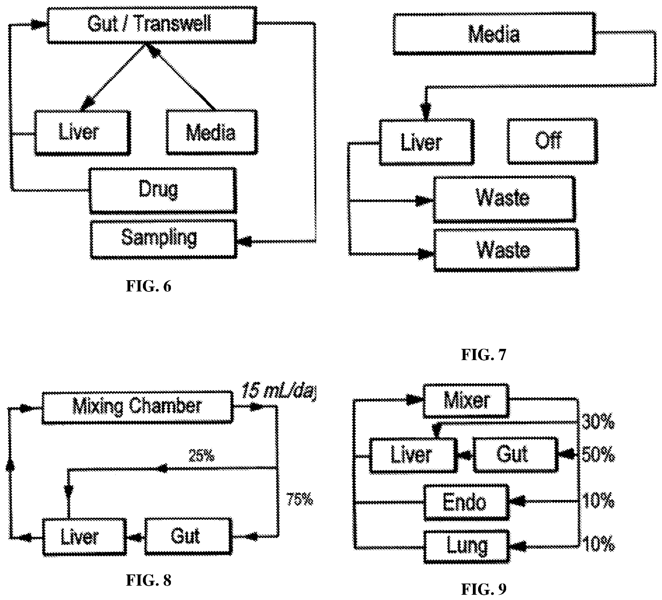

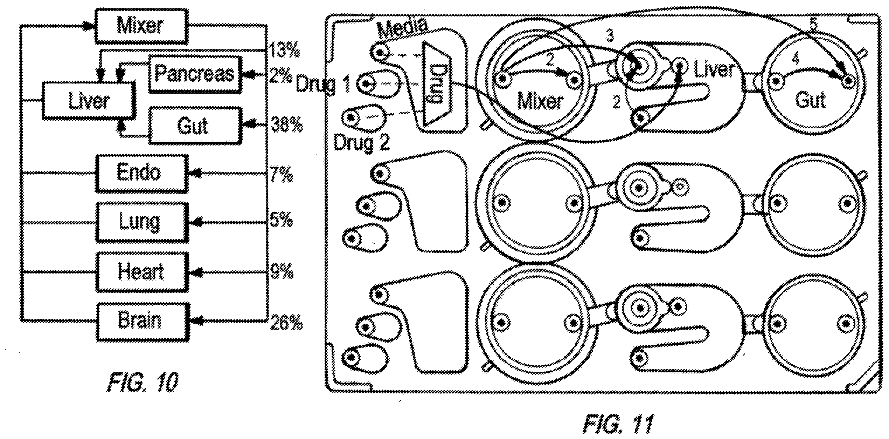

*vivo* systems, and the principles as shown in the exemplary 7-organ bioreactor are applicable to platforms of 2-way, 3-way, 4-way, or other numbers of MPS systems. Exclusion of one or more wells from use in a multi-well platform is feasible via alteration in software code for operation, and no hardware change is required. Each well is also reconfigurable for multiple uses. For example, a mixing chamber (Mixer well) may also be used as immune-competent gut MPS well, or be used with a TRANSWELL®. A liver MPS well may be used as a media reservoir or drug reservoir. Exemplary reconfigured use of a multi-well platform is shown in Figures 6-10. Flow partitioning is generally achieved by varying the frequency of pumping. Another exemplary configuration of multi-well platform is shown in Figure 11, where three drugs housed in three wells are delivered to liver well and gut well, while the wells are perfused and in interaction via Mixer well and the spillway between liver and gut.

(2) Means for Controlling Flow Direction and Level Self-Leveling Spillways

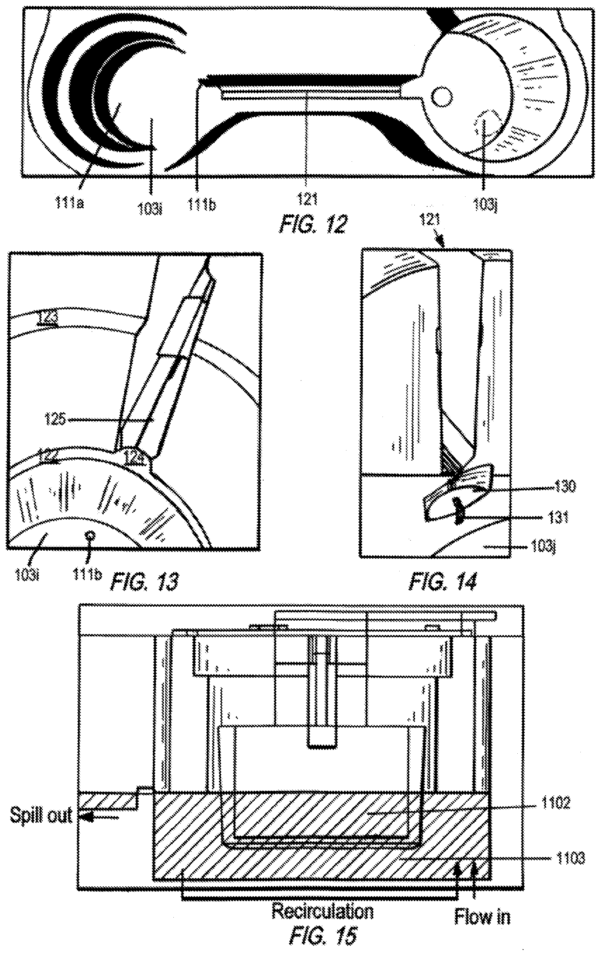

The apparatus achieves self-leveling of MPS wells passively and fluid return, generally to Mixer, by a system of spillway channels cut into the top side of the plate to deliver excess fluid back to the mixer. In general, a spillway includes a channel (e.g., open fluid) above certain of the bottom wells, which connects an inlet well to an exit well (Figure 12). Spillways eliminate the need for return pumps and level sensors for enforcing a balance between influx and efflux, while also allowing return flows to cross over the inlet MPS feed flows. In preferred embodiments, the spillways avoid breakage of fluid flow in the spillway when leveling is needed, and avoid the siphon effect to prevent drying out of wells.

The apparatus uses spontaneous capillary flow (self-wetting) and phase guiding principles to guide flow and wetting in fluid pathways to allow for more robust operation of open fluidic organ-on-chip systems. Unidirectional flow from a source well to a destination well is achieved with meniscus control features, detailed below, and other characteristics including additional groove geometry of the spillway conduit, controlled surface roughness, surface tension, and additional features in the entry and exit of the spillway. These one or more geometric features in fluid containers for the organs-on-chips apparatus allow for pinning of fluid in a radial fashion to limit the meniscus effect created by surface tension. This construction could allow for better passive fluid leveling which could then translate in more deterministic performance and measurement within these systems.

The spillways implement passive leveling in the following fashion. If fluid flow into the inlet well causes a net accumulation of fluid in the inlet well, the level in the inlet well will begin to rise. As the level begins to rise, the fluid will rise at the spillway, and thereby cause increased flow through the spillway into the exit well. If the level in the inlet well decreases, the fluid level at the spillway of the inlet well will drop, thereby decreasing the flow through the spillway. In this manner, the level in the inlet well is passively controlled to be approximately equal to a desired level. Such leveling is passive in that there is not an active process of sensing level and changing some pumping rate in response to this sensing of level. Rather the effects of gravity and surface tension combine to regulate flow in a passive manner not requiring explicit sensing and control.

To achieve proper spilling function, the spillway employs a low resistance flow path in the direction from source to sink, above the designed height of fluid in the source. In some embodiments, the path is impermeable to flow in from the sink to the source and the system, such that as a whole the spillway may be resistant to transient changes in fluid height due to tilting.

Entry geometry

Various inlet features are useful for stabilizing the source well meniscus, providing an entry into the spillway channel or a way of sealing the volume of the media in the source well.

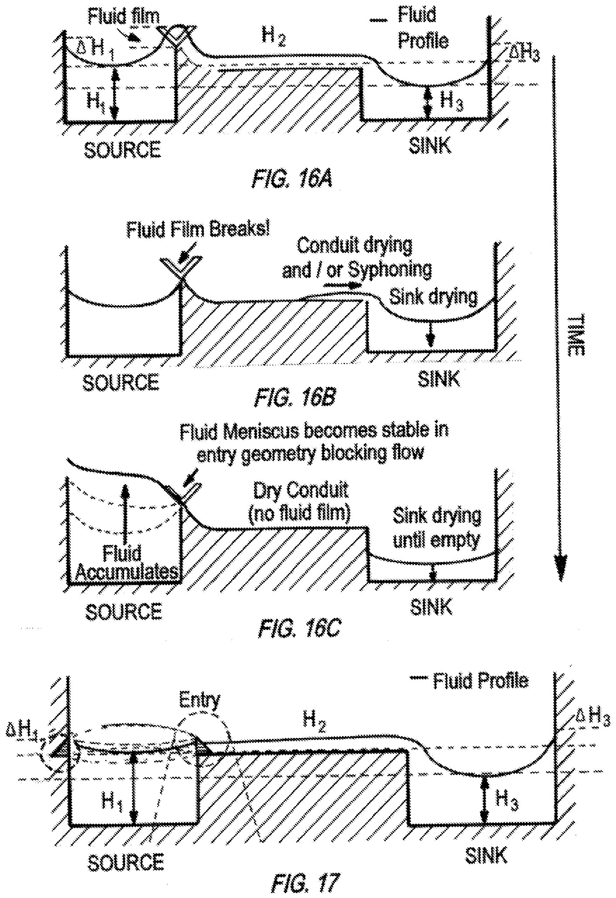

Figures 16A-16C show a time-course schematic of how a spillway with a V-cut at the source well (inlet well) experiences discontinuation of the fluid film (e.g., fluid film breaks) and thus the spillway conduit dries, causing fluid to accumulate in the source well and the sink well to dry until empty. This type of spillways start off operating in a metastable regime with a connected fluid profile that allows fluid transport. When fluid film breaks (specifically at entry step and V-cut geometries, the fluid finds it more energetically favorable to accumulate in the source well, thus increasing in height, rather than to advance in the spillway entry and spillover into the conduit and sink well (outlet well). When the height increases beyond a certain value, it eventually spills over; but for organs having large surface area, such as pancreas and liver, this increase in height requires a large amount of volume, which was found to be a major reason for the mixer to dry out after 12 hours in incubator in testing of the 7-way platforms using these geometries.

The following have been determined to improve efficacy:

Shallow and gentle entry for flat meniscus

Shallower and gentler entry geometry to the spillway minimizes energy for spilling fluid into conduit groove. A radial groove in the source well directs meniscus and makes use of height increases to produce spilling events.

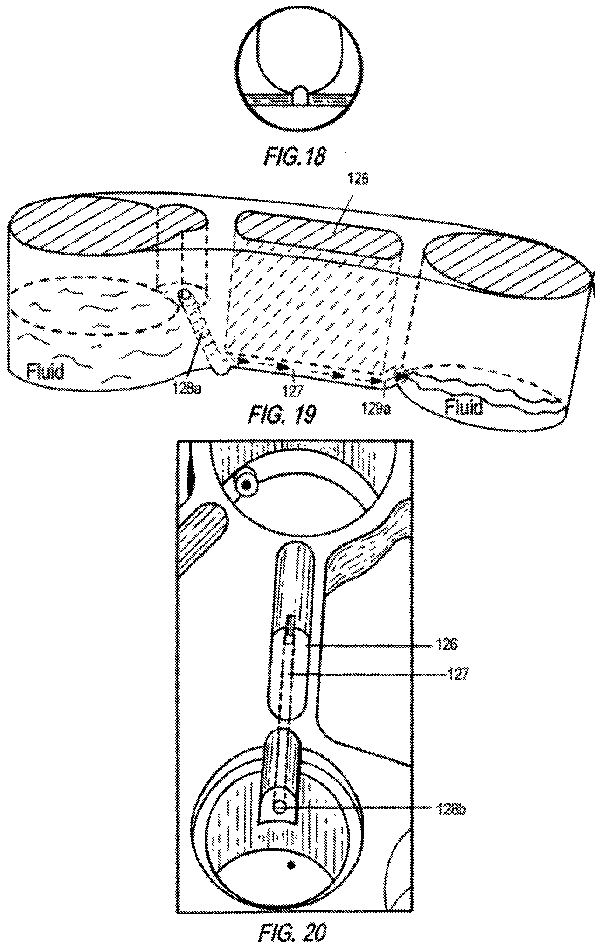

When fluid film is present and spillways are conducting fluid, the step and V-cut features may not prevent volume displacement from transient tilting or siphoning. Therefore, for some embodiments, an entry step and a V-cut are eliminated to minimize fluid film disruption at this level. Step barriers may be used to prevent further fluid build-ups, as shown in Figure 17 with a cross-sectional view of an exemplary entry without the V-cut shown in Figure 18.

When gravity dominates and surface tension effects are negligible as in large wells with larger interconnecting spillways, V-cuts are effective in determining the exact height of self-levelling and breaking the connection. For smaller geometries, it is more effective to have a direct entry into the spillways (and in one embodiment, have a meniscus pinning groove) and take care of breaking the fluid contact by the use of spillway exit features.

Fluid-pinning groove

In some embodiments, the entry to the spillway additionally includes a "fluid pinning" groove, which can be a 20-, 30-, 40-, 45, 50-, or 60-degree circumferential groove **122,** preferably 45-degree, in the fluid wells. This groove captures the fluid meniscus, which facilitates maintaining a defined fluid height and improves the dynamics of leveling and spillway operation. The bottom of this radial meniscus pinning groove aligns with the bottom of the spillway fluid flow channel as detailed in Figure 13. The pinned meniscus is unstable, and thus will spill over, so that the fluid does not rise beyond the height of the radial meniscus pinning groove.

Insertion of Teflon rings for deterministic fluid level.

Placing Teflon rings at different heights relative to the spillway determines the maximum fluid height before spilling. An inserted Teflon ring captures meniscus, therefore securing the liquid level not to go pass it. The ring also helps prevent evaporation.

***Embodiments***

Figure 13 shows one embodiment of the improved entry geometry for the spillway, in which a shallow and gentle entry of fluid via a radial meniscus pinning groove around the well, where the bottom of the meniscus pinning groove aligns with the bottom of a grooved fluid flow channel.

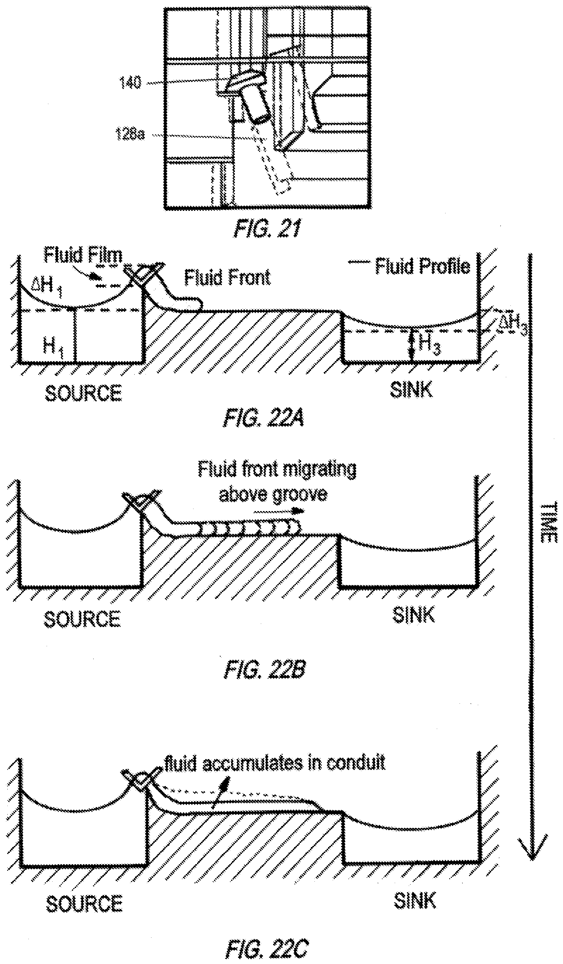

Figures 19 and 20 show another embodiment of an improved entry geometry for an open conduit spillway in a cross-sectional side view and a top view, respectively. A slanted conduit tower **128a** connects the source well to an open conduit **127,** which may have a spontaneous capillary flow (SCF) groove at the bottom. The entry geometry utilizes a hole-in-the-wall design, where a hole **128b** is created on a step surface to connect to the slanted conduit tower **128a.** A screw seal **140** may be placed to plug the opening hole of the conduit **128a** to isolate MPS interactions (Figure 21). The screw seal generally has an O-ring next to the thread to create a good seal once plugged into the hole.

*Conduit allowing for spontaneous capillary flow (SCF)*

Figures 22A-22C illustrate a time-course development of fluid across the spillway conduit from a spillway with a V-shaped entry geometry. When the conduit has not been primed or when spillway conduit is dry due to evaporation or fluid film disruption, the front of a migrating fluid coming from the source well forms a meniscus within the wall of the conduit, which advances slowly and accumulates fluid above the groove of the conduit. This spillway conduit issue was first observed in dye testing on a 7-way alpha spillway, where the spillway was wetted by fluid front but the fluid migration along the conduit was slow and required substantial volume to wet the entire spillway.

The following represent means for improving flow by altering conduit geometry.

Geometry and dimension to allow spontaneous capillary flow to assure robust wetting in channels.

The fluid movement efficiency along the channel was compared among a round-bottom, a V-shaped, and a rectangle-bottom open channel of a comparable small dimension. 2 $\mu$L of fluid droplet was added at one end of the open channel to measure the wetting distance without priming of the channel. A V-shaped channel was shown to exhibit a wetting distance of 103 mm; a rectangular shaped channel had a wetting distance of 44 mm, and a round-bottomed channel had a wetting distance of 7 mm. Both the V-shaped channel and the rectangle-bottom channel support Concus-Finn flow (Berthier J, et al., AIMS Biophysics, 1(1):31-48 (2014)). A greater wetting distance generally shows a greater wettability performance which maintains a continuous fluid flow in an open channel spillway.

Effect of material used to form the Conduit

A conduit with spontaneous capillary flow (SCF) maintains a fluid film and thus fluidic communication with minimal volume requirements and without any particular priming or pumping rate. To achieve SCF, the cross-section of the conduit should satisfy the following relationship:

$$\frac{p_F}{p_W} < \cos\theta$$

,

where

$P_F$ = The free (in contact with air) perimeter
$P_W$ = The wetted (in contact with wall) perimeter
$\theta$ = The generalized Cassie angle (the average contact angle of the material).

SCF results when the energy reduction from wetting walls outweighs the energy increase from extending the free surface. Using Gibbs thermodynamic equation, the general criterion for spontaneous capillary flow in composite-wall and air systems is the generalized Cassie angle $\theta$ must be <90°. The generalized Cassie angle is the average contact angle of the material. In preferred embodiments where the fluidic plate is made with polysulphone, the contact angle for media-polysulphone-air has been measured to be 30° < $\theta_c$ < 113° for polysulfone with water or media. This wide range of contact angles is based on the effects of surface micro pattering and in lesser degree small differences in polysulfone hydrophobicity and thermal effects of incubation environments. To satisfy the SCF relationship, the range of perimeter ratios that allow for SCF in the embodiments described herein ranges from 0 < $P_F/P_W$ < 0.866 (cos 30° ≈ 0.886; negative perimeter ratios are not possible, thus not considered). This is an exemplary estimation, and it is to be understood that other implementations may utilize alternative ratios. Practically, the contact angle anywhere in a channel is reasonably assumed to be ≤80°, considering the meniscus effect and/or poorly wettable surface (which may be machined to generate a smooth finish to encourage higher wettability). Therefore in a scenario with a prominent meniscus effect, or with poorly wettable surfaces, such that the contact angle is about 80°, the perimeter ratio goes **0 < $P_F/P_W$ < 0.18** (cos 80° ≈ 0.174; arccosine 0.1866 ≈ 80°) in order to satisfy the SCF relationship.

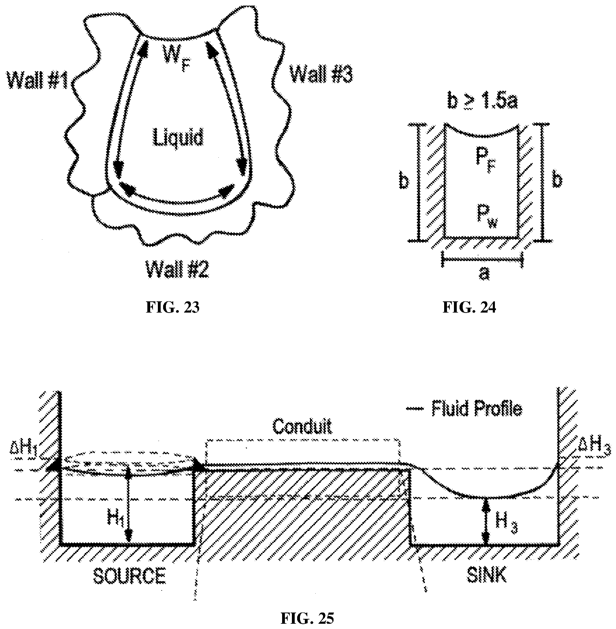

Figure 23 and Figure 24 provide a cross-section analysis of a channel of an arbitrary shape. Here, the perimeter of liquid exposed to air, $W_F$, would be the free perimeter, $P_F$, in the above relationship; and the sum of liquid perimeter in contact with three walls, $W_1+W_2+W_3$, would be the wetted perimeter, $P_W$, of the above relationship. Figure 24 illustrates an exemplary rectangle shaped channel with a width of $a$ and a height of $b$. To satisfy the SCF relationship, the perimeter ratio should follow:

$$\frac{P_F}{P_W} = \frac{1.5a}{(2b+a)} < \cos(80°).$$

When defining an aspect ratio, $\lambda$ = b/a, therefore b = $\lambda$a, the relationship goes

$$\frac{1.5a}{(2a\lambda+a)} < 0.18,$$

which can be calculated to derive a criterion for the aspect ratio to allow SCF by a poorly wettable surface and/or a channel surface with a prominent meniscus effect:

$$\lambda = \frac{b}{a} > 3.7 \approx 3.$$

Therefore, a small rectangle channel with an aspect ratio greater than 3 generally can achieve SCF.

In some embodiments considering manufacturing capabilities, the aspect ratios range is 2.5 < $\lambda$ < 5 to support the SCF design principle.

In some embodiments, spontaneous capillary flow is achieved in a triangular horizontal channel with an aspect ratio of about 2, where the wall smoothness is such that the contact angle is about 60°. The calculation of $P_F/P_W$ for a triangular channel would be different compared to a rectangular channel, but the same principles hold.

In some embodiments, a preferred fluid path within the spillway conduit is a rectangle or V-shaped channel with an aspect ratio greater than 3, which is within microfluidic dimensions to allow for capillary flow to occur(Figure 25 showing a continuous fluid film across the spillway). Upon an initial fluid contact with the conduit channel, a minimal volume of fluid in a channel with a geometry supporting SCF will quickly wet the entire geometry and produce a fluid film capable of efficiently transporting fluid from source to sink.

Capillary length and spillway width to assure gravity dependent spilling.

According to Brakke et al., Exp Math, 1(2):141-165 (1992), for water in contact with acrylic (which has a similar hydrophobicity to polysulphone), the capillary length, $[\gamma/(\rho^* g)]^{1/2}$ (where $\gamma$ is the surface tension, p is the density of the liquid, and g is gravity acceleration), is 2.7 mm. If the distance between the two walls of a channel (i.e., width of the spillway channel) is less than the capillary length, gravity has a negligible effect. Therefore, a spillway width of 2.1 mm places the system in a regime where gravity is less dominant than capillarity.

In some embodiments where spilling is desired to be driven by gravity (e.g., in conduit tower **128a),** the spillway width is greater than 3 mm.

### *Embodiments*

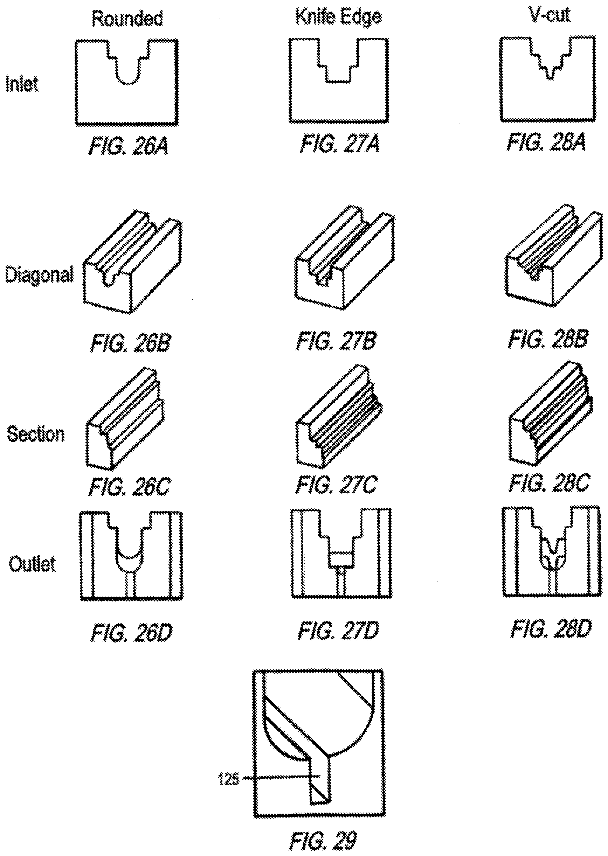

Figure 29 shows an exemplary spillway conduit geometry with a 3:1 aspect ratio rectangle-shaped groove to allow for spontaneous capillary flow. U-shaped channel above spillway is a relief cut to allow space for the drill bit collet.

In preferred embodiments, the surface tension spontaneously propagates once the liquid in the source well is leveled, and drives movement of fluid through the conduit to the target well.

### *Exit geometry with undercut design*

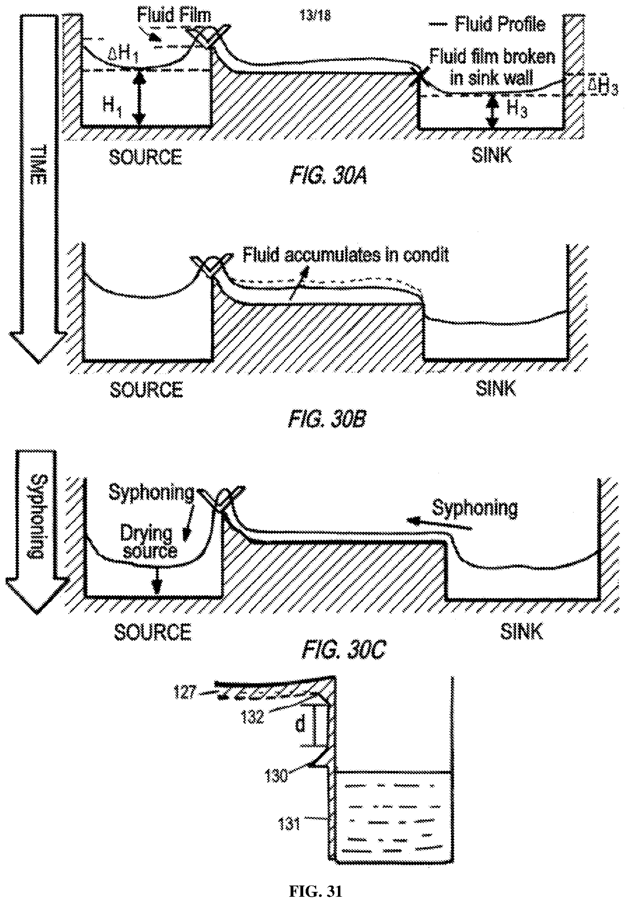

Figures 30A-30C illustrate spillway exit from a spillway with a V-shaped entry geometry and no additional exit geometry. When the spillway exit does not have a fluid film in the vertical wall, fluid starts accumulating in the conduit and leads to spilling bursts or even a stable meniscus at the exit geometry. This accumulation stops when the meniscus of fluid at the conduit makes contact with the meniscus at the sink, and a fluid film is reestablished. When fluid film is always present, a poor exit design may see the siphon effect even after the source fluid level is below the sink level.

This problem can be avoided or minimized using one or more of the following options:

Sharp undercut along a high aspect ratio vertical groove to prevent backward flow.

Figure 31 illustrates the spillway conduit **127** exits, via a slightly tapered, shallow slope (edge) **132,** to connect with a vertical groove **131** along the wall of the sink/destination well. A sharp undercut **130,** e.g., made with a milling machine, breaks the vertical groove **131** into two parts. The undercut is a cut into the wall of the sink well below the tapered, shallow slope **132,** and has an angle from the vertical line of greater than about 30° (e.g., 30°, 35°, 40°, 45°, 50°, 55°, 60°, or more, and any continuous angle in between the exemplary numbers). In some embodiments, the distance between the undercut **130** and the spillway conduit exit, d, is between about 5 to about 10 times the width of the spillway conduit, in order to prevent the syphon effect. The vertical groove **131** is designed to exhibit spontaneous capillary flow (SCF) and to maintain a fluid film. The vertical groove runs continuously from top to bottom, except where the undercut is present. This geometry helps maintain a stable fluid film connecting the conduit and sink as long as there is forward fluid directionality. In case of reverse flow (e.g., the syphon effect), the undercut cuts the fluid film and generates a fluid meniscus that will only re-connect the fluid film when forward flow is reestablished.

In some embodiments, the spillway exit vertical groove is configured to exhibit spontaneous capillary flow (SCP) using the same design parameters described in the SCF groove in the conduit, e.g., a high aspect ratio greater than 3. The undercut and the high aspect ratio vertical groove have been tested in a series of experiments in 3x3 alpha spillways and machined polysulfone block, leading to a controlled fluid film breakage and anti-syphoning effect. A stable vertical fluid film on the improved exit geometry does not easily evaporate and allows for fluid film restoration and flow upon forward flow at spillway exit is resumed.

Rounded slope exit and small-width groove to break film into droplets.

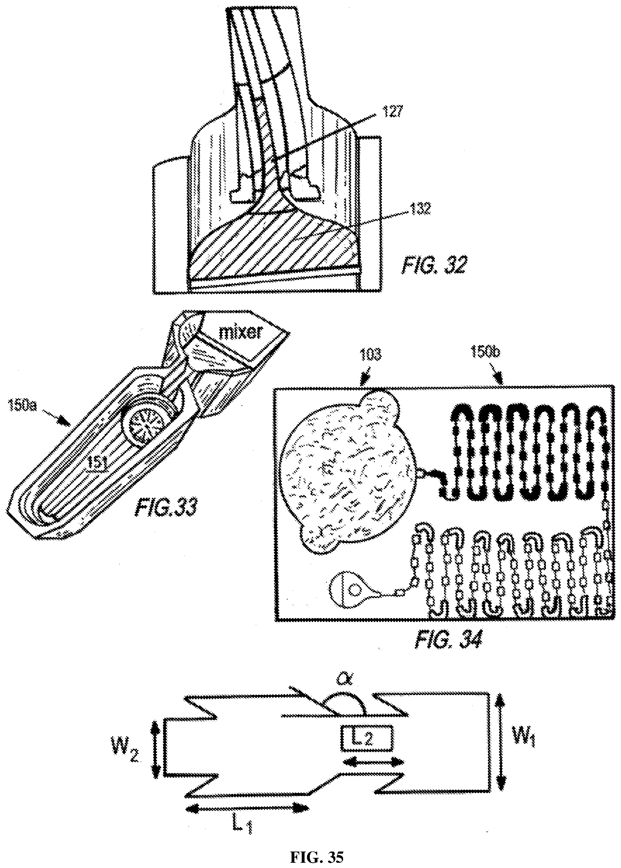

Another improved feature is to introduce a rounded slope exit/edge at the end of the spillway conduit. When the small-width SCF groove of the spillway conduit "meets" an enlarged, round-curved area (Figure 32), the stable liquid film in the small-width SCF groove (due to surface tension) becomes unstable at the enlarged round curved exit area, which is effectively broken into droplets and would fall ("sheds") into the sink well. This way, the source well becomes independent from the sink well, and unidirectionality of fluid flow is achieved.

In some embodiments, the entry geometry to the conduit from the source well has no slope, i.e., it drops from a sharp edge, while the exit geometry from the conduit encounters an enlarged, curved area, before liquid drops into the sink well.

Alternative upward exit from the conduit

In some embodiments where the SCF channel is below the desired liquid level in the sink well, an upward exit conduit with an exit hole is utilized, as shown by element **129a** of Figure 19.

*Embodiments*

Figure 31 illustrates the spillway exit with a undercut beneath the exit, and vertical groove for anti-siphon effect.
Wall-bound drops that are pinned on an edge of a planar wall are generally referred to as wall-edge bound drops. Wall-Edge bound drops are typically found in nature as dew hanging from the leaves of plants until a sizable volume is reached and the drop falls. When drops are pinned on a pointed wall edge, they are referred to as wall-edge-vertex-bound drops. Wall-edge-vertex-bound drop simulations show liquid interfaces in contact with highly wetting solid walls (forming a spillway exit) tend to drip as the angle decreases. This is because the energy decrease from wetting the walls is greater than the energy of the liquid-air interface, such that the contact area wants to expand indefinitely in corners with smaller angles where thin fluid filaments form. The creation of a thin fluid filament is relevant and desirable in situations where accurate control of fluid leveling and flow is needed for open-channel fluidic systems, as the meta-stability of these filaments can provide means to allow or stop fluid transport.

(3) Recirculation

Passive self-leveling may contribute to return of flow as described in detail above.
Typically, recirculation is used to ensure that within a well, the concentrations are well distributed and uniform. Thus, recirculation flowrates are typically higher than organ to organ flowrates.
Active recirculation, driven by within-well pumping, may increase oxygenation of the media. For example, recirculation may take place within each of lung, endometrium, gut, heart, CNS, liver, pancreas, and Mixer in a 7-way MPS platform. To reduce complexity in some embodiments, the recirculation within each of lung, endometrium, and gut may share one pump control for an identical recirculation flow rate; the recirculation within each of heart, CNS, and pancreas may share another pump control for an identical recirculation flow rate; and the recirculation within Mixer and within liver may share yet another pump control for an identical recirculation flow rate.

(4) Features to encourage oxygenation

Adequate perfusion rates to "meso-scale" tissues, commonly containing hundreds of thousands to many millions of cells, is difficult and critical to cell viability. Based on the oxygen consumption rate of liver, which has a high oxygen requirement, using cell culture medium as the circulating fluid, a flow rate between about 6 and 10 $\mu$L per second is needed per million of cells (Powers M J, et al., Biotechnol Bioeng 78, 257-69 (2002); Domansky K, et al., Lab on a Chip 10, 51-58 (2010); Ebrahimkhani MR, et al., Advanced Drug Delivery Reviews Apr, 132-57 (2014)). Because gas exchange can occur at the air-liquid interface in the open fluidic system in the disclosed apparatus, the platform material itself, though optional, does not need to be oxygen permeable.

*Oxygenation tail*

A tail in addition to the main well for cell cultures is preferably designed for organs such as liver that higher levels of oxygenation for survival. The oxygenation tail has features supporting better diffusion and mixing of oxygen into the media such as shallow walls, faster recirculation, and independent inflow and outflow lines.
Exemplary layouts of the oxygenation tail includes a guiding groove tail (Figure 33), a tail that is vertically rounded (e.g., and deepening), a flat tail with pinning columns, and a flat tail with meniscus pinning groove tail.
The tail preferably includes a slanted surface such that the depth of liquid can be as thin as 5 mm, 4 mm, 3 mm, 2 mm, or 1 mm, for sufficient aeration/oxygenation. In preferred embodiments, the apparatus supports cell culture survival for up to a month, two months, or longer.
In addition to the surface roughness and geometry of patterns on the tail surface, tortuosity of the tail as well as the width of the tail may be modified to enhance oxygenation. For example, zig-zag shaped, tortuous tails provide a means to enhance oxygenation requiring a reduced liquid volume for the liver module. Each turning loop or point is where meniscus can pin to. Figure 34 illustrates a zig-zag tail layout for the liver module. A total tail length of about 225 mm may support a total tail volume of about 80 $\mu$L, for enhanced passive oxygenation, i.e., increasing the surface area of liquid exposed to air. Figure 35 illustrates a phase-guiding geometry that is repeatedly present along the oxygenation tail. This tail has alternating maximum width and minimum width, $W_1$ and $W_2$, respectively ($W_1 > W_2$), in a repeated manner throughout the zigzag tail. Generally, within each segment of the tail between two U-shaped loops, there are two, three, four, or more repeats of the alternating maximum and minimum width. The alternated widths each has its own length, e.g., every maximum width $W_1$ has a length of $L_1$, and every minimum width $W_2$ has a length of $L_2$. Generally, $L_1$ is greater than $L_2$ to accommodate more volume. The phase guiding feature is attributable to the angled increase of the width. As shown in Figure 35, the angle, $\alpha$, represents the increase of tail width relative to the forward direction of fluid flow in the tail. The angled increase of tail width, i.e., the narrowing of tail width in the direction of the forward fluid flow, provides for better guidance for fluid directionality. $W_1$ may be in the range between 0.1 mm and 10 mm, for example between about 0.5 mm and about 1 mm. $W_2$ may be in the range between 0.05 mm and 5 mm, for example between about 0.3 mm and 0.5 mm. $L_1$ may be in the range between 0.5 mm and 10 mm. $L_2$ may be in the range between 0.1 mm and 10 mm. The angle, $\alpha$, may be in the range of between 90° and 180°. In one embodiments, $W_1$ is 0.8 mm, $W_2$ is 0.45 mm, $L_1$ is 1 mm, $L_2$ is 0.5 mm, and $\alpha$ is 150°. The depth of the zig-zag oxygenation tail may be a fixed depth or a gradually varying depth in the range between about 0.05 mm and 5 cm, for example between about 0.1 mm and about 10 mm. In one embodiment, the depth of the tail is fixed at 0.5 mm.

### *Active oxygenation pumping systems*

Another means to enhance oxygenation is to utilize active oxygenation pumping system both ways between the liver well and the tail.

In some embodiments, the liver culturing well has within itself a recirculation pumping system, such that it has bottom-to-top flow of oxygenated media. The oxygenation tail, generally containing liquid of a shallow depth, is recirculated within itself, such that the required oxygen concentration is reached in steady state. Active pumping allows the media from the well with scaffold (generally low on oxygen due to metabolic consumption) to be pumped to the oxygen-rich tail portion. The oxygenated media from the tail is then pumped back to the well.

(5) Removable Insert

### *Removable scaffold integrable for perfusion*

Removable scaffolds may be used for MPS of choice, e.g., liver and pancreas, allowing off-platform seeding, manipulation, and assaying of the perfused tissues. Previous scaffolds by others are difficult to remove from the platform without causing damage or contamination. In some embodiments, the removable scaffolds hold the filters and retaining rings that are a standard size, e.g., compatible with the disclosed platform and/or commercially available LIVERCHIP®.

Scaffolds are configured to allow gentle insertion and removal via rotation and sliding along a sloped guide ramp. Some MPS compartments designed for use with these scaffolds include a sloped ramp to guide the insertion and/or removal of the scaffold. A radial seal with the platform is established with a low-binding o-ring (e.g., VITON® o-ring), allowing perfusion of the entire removable device.

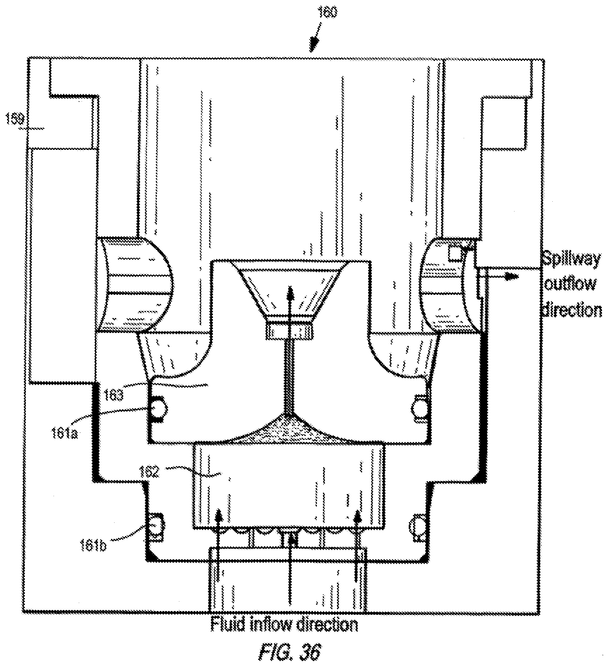

Figure 36 illustrates an exemplary modular, removable perfusion scaffold that allows perfused cell constructs to be gently removed from the surrounding platform. The device includes a cup-like shell with radial o-ring seals **161a/161b** and a flow-diffusing support structure at its base. Cells with or without biomaterials can grow on top of this support **162.** On the sides of the device in the upper body or the extension arm of the scaffold, two holes allow for manipulation with sterile tweezers and small flanges help to guide it along a ramped thread. The ramp **159** allows gentle insertion and removal via rotation, rather than vertical force. Torqueing the scaffold into place minimizes the fluid pressure experienced by the cells during insertion and removal.

Portability permits a number of functions that improve the usability of a multi-well bioreactor. For example, constructs can be cultured in isolation, in unique cell media, and then selected for health and viability before they are joined together for a human-on-a-chip experiment. A removable scaffold also allows complete isolation of one cell population from the multi-well bioreactor, allowing external assays of cell health and metabolism to be performed without tainting the shared media with potentially harmful reagents.

The scaffold supports fluid inflow from below, i.e., the bottom surface of the well, and spillway outflow to other wells on the platform.

In some embodiment, a fluid aggregation device **163** is optionally added on the removable, perfused scaffold to collect flow into a narrow orifice. Fluid is mixed, and aggregated fluid is collected past a fixed location. At the outflow location, the oxygen tension or other fluid properties can be queried by a small probe resting inside the top of the device. This way, sensors for average $O_2$ measurement do not require dipping into the media or a part of the culture.

In some embodiments, a thin scaffold with a thin bottom/wall thickness between about 0.05 mm and about 5 mm, preferably between about 0.1 mm and about 1 mm, or about 0.25 mm, situated on a membrane, is utilized to seed liver-associated cells for enhanced oxygenation, where the scaffold is perforated with an array of channels (e.g., -0.3 mm diameter) and is maintained in a re-circulating flow multi-well plate bioreactor. Liver cells seeded into the scaffold form 3D tissue-like structures, which are perfused at flow rates sufficient to create a physiological oxygen tension drop across the scaffold without excessive shear (Yates C, et al., Adv. Cancer Res. 97, 225-246 (2007)) and which can be maintained in a functional state for weeks in serum-free culture medium.

***TRANSWELL®***

The apparatus can contain wells that are compatible to hold multiple insert vehicles for cell culture, such as commercially available TRANSWELL® inserts or custom biomaterial scaffolds to support cells or organoids.

(6) Moat To Reduce Evaporation

Additionally or alternatively, some embodiments of the apparatus include a humidity moat (element **104** in Figure 5) to increase local humidity and reduce evaporation from the cell culture media. The moat may be connected to external fluid source or fluid pumped in via build-in fluid channels in the fluidic plate. Monitoring and pumping of fluid into the moat may be needed to compensate for loss of liquid due to evaporation, which is generally dependent on flow variations in the organ culture wells. The inplatform moats or micro evaporation chambers can be placed in any region of the fluidic plate to increase the moat area to minimize evaporation from the wells, allowing for the creation of a humid microenvironment around the microphysiological well zones. Local heating in the moats may also be used so most of the evaporation to maintain the high relative humidity above the platforms comes from the moats.

(7) Means for addition or withdrawal of agent/specimen

The apparatus may be connected to or used with one or more auto-sampling devices. For example, the auto-sampling devices may be fluidically connected to a low wetting sample collection tube.

**(8) Pneumatic actuation**

On-board pumping saves dramatically on space and cost compared to commercial syringe or peristaltic pumps, is more scalable, and allows closed-loop operation with very low dead volumes. Dynamic control of flow rates and directionality enables precise modulation of concentration profiles, allowing experimental operation to be scaled to match clinical/physiological distributions. Flow partitioning is controlled by imposing specific pumping frequency in the individual microphisiological systems, leading to specific flow-rates and; therefore, "partitioning" of flow.

Pneumatic manifold/plate

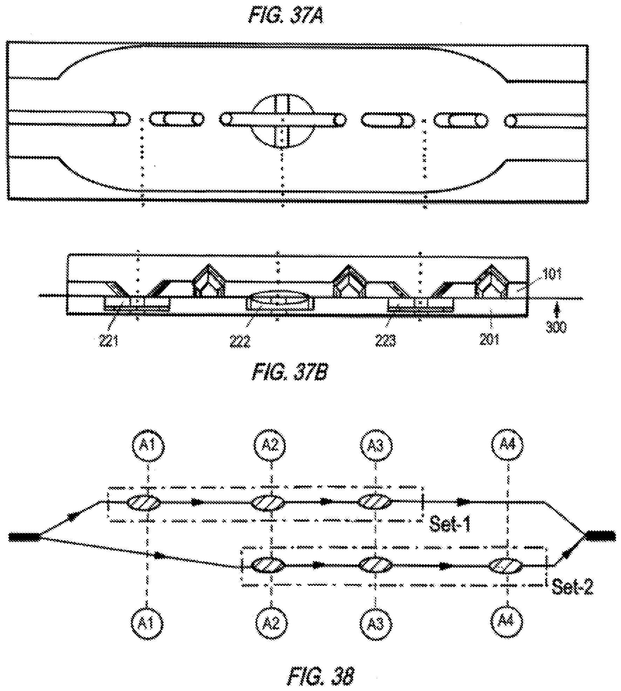

Pneumatically controlled fluid flow in the fluidic plate is generally achieved via a three-chamber unit e.g., **220a, 220b,** or **220c** of Figure 4. Figures 37A and 37B illustrate the details of an exemplary three-chamber unit containing a pump in the center and two valves, each on a side. When actuated sequentially, this valve arrangement can provide directionality in flow by preventing backflow while allowing for forward fluid displacement. The well-characterized, reliable valve-pump-valve units provide fixed strokes of fluid, which generate deterministic fluid flow. This supports a broad, dynamic pumping range between about 1 µL/day and about 10 mL/minute. In some embodiments, one or more or all of the pumping channels have reversible flow, supporting priming, sampling, and/or media/drug delivery configurations.
Generally, the pneumatic layer uses a pass-through design, where air-conducting actuation lines with air inlets and air outlets **210a** and **210b** (entry and exit being relative to the orientation of the plate) pass horizontally through the pneumatic plate, preferably in straight paths. Straight paths of air-conducting actuation lines occupy less of the total platform footprint, and they support a faster pneumatic response (e.g., fast pressure change due to a low volume). Symmetrical air inlets and air outlets allow platforms to be daisy chained to run simultaneously, connecting the outlets of one plate directly to the next with short lengths of tubing.
The pneumatic manifold generally employs a single bonded layer of material that allows for the creation of internal pneumatic channels. The pneumatic actuator membrane is generally a single layer polymeric material, e.g., polyurethane, that may be pressed between the pneumatic plate and the fluidic plate, or attached to one of the plates. The fluidic side in this case contains fluidic channels with micron range resolution geometries that allow for direction and evacuation of fluid. Higher resolution of the fluidic channel generally leads to a slower speed of fluid movement, but it may allow for smaller death volumes.

4-Lane dual-channel pump

In addition to the valve-pump-valve (V-P-V) pneumatic actuation configuration, a pump-pump-pump (P-P-P) configuration can be added to allow for a peristaltic movement of fluid.

Two or three sets of the three-chamber units may share one or two air-conducting actuation lines, as shown in Figure 38. When a fluidic channel (of the fluidic plate) splits into two channels that are pneumatically regulated by both a set of V-P-V pump and a set of P-P-P pump, which are placed one actuation line off and are 180° out-of-phase, the overall fluid combined from these two pulsating strokes has a smooth volume profile. Four actuation lines for these two sets of pumps accounts for four degrees of freedom, which requires only one more pneumatic line than the V-P-V configuration.

One or more x-chamber units (x>=3) may be placed with one or more air-conducting actuation lines off, in a similar principle to that shown in Figure 38, to have a customized smoothness of flow volume.

Modular pumping

Independent pumping allows for a different, e.g., higher, flow rate than that offered by the shared pumps. The incorporation of the fluid wells into the plate can reduce or eliminate the need for tubing, but the pump designs can be amenable to driving external flows as well. Connections, such as ferrule connections, can be used to interface the built-in pumps with external tubing, allowing a pumping manifold to drive a large number of flows simultaneously and in a compact package.

### Pump block for single-pass or recirculation perfusion

The top, or fluidic layer, contains the MPS compartments and the pumps and channels that interconnect them. Below the fluidic layer, a thin membrane such as a polyurethane membrane provides a sealing surface for the channels and functions as the actuation layer for the pumps. The bottom layer is a manifold (e.g., an acrylic manifold) that provides pneumatic actuation of the pumps by routing compressed air to the base of each pump chamber. When vacuum is applied, the membrane is pulled down toward the pneumatic layer, filling the pump with fluid. Conversely, when pressure is applied, the membrane is forced up into the fluidic plate, driving fluid out of the pump. By actuating three chambers in series, a fixed displacement peristaltic pump is formed, allowing fluid to be moved linearly and against head pressure without backflow (Domansky K, et al. Lab Chip 10(1):51-58 (2010); Walker I, et al. Journal of Micromechanics and Microengineering 17(5):891 (2007)).

### Geometry to reduce membrane stress

Different geometries of the pump other than one shown in Figure 37B may be used. An alternative form includes designs where the horizontal channels connecting the pump to the valves has been removed, leaving only the V-shaped connection that directly links two adjacent chambers. The rational behind these V-geometries is that these features pneumatically isolate one chamber from the other when the membrane deflects such that when one valve is actuated, its adjacent valve doesn't respond. Alternative configurations of pump geometry may reduce membrane stress and increase longevity of the actuation system and its consistency.

In some embodiments, further modifications to pump cavity geometries are created to render one concave contact and one convex contact between the membrane and the different V-shaped bridges, such that to prevent membrane deformation and breakage.

### Validation of pumping

Parity between the intended and actual flow rates enables wellmixing and intended molecular biodistribution among MPSs on a platform. Validation of the hardware may include direct measurements of pump rates using a capillary flow measurement tool. In some embodiments, the tool is interfaced with the outlets in each MPS compartment such that flow may be measured as a function of time required to fill a fixed length of tubing. Deviations of flow rates from one fluidic plate to another may be attributable to slight machining differences in the depth of the pump chamber. Nevertheless, software calibration factors calculated from the measurements may be entered to correct the pumping rates to within about ± 5%, ± 4%, ± 3%, ± 2%, or ± 1% of the target flow rates to adjust individual pumps. Generally, a small margin of error still allows for reliable and deterministic operation, and accurate data interpretation.

### (9) Means for non-contact fluid level sensing

Capacitive sensing with a three-electrode design

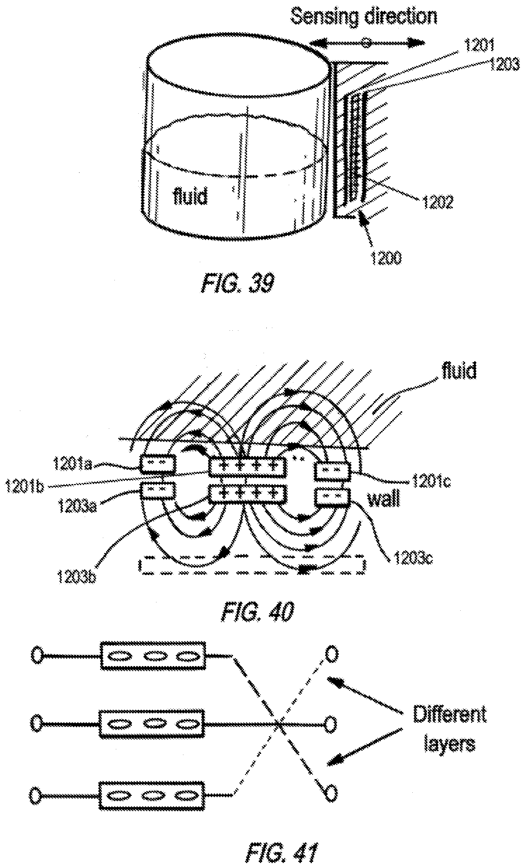

The fluid level in a MPS well may be measured in a non-contacting manner using capacitance sensing. Electric charges go through plastic, such that probe can be placed next to the well but not in contact with the media/culture of the well to avoid possible contamination. A capacitive sensing probe may be embedded in the wall material of wells (e.g., made from plastic). The circuit senses capacitance through the wall without fluid contact. Capacitive sensing electrodes sit behind the layer of plastic isolated from fluid. As shown in Figure 39, front electrodes **1201** measure capacitance close to the fluid, while back electrodes **1203** measure capacitance of plastic only (as reference). Front electrodes and back electrodes may be built on two sides of, therefore backed by, a polychlorinated biphenyl (PCB) board **1202** or a flex backing. The front electrodes have a sensing electrode in the middle and two reference electrodes, one on each side, which are coplanar to the central sensing electrode (Figure 40). This organization of reference electrodes and the sensing electrodes allows for good matching. Mirror opposing electrodes provide self-guarding.

Previous designs places one negative (reference electrode) conductive plate side-by-side and coplanar to the one positive (sensing electrode) in an attempt to measure liquid level from within the well wall in a non-contact manner. This causes the reference capacitor, Cref, to be in the wrong place and results in inaccurate measurement of fluid level.

The apparatus utilizes an improved design containing three electrodes of symmetry, i.e., two reference electrodes coplanar to and symmetrical about a central sensing electrode, coupled with a mirror set of electrodes on the back side of a PCB board. This results in Cref in the right place for self-guarding and better matching.

### Closed-loop feedback controlled system and its operation

Generally a closed-loop feedback-controlled pump system for mesoand microfluidic applications includes (1) at least one pump, capable of dispensing fluid from at least one fluid supply reservoir, (2) a non-contact fluid level sensor, capable of sensing fluid height differences in the order of micrometers or millimeters over an extended period of time, as well as converting the fluid height data to digital signals, and (3) a control unit, capable of processing the signal, sending orders, and/or executing steps to alter the amount of fluid in the fluid reservoir and/or the flow rate of fluid dispensed therefrom based on external/user input, to maintain the amount of fluid in the fluid reservoir and/or a steady flow rate of fluid dispensed therefrom based on signal collected from the non-contact fluid level sensor, or both.

In some embodiments, a closed-loop feedback-controlled pump system includes a gravity-driven pump having a fluid supply reservoir, and is connected to at least one fluidic channel of a meso- and/or microfluidic device, where the pump supplies controlled fluid flow that is decoupled from pressure changes in the gravity-driven pump. In a preferred embodiment, the pump has at least one gravity-dominated fluid supply reservoir to induce fluid flow through hydrostatic pressure into and/or from the fluidic device being driven.

Generally an auxiliary, additional fluid reservoir is fluidically connected to the fluid supply reservoir (also called the main driving reservoir). When fluid is dispensed from the supply reservoir, the non-contact fluid sensor senses the reduction of fluid height in this supply reservoir and signals the control unit to induce fluid from the auxiliary fluid reservoir to flow into the supply reservoir in order to maintain, establish, or re-establish a set fluid height in the pump, thereby allowing for a steadystream fluid flow from the pump. This creates an automated fluid sensing and feedback control system.

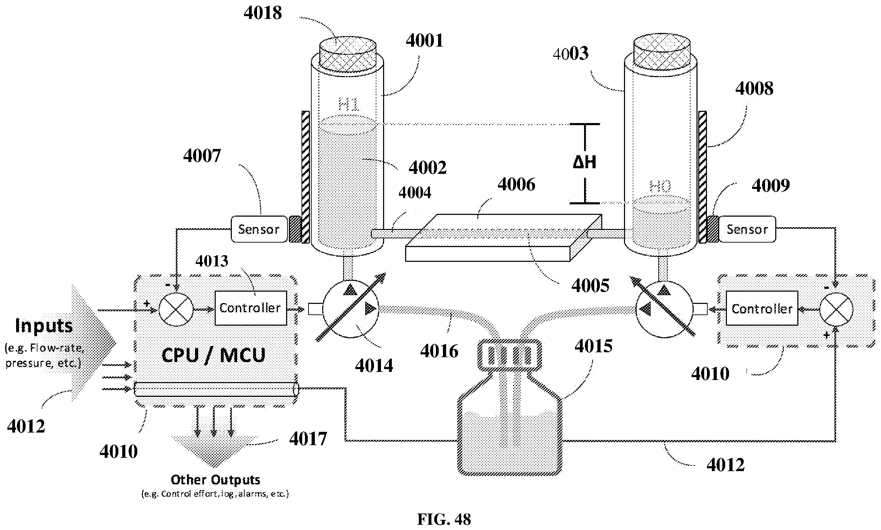

Figure 48 shows an exemplary bidirectional feedback controlled, closed-loop gravity-driven pump system to induce fluid flow through a microfluidic channel. This gravity-driven pump contains two hydrostatic pressure chambers, i.e., two driving/supply reservoirs of fluid. A first driving reservoir **4001** contains fluid **4002** to a desired height (H1), and a second driving reservoir **4003** maintains fluid at another fluid height (H0). The height difference (ΔH) between H1 and H0 drives gravity-dominated fluid flow through an outlet and/or connector **4004,** which is connected with at least one channel **4005** in a meso- and/or microfluidic device **4006.** The fluid height of each of the reservoirs is monitored by a non-contact fluid-level sensor assembly unit including a transducer **4007,** a set of electrodes also referred to as a sensing arrangement **4008,** and a sensing interphase **4009.** Acquired fluid height signal is fed to a computing unit **4010** generally including at least one computer processing unit (CPU) or a microcontroller unit (MCU), capable of performing a comparison with respect to a reference signal 012 (e.g. user input). Closed-loop feedback in the system is based on the results of this comparison, the computing unit **4010** implements a desired control scheme **4013** to drive a fluid input and/or output into or out of either one of the driving reservoirs to control fluid heights H1 and/or H0. This is achieved via a bidirectional piezoelectric pump or a peristaltic pump **4014** for each of the driving reservoir to be controlled, and the fluid to be added to or extracted from the driving reservoir is obtained, stored, and/or recirculated using an additional fluid reservoir **4015** which is fluidically connected (e.g., through a tubing **4016)** to the system. In this way, controlled fluid heights in the driving reservoirs reduces significant pulsatility of flow at the outlet of a driving reservoir or the connector **4004.** The main output of the feedback controlled, gravity-driven pump system is the controlled gravity-driven flow and pressure profile imposed in a decoupled manner to at least one microfluidic channel **4006.** Other outputs **4017** from the system can potentially be transmitted out from the CPU/ MCU to augment the overall functionality of the system. For fluid sterility purposes, an open driving reservoir for fluid includes a mechanical barrier **4018** (i.e. air filter) to prevent contamination while still allowing for a desirable gas/fluid interface capable of acting as a bubble trap for the gravity-driven pumping system.

Another embodiment is a unidirectional, feedback controlled closed-loop gravity-driven pump system which induces flow through a microfluidic channel. This gravity-driven pump system contains one hydrostatic pressure chamber, i.e., one driving (fluid supply) reservoir filled with fluid at a height (H1). The fluid outlet is near the bottom of this driving reservoir. The height difference (∆H) between H1 and the fluid outlet drives gravity-dominated flow out from the outlet, which through a horizontal connector is connected with at least one channel of a meso- and/or microfluidic device positioned at or near the height of the outlet and/or connector. Although the connector may be tilted or non-horizontal, generally the connector is preferred to be horizontal such that the entrance of fluid to the microfluidic device is the same height as the fluid outlet of the driving reservoir, i.e., the connector provides horizontal fluid flow from the outlet of the driving reservoir to the entrance of the microfluidic device.

After fluid flows through the microfluidic channel, an outlet of the channel may be opened to atmospheric pressure; alternatively or additionally, an outlet of the fluid flown through the microfluidic channel may be in an additional fluid reservoir for recirculation for the system. Recirculation in addition to the open-air outlet can be controlled through a fluid manifold. The fluid height in the driving reservoir is monitored by a non-contact fluid-level sensor assembly including a transducer, a sensing arrangement (e.g. optimized capacitance measurement electrodes), and a sensing interphase between the sensing arrangement and the transducer. Acquired fluid height signal is fed to a computer processing unit (CPU) or microcontroller unit (MCU) containing a unit for performing a comparison with respect to a reference signal (e.g. desired user input). Based on the result of this comparison, the computing unit implements a desired control scheme via a controller to drive a mechanism (i.e. bidirectional piezoelectric pumps, peristaltic pumps, etc.) of fluid input and/or output to alter or maintain fluid height H1 in the driving reservoir without adding significant pulsatility at the outlet. Added or extracted fluid from the driving reservoir is obtained, stored, and/or recirculated using the additional fluid reservoir connected to the system through fluidic connections. The main output of the feedback controlled, gravity-pump system is the controlled gravity-driven flow through the microfluidic channel which exits at the selected microfluidic outlet. The flow rate provided by this configuration of the pump is related to the height of the column of fluid (e.g., liquid) in the reservoir, so the flow rate is coupled to the pressure in the system. Other outputs from the system can be transmitted out from the CPU/ MCU to augment the overall functionality of the system. For fluid sterility purposes, the open driving reservoir may include a mechanical barrier (i.e. air filter) to prevent contamination while still allowing for a desirable gas/fluid interface capable of acting as a bubble trap for the gravity-driven pumping system.

**1. Pump**

Any pumping mechanism can be coupled with the non-contact fluid height sensor as detailed below to provide a closed-loop, feedback controlled system for fluid sensing and handling at a small scale, which is further integrated with a microfluidic device of choice. Preferably, a gravity-driven pump is coupled with a capacitive fluid height/level sensor to establish a closed-loop, feedback controlled system.

One closed-loop, feedback controlled system may contain one or more pumps, preferably at least one gravity-driven pump. A gravity-driven pump may contain one, two, or more fluid supply reservoirs in order to permit unidirectional, bidirectional, or other formats of fluid flow directionality.

*Fluid supply reservoir*

A fluid supply reservoir is part of, or the whole of, a gravity-driven pump to induce fluid flow to a connected microfluidic channel, well, or device or another vehicle.

In a gravity-driven fluid supply reservoir, the fluid height is correlated with the hydrostatic fluid pressure at an outlet of this reservoir, according to formula (1).

$$P = \rho \times g \times h \qquad (1)$$

Where p is the density of the fluid, g is acceleration due to gravity, and h is the height of the column or tower of fluid in the fluid reservoir from the outlet or from a depth where the hydrostatic pressure is calculated.

Gravitationally dispensed fluid through this outlet can flow into a meso- and/or microfluidic device that is connected to the outlet of the pump. The outlet of the gravity-driven pump is preferably located at the bottom or near the bottom of a fluid reservoir, but can be located at any position that allows for the generation of desired hydrostatic pressures to drive flow from and/or to this reservoir. The outlet of the gravity-driven pump may have a round, square, or any other shape that allows for leak-proof fluidic connection to a microfluidic device or other devices directly or indirectly, e.g., through a tube and optionally with an adaptor. The outlet of the gravity-driven pump may have a size as desired.

The fluid reservoirs, the connector and fluidic devices can be made of a variety of materials including, but not limited to, polydimethylsiloxane elastomer ("PDMS"), polysulfone, polyurethane, cyclic olefin copolymer (COC) or glass.

In some embodiments, fluid reservoirs are made of materials with electrical permittivity constants of at least one order of magnitude less than that of the fluid to be measured (which is approx. 80 for water at 20°C), coupled with capacitive fringing as the mechanism of sensing.

*Auxiliary fluid reservoir*

For replenishing the fluid supply reservoir and/or temporary storage of fluid flown out from the supply reservoir, an auxiliary reservoir is generally connected to the supply reservoir and a valve or a pump that is operated by a CPU or microcontroller unit.

*Filter/barrier*

In some embodiments, a mechanical barrier (e.g., an air filter) is added to the pump to prevent contamination while still allowing for a desirable gas/liquid interface capable of acting as a bubble trap for the gravity-driven pumping system. Due to the small channel dimensions in most microfluidic systems, the presence of large bubbles can plug flow and prevent adequate operation of these devices. Thus, the bubble trapping feature imparted by a mechanical barrier allows for smooth operation of the gravity-driven pump system for various applications.

**2. Non-contact fluid height sensor and its assembly**

Preferably, the fluid height at the driving (fluid supply) reservoir is monitored using a non-contact fluid level sensor. The signal from the sensor is fed to a processing unit and/or a control system capable of driving fluid flow from a secondary reservoir to modify the fluid height at the main reservoir. The sensor is generally set with a reference signal of a desired fluid height corresponding to a desired hydrostatic pressure at the reservoir outlet. Fluid into the driving reservoir is generally induced, monitored, and maintained at a desired fluid height as measured continuously or periodically by the sensor.

The sensor and the response unit (e.g., processing unit and/or a control system) permit a closed-loop feedback of a gravity-dominated pump to compensate for fluid height changes to avoid or minimize undesirable transient flows caused by liquid level decrease in the pump. They also permit full dynamic control of deliverable flow and pressure profiles.

In some embodiments, the flow induced by the gravity-driven pump has a constant flow-rate. The closed-loop feedback control of the pumps allows for the use of smaller driving reservoirs and smaller fluid volumes due to a recirculation setup.

In some embodiments, the flow induced by the main reservoir/pump is a transient flow, as monitored by the non-contact fluid level sensor. For example, the flow induced by the main reservoir/pump is directed to a medium, e.g., a biomaterial such as hydrogel, where hydraulic permeability changes over time. The sensor can monitor this change and act as a reporting tool of imposed fluidic conditions. For a microfluidic channel/medium positioned at a height position $h = 0$ compared to the column of fluid in the fluid supply reservoir, there is no backflow to the gravity-driven pump absent external force or pressure to the system.

In a preferred embodiment, the non-contact fluid height sensor is a capacitive sensory system. A capacitive sensing probe may be embedded in the wall material of wells (e.g., made from plastic). A capacitive sensory system provides closed-loop feedback functionality to a gravity-driven pump. Although capacitive sensors for assessment of fluid height in reservoirs containing large fluid volumes (with > 100 mL) have been described, capacitive fluid level sensing to smaller reservoirs (with < 100 mL) is not a straightforward extension from previous sensors and electrode arrangement, because they do not provide enough sensitivity for measuring small fluid height increases in meso- and microfluidic reservoirs using commercially available instrumentation like capacitance-to-digital converters (CDC). Capacitive liquid level sensing operates independently of specific gravity, conductivity, or viscosity (Baxter, L.K., 1996. Capacitive Sensors: Design and Applications, vol. 1 of IEEE Press Series on Electronics Technology); and requires minimal instrumentation.

Typically, a capacitive sensory system includes a transducer, an electrode arrangement, and a sensing interphase. Fluid to be sensed/measured in height changes by a capacitive sensor has a dielectric constant that is significantly different from that of the air and of any material used to contain/reserve/hold the fluids. For example, air has a dielectric constant around 1.0, and the dielectric constant of most aqueous solutions ranges from 50 to 80, suitable for capacitive sensing in most meso- and/or microfluidic applications. The capacitive sensory system for measuring fluid height in micro- or mesofluidic devices is capable of detecting height changes in the orders of centimeters, millimeters, or microns. For example, the capacitive sensory system is capable of resolving fluid height differences or changes as small as 1 cm, 100 mm, 10 mm, 1 mm, 100 $\mu$m, 10 $\mu$m, or 1 $\mu$m. Typically, the range of the measured capacitance is from -4 pF to 4 pF, preferably around $\pm$ 0.1 pF, more preferably about 0.1 pF. The conversion time of the capacitive sensor ranges from 0.1 to 100 milliseconds. With the conversion, a dynamic range of 21-bit digital signal is achieved for a capacitance between -4 pF and 4 pF.

*Electrode arrangement*

For capacitive sensing, two or more conductive coplanar plates are positioned to measure and acquire the fringing capacitance associated with the medium or media around the plates (Li, X.B., 2006. IEEE Sensors Journal, 6(2), pp.434-440). The capacitance measurement changes proportionally to the height of the fluid in close proximity to the electrodes.

The electrode arrangement has a sensing arrangement and a parallel, back arrangement for shielding to enhance accuracy.

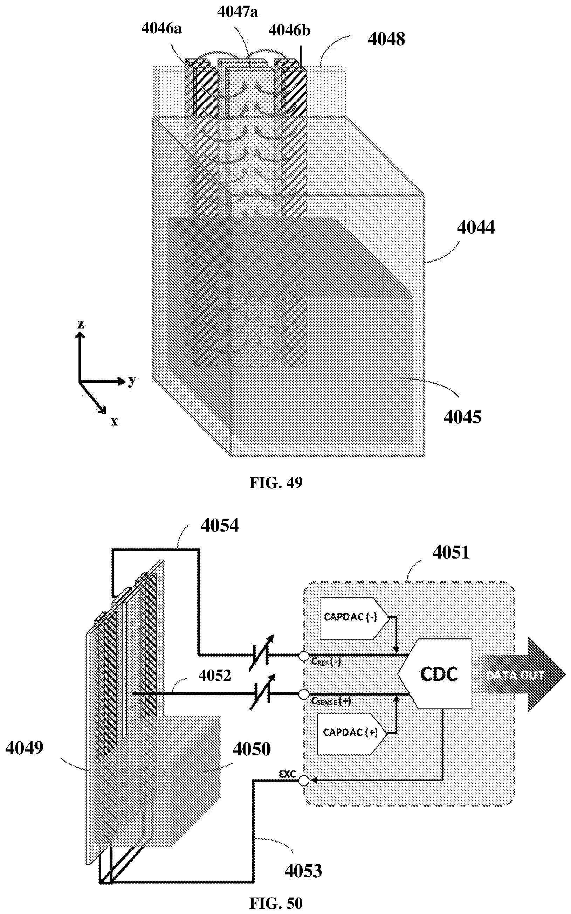

Figure 49 illustrates an exemplary arrangement of electrodes. This arrangement allows for high sensitivity of fluid sensing in applications with small fluid volumes (tracking volumes <100ml), which is particularly suitable for gravity driven pumping systems for meso- and microfluidic devices as shown in Figures 1 and 2. In this arrangement, the fluid **4045** to be measured is contained in a reservoir **4044** of a constant, arbitrary x-y cross-sectional area situated in close proximity to the capacitive sensing electrode arrangement (e.g., distance <10 mm including about 9, 8, 7, 6, 5, 4, 3, 2, 1 mm). The fluid reservoir **4044** is generally made of materials with electrical permittivity constants of at least one order of magnitude less than that of the fluid to be measured (which is approx. 80 for water at 20°C). Two coplanar excitation electrodes **4046a** and **4046b** are each positioned at one side of a coplanar sensing electrode **4047a** to drive fringing capacitance in the direction of the arrows. Two symmetrical gaps made of air or a dielectric material separates the side excitation electrodes **4046a** and **4046b** from the central sensing electrode **4047a.** The distance of this specific gap can be increased or reduced to modify the penetration of the most sensitive fringing pathways in this arrangement. Generally the gap is in the range of 0.1 to 5 mm.

To further improve the signal-to-noise ratio of this sensing configuration, a mirrored arrangement of electrodes (facing an air or dielectric filled region) is placed parallel to the primary sensing arrangement. The primary sensing arrangement faces the fluid and is separated from the fluid by only a small thickness of reservoir material, e.g., 0.1 to 5 mm thickness. The reservoir is generally made with materials having a small electrical permittivity at room temperature under 1kHz excitation between 1 and 20. This parallel back arrangement is separated from the primary sensing electrode arrangement by a thin dielectric (thickness preferably < 3 mm; may be 10, 9, 8, 7, 6, 5, 4, 3, 2, 1 mm or smaller thickness). Alternatively, the primary sensing arrangement and the parallel back arrangement are built on two sides of, therefore backed by, a polychlorinated biphenyl (PCB) board or a flex backing. The parallel back electrode arrangement provides self-shielding or guarding to the primary sensing electrode arrangement, and allows for suitable reference for noise-resistant differential capacitance readings. Preferably the widths of excitation track electrodes are similar (e.g., within 15% difference from one another); the width ratio of each sensing electrode to each excitation electrode is preferably around 2:1, but can be generally 1.2:1, 1.4:1, 1.6:1, 1.8:1, 2.5:1, or greater. In Figure 49, the width refers to the y direction. The length of the sensing electrode arrangement can be arbitrarily defined to meet variable fluid height ranges. In Figure 49, the length refers to the z direction.

Figure 50 illustrates a flow diagram functionally connecting among the capacitive sensing electrode arrangement **4049** described in Figure 49, the sensed fluid height **4050,** and the standard electrical terminals of commercially available capacitance-to-digital-converters **4051,** forming the basis for a fluid level sensing and feedback system for a gravity-driven pump for meso- an/or microfluidic devices. The primary sensing electrode is connected to terminal $C_{sense}(+)$ via planar electrode **4052,** and the coplanar side excitation electrodes of the primary sensing arrangement and those of the parallel reference arrangement are connected to the excitation terminal **4053**. In some embodiments, a differential capacitance reading is desired for noise reduction, the back sensing electrode in the parallel reference arrangement is connected to a negative capacitance reading terminal $C_{REF}(-)$ via planar electrode **4054.**

Governing equations can be easily derived for coplanar electrode arrangements in a capacitive sensing mode. For this specific configuration, the fluid level ($Level_{Fluid}$) can be approximated as shown in formulae (2)-(4),

$$Level_{Fluid} \approx C_{sense}(+)/C_{REF}(-), \qquad (2)$$

$$C_{sense}(+) \approx \varepsilon_0 \varepsilon_R \cdot (Level_{Fluid}) \cdot w/d, \quad (3)$$

$$C_{sense}(-) \approx \varepsilon_0 \varepsilon_R \cdot (L_{Ref}) \cdot w/d. \qquad (4)$$

$Level_{Fluid}$ is the length of the sense electrode in a closed proximity to fluid, $\varepsilon_0$ is the electrical permittivity of free space in vacuum, $\varepsilon_R$ is the electrical permittivity of the fluid, $L_{Ref}$ is the length of the reference electrode, w is the sensing electrode width, and d is the length of the mean fringing arc created between the excitation and sensing electrodes. This sensing arrangement is self-shielding, providing a substantial improvement over previously described capacitive sensing electrode arrangements and thereby allowing for small fluid change measurement in fluid reservoirs of a reduced volume.

Figure 51 illustrates a cross-sectional detail (in the x-y plane) of the electrode arrangement described in Figures 49 and 50. Figure 51 shows an excitation-sensing-excitation (ESE) inter-digitating electrode design with a primary sensing arrangement facing (electromagnetic fringe penetrating) the measured fluid **4055** and a parallel, back electrode arrangement facing the surrounding environment **4056** (e.g., the wall of the reservoir). The symmetry axis **4057** on this projection delimits where the largest capacitance fringes meet, i.e., at the center of the primary sensing and/or reference electrodes. Charge polarization **4058** on the excitation electrode and charge polarization **4059** on the sensing electrode are as shown. The electromagnetic fringing **4060** of this electrode arrangement supports the self-shielding capabilities where parasitic capacitances running through the thin inter-electrode dielectric **4061** are minimized or entirely eliminated. This electrode arrangement can be placed directly in contact with the reservoir wall dielectric material **4062** or through an air-dielectric interphase.

Figure 52 illustrates an embodiment of the capacitive fluid-level sensor depicted in Figure 50 and adapted for traditional and flexible printed circuit board manufacturing. In this preferred sensor embodiment, a front region **4063** and a back region **4064** for electronic component placement are each electrically connected to excitation electrodes **4065a** and **4065b** and the sensing electrode **4066**. This sensor can be of any arbitrary length at either the electrode level or the connector level, or both, which is denoted by the double wave symbol **4067**. This sensor can be connected to a CPU/MCU through electrical connections **4068** (e.g. ZIF connector or otherwise). An electronic schematic of this embodiment is shown in Figure 9 of U.S. Application No. 62/556,595 including a commercially available capacitance-to-digital converter chip (e.g. AD7746) as well as required lines, resistors and capacitors for supply and communication.

*Sensing interphase*

A sensing interphase refers to the medium or space in between the conductive material of the excitation or sensing electrodes and the fluid to be measured. A capacitive fluid level sensor electrode can be placed directly in contact with the reservoir wall dielectric material (with the wall material being the interphase) or through an air-dielectric interphase.

*Transducer*

A capacitance-to-digital converter converts to digital signals an acquired capacitance measurement that changes proportionally to the height of the fluid in close proximity to the electrodes (U.S. Patent No. 7,129,714). A CDC sensor can provide a continuous fluid-level measurement.

Other types of transducers include ultrasonic transducer, analog-to-digital converter.

## 3. Additional pump or valve

To replenish fluid in the supply reservoir and/or direct fluid recirculation, an additional pump or valve is included in the system. For example, a piezoelectric pump or a peristaltic pump is used under the control by the CPU or microcontroller to direct fluid flow from an auxiliary reservoir to the fluid supply reservoir, or to direct fluid flow from the exit of a microfluidic channel, well, or device to the auxiliary reservoir, as shown in Figure 48. Piezoelectric pumps are described in U.S. Patent No. 3,963,380 and US patent No. 3,803,424. Peristaltic pumps are described in U.S. Patent Nos. 5,482,447 and 6,213,739 and US 20140079571 and US20160051935.

## 4. A control unit and the operation

As an exemplary illustration, Figure 3 of US. Application No. 62/556,595 shows the assembly and the operation of the sensors for fluid height in a driving reservoir and the closed-loop feedback control. An input reference signal, defined in terms of desired instantaneous flow rates, dynamic profiles, and/or output pressures, is compared to the measurement from a non-contact fluid-level sensor to generate an error signal e(t). This error signal is then compensated by a controller with transfer function C(s) using at least one of the N number of fluidic input and/or output mechanisms connected to a fluidic plant. This fluidic plant may be a gravity-driven pump itself and/or include at least one fluid reservoir connected, meso- and/or microfluidic device, all of which can be described through transfer function P(s).

The closed-loop feedback control system may operate under controlled constant pressure profile despite a dynamic flow rate profile, showing the flow decoupling functionality of a closed-loop gravity driven pump. The system may operate under controlled constant flow rate profile despite a dynamic pressure profile, showing pressure decoupling functionality of a closed-loop gravity driven pump.

Feedback control

Measurements of a fluid level in a fluid supply reservoir can be transmitted to the control unit that regulates the inflow of liquid replenished into the fluid supply reservoir. Feedback of the instant fluid level in the supply reservoir facilitates adjustment to control of a set-point hydrostatic pressure or flow rate from the reservoir.

Control software is configured to be instantiated/installed on or with a microcontroller (e.g., a NATIONAL INSTRUMENTS MYRIO microcontroller) or a processing unit to allow for continuous operation of fluid handling in the closed-loop, feedback controlled system. Although there may be no need for a dedicated laptop or desktop computer, it is to be understood that some embodiments may utilize such a dedicated computer. A software operates the feedback controlled pumps for the purposes of: fluid replenishment and/or mixing for the supply reservoir; introduction of external fluid to the closed-loop, feedback controlled system (feeding); removal of fluid from the closed-loop, feedback controlled system (sampling and/or waste collection); and/or dosing of test compounds, growth factors, drugs, or other chemicals/proteins of experimental interest (dosing).

In some embodiments, there is also the capability to add an alarm for drift of hydrostatic pressure (or fluid height). In some embodiments, long-term data logging is implemented.

In some embodiments, individual and global correction factors are incorporated in the software to allow correction for variability in the system. By determining a correction factor (iteratively and/or experimentally), the rate of fluid handling can be tuned to be very exact, where pumps were measured, calibrated, and re-measured to target operability. The software correction factors improve the performance of the pumps and minimize manufacturing variations across platforms.

In some embodiments, the microcontroller is WiFi compatible. The software can be configured with a web UI and backend (e.g., LabView backend) to control the pumps. This allows the user to access the control panel of the software in a web browser without having to connect physically to the microcontroller, allowing remote control and monitoring of experiments from across the room or across the world.

An exemplary information flow from user to output includes the following. A user accesses webUI over the local network or via VPN remotely. Control changes are passed from the WebUI to the backend, which adjusts the fluid level in the supply reservoir, as confirmed and monitored by the non-contact fluid height sensor, to meet the desired flow rates (accounting for individual and global pump calibration factors) into a microfluidic channel, well, or device. The microcontroller then outputs a digital on/off signal to a control board that amplifies that signal to direct replenishment of the supply reservoir and/or recirculation of the fluid.

In some embodiments, there is a debug mode that allows manual operation of every single pump, for the purposes of finding malfunctioning pump or manually opening/closing individual pumps and valves in the system.

Also, the feedback controlled system for fluid manipulation through a microfluidic device may have one or more input and output devices, including one or more displays. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computer may receive input information through speech recognition or in other audible format.

Such computers may be interconnected by one or more networks in any suitable form, including a local area network or a wide area network, such as an enterprise network, and intelligent network (IN) or the Internet. Such networks may be based on any suitable technology and may operate according to any suitable protocol and may include wireless networks, wired networks or fiber optic networks.

The various methods or processes outlined may be coded as software that is executable on one or more processors that employ any one of a variety of operating systems or platforms. Additionally, such software may be written using any of a number of suitable programming languages and/or programming or scripting tools, and also may be compiled as executable machine language code or intermediate code that is executed on a framework or virtual machine.

In some embodiments, the software to operate the closed-loop feedback controlled system is embodied as a computer readable storage medium (or multiple computer readable storage media) (e.g., a computer memory, one or more floppy discs, compact discs, optical discs, magnetic tapes, flash memories, circuit configurations in Field Programmable Gate Arrays or other semiconductor devices, or other non-transitory medium or tangible computer storage medium) encoded with one or more programs that, when executed on one or more computers or other processors, perform methods that implement the various embodiments of the invention discussed above. The computer readable medium or media can be transportable, such that the program or programs stored thereon can be loaded onto one or more different computers or other processors to implement various aspects as discussed above.

Computer-executable instructions may be in many forms, such as program modules, executed by one or more computers or other devices. Generally, program modules include routines, programs, objects, components, data structures, etc. that perform particular tasks or implement particular abstract data types. Typically the functionality of the program modules may be combined or distributed as desired in various embodiments. Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

Optical measurement

Light illumination of the fluid surface may indicate the depth of liquid in a well.

Pressure sensing

In some implementations it may be advantageous to use a pressure sensor to determine the height of fluid in a well. This is possible given the well-known relationship between pressure and height given by $P = rho * g * h$, where $P$ is pressure, rho is fluid density, $g$ is the acceleration of gravity, and $h$ is the fluid height. A pressure sensor of known types in the art may be incorporated in fluidic connection to a well such that the pressure sensor is measuring pressure in the well at a known reference height.

Feedback control of pumps for volumes and flow rates Measurements of a fluid level in a well can be transmitted to the control unit that regulates the flow rates of liquid pumped into one or more MPS wells. With a capacitive sensing measurement of the fluid height in a well, the volume of liquid in that well can be calculated with a known surface are of the well. A real-time measurement of a fluid level therefore provides information of the volume flow rate (i.e., difference in the fluid heights over the period of time). The feedback facilitates control of a set-point volume and pumping flow rate to MPS wells.

**(10) Means for temperature and pressure sensing and control**

Temperature is controlled by placing these platforms in an incubator, which maintains the global temperature within to $37 \pm 0.5$ deg. Pressure sensing can be done with any of the static pressure sensing sensor types known in the art and give an indication of fluid height in the wells. Incorporating sensors to measure the well fluid height, using capacitive fluid level sensors or pressure sensors, in a feedback loop can help in actively controlling the well fluid volumes.

**(11) Assembly of integrated components**

Securing the pneumatic side with the fluid side

In some embodiments, bolting through alignment pins may be used as the means to assemble the pneumatic side with the fluid side of the bioreactor. Insufficient sealing may result in fluid leakage.

Clamping may be used as an alternative means for securing the pneumatic side with the fluid side of the bioreactor. Mechanical, as well as magnetic, clamps may be used to clamp the fluidic plate, the actuation membrane, and the pneumatic plate together.

Whippletree pressure distribution mechanism may be utilized in distributing pressure across different air actuation lines or across platforms.

Daisy chain of multiple bioreactors

In some embodiments, two or more multi-organ MPS platforms are chained one after another at the air inlets and outlets. With the pass-through, straight-path design of air-conducting actuation lines across the pneumatic plate, two or more platforms share pneumatics and a same set of controller. No additional hardware is needed to scale up the number of platforms in a group. With symmetrical air inlets and outlets on each bioreactor, daisy chaining is easy to set up and disassemble. This feature saves time, cost, and space for operating several bioreactors/MPS platforms at a same time.

Multilayered organ-on-chip fluidic and pneumatic plates

Figure 41 illustrates a criss-cross design of pumping system for multilayer stacking configurations of platforms.

Multilayered organ-on-chip plates may be assembled via internal channels, made by either bonding of independent layers or 3D printing. The connections between pumps can overlap for this higher density of fluidic plates. It is also compatible with different pumping and valving configurations (e.g., such as those described in pneumatic actuation). The ability to have multilayered plates enables internal channels, which may replace the V-cuts in the valves, which reduces the pressure spike due to valve operation and improves the performance for more deterministic pumping profiles.

Multilayered plates may have several benefits over single-layer clamped plates. Higher density of pumps and channels generally allows for better sealing, reduction of overall device footprint, no cleaning needed for disposable manifolds, and an increased capability to multiplex controls with crossing channels. It is also easier to divert channels around areas where imaging is desired, or where sensors need to be inserted for measurement, in a multilayer plate configuration that the single-layer chain configuration. It provides more freedom in the layout of culture wells and the capability to incorporate new pump configurations.

External connection

The multi-layer bioreactor apparatus may be connected to a microcontroller and an external pneumatic solenoid manifold to provide a source of pumping from outside. For incubation of the bioreactor apparatus, a pneumatic solenoid manifold is connected that controls 36 or a customized number of channels of tubing running through the back of the incubator to intermediary connectors. Inside the incubator, tubing is attached to the platform/bioreactor through valved breakaway couplings to allow easy removal from the incubator for media changes and sampling. The connectors and software architecture allow the setup to be compatible with the 2-way, 3-way, 4-way, 5-way, 6-way, 7-way, or a customizable number of multi-organ platforms, as well as many future platform variants, with minimal modification to the software configuration. Pump flow rates and calibration factors are set through a graphical user interface on a laptop, and can be sent to a customized microcontroller (e.g., National Instruments myRIO-1900) over USB or WiFi. Both manual and pre-programmed control of pump rates are available depending on the experimental needs, and the microcontroller can run independently of the laptop.

In some embodiments, a multi-organ MPS platform is connected to a local reservoir between controller and pump. In other embodiments, it is connected to external microfluidic device for import of external supply and export of waste.

Computerized operation

Control software is configured to be instantiated/installed on or with an appropriate device, such a microcontroller (e.g., a NATIONAL INSTRUMENTS MYRIO microcontroller) to allow continuous operation of a physiomimetic platform without the need for a dedicated laptop or desktop computer, although it is to be understood that some embodiments may utilize such a dedicated computer. The software operates the pneumatic pumps contained in the platform for the purposes of: fluid replenishment and mixing (which provides nutrients and oxygen to the cells); introduction of fresh media from an internal or external source (feeding); removal of media to an internal or external collection vessel (sampling and waste collection); and/or dosing of test compounds, growth factors, drugs, or other chemicals/proteins of experimental interest (dosing).

By providing a graphical user interface for the control of mixing, feeding, sampling, and dosing, this software facilitates the execution of complex experiments meant to replicate physiological interaction of compartmentalized organs.

The components and/or software also provide real-time feedback from pressure and vacuum sensors integrated into the hardware, which can contain the microcontroller, pneumatic solenoids, pressure sensors, and/or power distribution electronics. In some embodiments, there is also the capability to add an alarm for drift of pressure and vacuum out of acceptable ranges, and long-term data logging of these values can be implemented.

In some embodiments, individual and global correction factors are incorporated in the software to allow correction for manufacturing variability in pumps on the platform. For example, two pumps operating at the same frequency (e.g., 2 Hz) will not always pump at exactly the same rate if one is machined slightly deeper than the other. By determining a correction factor (iteratively and/or experimentally), the rate of the pump can be tuned to be very exact, where pumps were measured, calibrated, and re-measured to target 1 μL/s at 2 Hz. The software correction factors improve the performance of the pumps and minimize manufacturing variations across platforms.

In some embodiments, the microcontroller is WiFi compatible. The software can be configured with a web UI and backend (e.g., LabView backend) to control the pumps. This allows the user to access the control panel of the software in a web browser without having to connect physically to the microcontroller, allowing remote control and monitoring of experiments from across the room or across the world.

An exemplary information flow from user to output includes the following. A user accesses webUI over the local network or via VPN remotely. Control changes are passed from the WebUI to the backend, which adjusts the timing of the solenoid actuation to meet the desired flow rates (accounting for individual and global pump calibration factors). The microcontroller then outputs a 3V digital on/off signal to a control board that amplifies that signal to a 12V analog actuation of the desired solenoids.

In some embodiments, there is a debug mode that allows manual operation of every single solenoid, for the purposes of finding malfunctioning solenoids or manually opening/closing individual pumps and valves of the platform.

Depending on different platform hardware design, the software is implemented in a number of different ways, including: 4-way platform software - controls pumping and calibration factors, displays pressure/vacuum data for 4 organ platform; 7-way platform software - controls pumping and calibration factors, displays pressure/vacuum data for 7 organ platform, a program mode to define automated flow rate changes over time, and automated feeding and sampling controls from external ports on the platform; 3xGL platform software - controls pumping and calibration factors, displays pressure/vacuum data for 3 organ platform, includes a program mode to define automated flow rate changes over time, controls automated feeding and drug dosing (controlled volumes) to organs.

Also, a computer may have one or more input and output devices, including one or more displays as disclosed herein. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computer may receive input information through speech recognition or in other audible format.

Such computers may be interconnected by one or more networks in any suitable form, including a local area network or a wide area network, such as an enterprise network, and intelligent network (IN) or the Internet. Such networks may be based on any suitable technology and may operate according to any suitable protocol and may include wireless networks, wired networks or fiber optic networks.

The various methods or processes outlined herein may be coded as software that is executable on one or more processors that employ any one of a variety of operating systems or platforms. Additionally, such software may be written using any of a number of suitable programming languages and/or programming or scripting tools, and also may be compiled as executable machine language code or intermediate code that is executed on a framework or virtual machine.

In some embodiments, the disclosed software to operate the multi-MPS platforms is embodied as a computer readable storage medium (or multiple computer readable storage media) (e.g., a computer memory, one or more floppy discs, compact discs, optical discs, magnetic tapes, flash memories, circuit configurations in Field Programmable Gate Arrays or other semiconductor devices, or other non-transitory medium or tangible computer storage medium) encoded with one or more programs that, when executed on one or more computers or other processors, perform methods that implement the various embodiments of the invention discussed above. The computer readable medium or media can be transportable, such that the program or programs stored thereon can be loaded onto one or more different computers or other processors to implement various aspects as discussed above.

Computer-executable instructions may be in many forms, such as program modules, executed by one or more computers or other devices. Generally, program modules include routines, programs, objects, components, data structures, etc. that perform particular tasks or implement particular abstract data types. Typically the functionality of the program modules may be combined or distributed as desired in various embodiments. Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

The acts performed as part of the method may be ordered in any suitable way. Accordingly, embodiments may be constructed in which acts are performed in an order different than illustrated, which may include performing some acts simultaneously, even though shown as sequential acts in illustrative embodiments.

*(12) Apical flow module*

An apical flow module formed of an apical insert assembly and inlet and outlet tubing for supplying fluid to the apical flow module may be incorporated with the fluidic plate of a multi-well bioreactor. The apical insert assembly may be used together with removable inserts, such as 12-well or 24-well adapted TRANSWELL® inserts. Typically, the apical insert assembly includes an inlet point and an outlet point exchanging fluid at the apical side of the multi-well culture system, while the wells of the fluidic plate provide fluid exchange on the basal side of the multi-well culture system.

This combination allows co-culturing tissues with different oxygen, nutrient, and/or therapeutic treatment requirements. For example, an oxygen gradient may be established in the multi-well co-culture system where an artificial gut with a wide range of partial and obligate anaerobes is cocultured with tissues or cells from the liver, lung, pancreas, heart, the nervous system, immune system, and so on, to study the effect of the gut microbiome on the respective organs.

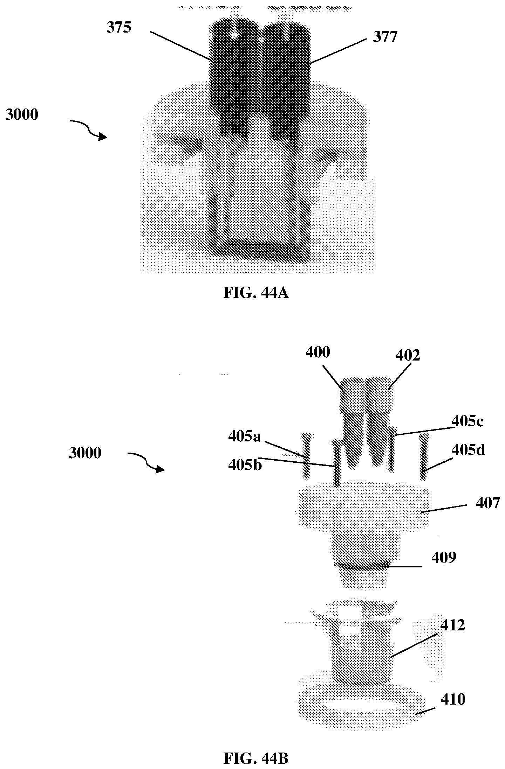

An example of establishing an oxygen gradient is presented in Figures 44A, 44B, and 45, where an apical insert assembly **3000** with a transwell **412** may be used with a fluidic plate **3100** to co-culture epithelial barrier cultures with microbiome and immune cells. Commensal bacteria, including *B. Fragilis, Lactobacillus,* and others form a protective barrier against pathogenic invasion, while also modulating functions in both the epithelial barrier and immune system. Interruption by pathogenic species such as *E. Coli, Salmonella,* etc. will disrupt normal microbiome function and cause inflammation and damage.

### III. Fabrication of Apparatus

The apparatus described above may be fabricated through molding, machining, and sterilization processes. A monolithic surface micromachined fluidic plate is preferred. It provides reliable performance and it is easy to clean. All fluid contacting surfaces are accessible for cleaning. All components have relatively long life time, and no delamination occurs in sterilization processes such as autoclave. Pneumatics can be easily cleared of condensation. Generally, the apparatus uses only two plate components bonded together, such that all pneumatic channels occupy the same plane within the plate. Inlets may be stacked by interleaving their channels and using drilled features to connect the inlets at different vertical positions to the channel layer, thus packing them more densely on the side face of the manifold.

The turnaround cycle for modularized computer-aided design (CAD) and machining is relatively quick. It is easy and rapidly customizable according to researcher's individual needs.

### A. Materials

The organs-on-a-chip systems may be fabricated from polydimethylsiloxane (PDMS), polysulfone, and other materials. PDMS is a versatile elastomer that is easy to mold (and thus highly amenable for prototyping), has good optical properties, and is oxygen permeable. In some embodiments, hydrophobic compounds including steroid hormones and many drugs exhibit strong partitioning into PDMS, thus precluding quantitative analysis and control of drug exposures (Toepke MW, et al., Lab Chip 6, 1484-1486 (2006)).

In preferred embodiments, the fluidic plate is fabricated from polysulfone (PSF). PSF is a rigid, amber colored, machinable thermoplastic with food grade FDA approval (21CFR177.1655) and USP Class VI biocompatibility. It is generally resistant to a wide range of chemical solvents, can be autoclaved, and is commonly used for instrumentation and medical devices. PSF also has dramatically lower surface adsorption and almost no bulk absorption of hydrophobic and lipophilic compounds (Ng SF, et al., Pharmaceutics 2, 209-223 (2010)).

All fluidic surfaces of the disclosed apparatus may be passivated prior to each experiment using serum albumin to further reduce the binding of biological molecules or drugs in the platform. The fluidic plate can also be cleaned and reused as many times as needed.

The top fluidic plate may be machined from a monolithic block of selected material, e.g., polysulfone (PSF) plastic, to include compartments to accommodate each MPS and an interconnecting chamber (called mixer or mixing chamber) to integrate and mix return flows, representing systemic circulation. Microfluidic channels and pumps are machined into the underside of the fluidic plate to convey fluid from the mixing chamber to each MPS. The individually addressable micro-pumps are fabricated in-line with the built-in fluid channels, and may be based on a 3-chamber, peristaltic pump-pump-pump design or a valve-pump-valve design. Additional pumps under each well provide recirculation flow, reducing nutrient and oxygen gradients within each compartment.

### B. Techniques for Assembly and Bonding

The fluidic plate, pneumatic plate, and membrane (in sterilization bags) are generally assembled in a biosafety cabinet. Before assembly, a sterile microplate lid is generally taped onto the fluidic plate to protect the sterility of the cell culture region. The layers can then be assembled upside down to aid with visual alignment through the acrylic plate. Once the alignment pins mate with the fluidic plate, the platform can be carefully removed from the hood, keeping pressure to maintain the seal between the plates. Screws can be inserted and tightened in a nonsterile environment as long as the plates are not separated. Two fully assembled platforms can be daisy chained by connecting them with short lengths of tubing connecting straight across to the corresponding ports. Daisy chained platforms are most easily transported with a large metal tray.

Platforms are assembled at a few days (e.g., 4 days) prior to the start of cell-culture experiment of interest. Surface passivation (priming) of sterile platforms can be conducted with 1% BSA and penicillin-streptomycin in PBS in volumes appropriate to each compartment. Pump function and tubing connections can generally be visually confirmed by pumping from the mixer to each dry compartment, then by running the recirculation pumps backwards to clear all air from the channels. Spillways can be manually wetted with small volumes to ensure spillway operation. Platforms are usually run overnight in the incubator to passivate and confirm full operation before the addition of cells.

In some embodiments, fluidic plates are bonded to create closed fluidic paths using a sintering method between plastic plates of specific pointedness.

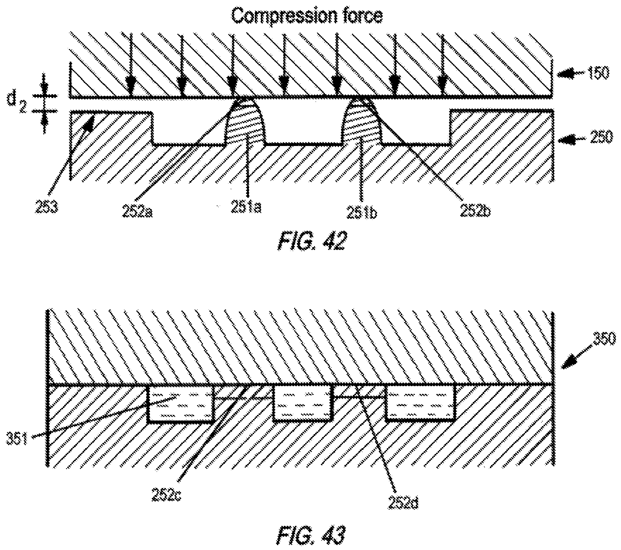

Figures 42 and 43 illustrate a sintering method to bond multiple components, where a bottom plate **250** has one or more small (e.g., pointed) contact areas **251a/251b** with a top plate **150** and some flat surface areas **253** that are shorter in height by a difference of $d_2$ than the vertices of the small contact areas **251a/251b.** Following force and treatments to sinter the top plate with the bottom plate, previously small surface areas are deformed and fused with the flat bottom surface of the top plate, to a height set by the joint height of the contacted flat surfaces **253,** resulting in a fused component **350** having internal space/volume **351** for passage of fluid.

In some embodiments, polyurethane (PU) membranes between about 20 and 200 microns thick, preferably between 50 and 100 microns thick, such as 50 microns thick, may be stretched on tension rings to maintain a constant tension. They can be laser cut with the corresponding pattern of screw holes on the pneumatic plate, if screw holes are present to align the top plate with the pneumatic plate.

A membrane diaphragm (optionally containing elastomer in regions corresponding to the pump and valve of the pneumatics) can be stretched between the pneumatics plate and the plate for the fluidic culture, and pressed to adhere to the pneumatic plate. In some embodiments, automation is used to attach the membrane to the fluidic plate.

Alternatively, elastomer patches may be used on the membrane layer to create seals and hermetic pathways in fluidic plates. Elastomer material may be used only at regions of a membrane or a patch corresponding to pneumatic pump and valves. Membranes containing elastomer patches can be prepared ahead of time and kept sterile for assembly of the chip. This would facilitate the assembly and operation of organonchip plates where an elastomer is deflected to create a pumping action only in very localized regions of the plate. A wide range of elastomer types and thicknesses may be applicable.

### C. Surface Treatment to Control Wettability

Surface patterning may be used to control wettability of open fluidic passages in the organ-on-chip plates. Machining patterns include zebra (linear), shark, concentric, and smooth surfaces.

The use of different machining processes and micro texturization can dramatically affect wettability of culture plates for organs-on- chips. Surface finish may significantly modify polysulfone wettability up to about a 40° change in the contact angle with water or a cell-culture media. Incubator conditions may also increases wettability to a slight extent of about 2-3° difference. It may be preferable for mesofluidic devices to have an increased wettability in order to improve the performance.

In general, dark polysulfone is more hydrophobic than light polysulfone. Selection of different grades of polysulfone provides another means to vary the wettability of the plates.

### D. Sterilization

One or more sterilization procedures may be performed on the cell-culturing fluidic plate, the actuation membrane, and optionally the pneumatic plate. Sterilization techniques include gas treatment (e.g., ethylene oxide), ionizing radiation, sonication, surface treatment (e.g., surfactant), and autoclave.

Generally before use, the top plate (e.g., polysulfone top plate) is cleaned and sterilized. First, the plate can be submerged in about 10% bleach for about 30 to 60 minutes, followed by a short rinse in distilled water. A residue-free surfactant was then used to remove any remaining contaminants by sonicating, submerged in about 10% solution (e.g., 7x solution, MP Biomedicals #MP0976680HP) for about 15 minutes. Two subsequent 15-minute sonication cycles in fresh deionized water follow to remove all surfactant before a final deionized water rinse. The plate can then be air dried, sealed in a sterilization bag, and autoclaved.

Generally, the pneumatic plate does not require formal sterilization, but prior to assembly it may be wiped thoroughly with a kimwipe sprayed with 70% ethanol to remove any dust or particles from the sealing areas that contact the membrane.

Pneumatic actuator membranes may be rinsed in about 10% 7x solution and with excess deionized water. Generally, an ethylene oxide gas sterilization step follows after the membranes are air dried, and the membrane is allowed 24 hours to degas in a chemical fume hood.

### E. Cells

Differentiated cell types and specialized cell types such as stem cells and paneth cells, as well as microbiome for some embodiments such as gut MPS, may be added to the platform.

## 1. Eukaryotic cells

The microphysiological systems (MPSs) supported by the platform may comprise primary cells, cell lines, pluripotent stem cells, progenitor cells, organoids, or any combination of mammalian or non-mammalian cells. For example, epithelial monolayers formed on transwell inserts from Caco2 cells or Caco-2 cells mixed with HT29 cells is one model of the gut, where circulation in the basal compartment (beneath the transwell) serves to improve the mixing and thus transport of drugs and other agents from the apical side of the epithelial layer to the basal side of the epithelial layer; the mixing facilitates the rapid distribution of drugs and other compounds in the basal compartment, and thus improves overall mixing between different MPS units on the platform. This model can be made more sophisticated by adding a source of immune cells (e.g. dendritic cells or macrophages from human peripheral blood monocytes or other sources) to the basal side of the membrane. It can be made even more sophisticated by culturing the epithelium on top of stroma encapsulated in an extracellular matrix gel; a similar arrangement can be used with primary intestinal cells. A second type of flow module is exemplified by the Liverchip - type arrangement, where flow is pumped through a scaffold containing 3D tissues comprising multiple cell types on a scaffold designed to distribute flow through the tissue. In another configuration, a closed microfluidic device with flow channels on either side of a central gel region may support tissues like 3D islets or lymph nodes, where endothelial cells seeded into the gel with the islets or lymph nodes form 3D vessels that allow perfusion of the islets or lymph nodes through the channels. Islets or lymph nodes may also be maintained in a gel in a transwell insert, and the basal side of the transwell insert can be covered with endothelial cells. Finally, cells may be added to the central circulation unit or any of the individual MPS circulation units to allow cell trafficking. For example, PBMC added to the basal compartment of the gut can traffic to the stroma across the membrane under inflammatory signals.

In some embodiments where triple negative breast cancer (TNBC) (i.e., lacking expression of estrogen, progesterone, and Her2 receptors) micrometastases in liver is modeled in the disclosed apparatus, MDA-MB-231 cells along with primary human hepatocytes and non-parenchymal cells may be cultured.

In some embodiments where gut and liver MPS are modeled to assess inflammatory-related stimulation of dormant micrometastases, absorptive enterocytes (e.g., CC2BB/el line) and mucin-secreting goblet cells (e.g., HT29-MTX line) may be seeded on the apical surface generally at a number ratio between 20:1 and 5:1, more preferably between 13:1 and 7:1, or about 9:1; whereas dendritic cells, obtained from in vitro differentiation of human PBMCs-derived monocytes, may be seeded on the lower side of the membrane of a TRANSWELL® in one well of the apparatus.

Other cell lines or cell types may be added dependent on use.

## 2. Microbiome

The microbiome includes and ecological community of commensal, symbiotic and pathogenic microorganisms. Commensal microorganisms colonize the host and establish a non-harmful coexistence. The relationship with their host is called symbiotic when microorganisms perform tasks that are known to be useful for the host, and parasitic/pathogenic, when disadvantageous to the host. Commensal microorganisms may be symbiotic microorganisms.

The microbiome can include bacteria, fungi, archaea and viruses that inhabit the skin and mucosal surfaces of the mouth, nose, digestive tract, and vagina of a host.

Examples of commensal microorganisms include *Bacteroidetes* and *Firmicutes, Actinobacteria, Proteobacteria,* and *Verrucomicrobia,* methanogenic archaea (mainly *Methanobrevibacter smithii*), eukarya (mainly yeasts), and viruses (primarily phage) (Lozupone et al., Nature, 489(7415):220-230 (2012)). See also Table 15 below (adapted from Hakansson and Molin, Nutrients, 3(6):637-682 (2011)).

**Table 15.** Taxa dominating the bacterial microbiota of the gastro-intestinal (GI)-tract.

| Phyla/Division | Class | Family | Genus |
|---|---|---|---|
| *Actinobacteria* | *Actinobacteria* | *Micrococcaceae* | *Rothia* |
| *Actinobacteria* | *Actinobacteria* | *Bifidobacteriaceae* | *Bifidobacterium* |
| *Firmicutes* | *Bacilli* | *Streptoccaceae* | *Streptococcus* |
| *Firmicutes* | *Bacilli* | *Lactobacillaceae* | *Lactobacillus* |
| *Firmicutes* | *Bacilli* | *Enterococcaceae* | *Enterococcus* |
| *Firmicutes* | *Negativicutes* | *Veillonellaceae* | *Veillonella* |
| *Firmicutes* | *Negativicutes* | *Veillonellaceae* | *Dialiser* |
| *Firmicutes* | *Clostridia* | unclassified *Clostridiales* | *Mogibacterium* |
| *Firmicutes* | *Clostridia* | *Peptostreptococcaceae* | *Peptostreptococcus* |
| *Firmicutes* | *Clostridia* | *Lachnospiraceae* | *Coprococcus* |

(continued)

| Phyla/Division | Class | Family | Genus |
|---|---|---|---|
| Firmicutes | Clostridia | Lachnospiraceae | Dorea |
| Firmicutes | Clostridia | Lachnospiraceae | Roseburia |
| Firmicutes | Clostridia | Lachnospiraceae | Butyrivibrio |
| Firmicutes | Clostridia | Ruminococcaceae | Ruminococcus |
| Firmicutes | Clostridia | Ruminococcaceae | Faecalibacterium |
| Firmicutes | Clostridia | Ruminococcaceae | Anaerotruncus |
| Firmicutes | Clostridia | Ruminococcaceae | Subdoligranulum |
| Firmicutes | Clostridia | Clostridiaceae | Clostridium |
| Firmicutes | Clostridia | Clostridiaceae | Blautia |
| Firmicutes | Clostridia | Eubacteriaceae | Eubacterium |
| Firmicutes | Clostridia | unclassified | Collinsella |
| Firmicutes | Erysipelotrichia | Erysipelotrichaceae | Holdemania |
| Proteobacteria | Betaproteobacteria | Alcaligenaceae | Sutterella |
| Proteobacteria | Betaproteobacteria | Neisseriaceae | Neisseria |
| Proteobacteria | Deltaproteobacteria | Desulfovibrionaceae | Bilophila |
| Proteobacteria | Gammaproteobacteria | Pasteurellaceae | Haemophilus |
| Proteobacteria | Gammaproteobacteria | Enterobacteriaceae | Enterobacter |
| Proteobacteria | Gammaproteobacteria | Enterobacteriaceae | Serratia |
| Proteobacteria | Gammaproteobacteria | Enterobacteriaceae | Escherichia |
| Proteobacteria | Gammaproteobacteria | Enterobacteriaceae | Klebsiella |
| Proteobacteria | Gammaproteobacteria | Moraxellaceae | Acinetobacter |
| Proteobacteria | Gammaproteobacteria | Pseudomonadaseae | Pseudomonas |
| Proteobacteria | Gammaproteobacteria | Cardiobacteriaceae | Cardiobacterium |
| Bacteroidetes | Bacteroidia | Prevotellaceae | Prevotella |
| Bacteroidetes | Bacteroidia | Porphyromonadaceae | Porphyromonas |
| Bacteroidetes | Bacteroidia | Porphyromonadaceae | Parabacteroides |
| Bacteroidetes | Bacteroidia | Bacteroidaceae | Bacteroides |
| Bacteroidetes | Bacteroidia | Rikenellaceae | Alistipes |
| Fusobacteria | Fusobacteria | Fusobacteriaceae | Fusobacterium |
| Spirochaetae | Spirochaetes | Brachyspiraceae | Brachyspira |
| Verrucomicrobia | Verrucomicrobiae | Verrucomicrobiaceae | Akkermansia |

Examples of pathogenic microorganisms include *Ericchia coli* and *Salmonella* species, *Clostridium difficile, Vibrio cholerae, Shigella* and *Campylobacter,* Rotavirus and Calicivirus (formerly Norwalk virus), and some protozoa (especially *Entamoeba histolytica, Giardia lamblia, Strongyloides stercoralis*) (Gorbach, Chapter 95 "Microbiology of the Gastrointestinal Tract" in Medical Microbiology. 4th edition (1996)).

Disease states may exhibit either the presence of a novel microbe(s), absence of a commensal microbe(s), or an alteration in the proportion of microbes.

Diseases with alterations in the microbiome include Crohn's disease, ulcerative colitis, obesity, asthma, allergies, metabolic syndrome, diabetes, psoriasis, eczema, rosacea, atopic dermatitis, gastrointestinal reflux disease, cancers of the gastrointestinal tract, bacterial vaginosis, neurodevelopmental conditions such as autism spectrum disorders, Alzheimer's disease, Parkinson's disease, and numerous infections, among others. For example, in Crohn's disease, concentrations of *Bacterioides, Eubacteria* and *Peptostreptococcus* are increased whereas *Bifidobacteria* numbers are reduced (Linskens et al., Scand J Gastroenterol Suppl. 2001;(234):29-40 ); in ulcerative colitis, the number of facultative anaerobes is increased. In obese subjects, the relative proportion of *Bacteroidetes* has been shown to be decreased relative to lean people (Ley et al., Nature. 2006 Dec 21;444(7122):1022-3), and possible links of microbial imbalances with the development of diabetes have also been discussed (Cani et al., Pathol Biol (Paris). 2008 Jul;56(5):305-9). Segmented Filamentous Bacteria have been shown to play a critical role in prevention of infection and development of autoimmune diseases (Ivanov et al, Cell. 139(3):485-98, 2009). In the skin, a role for the indigenous microbiota in health and disease in both infectious and noninfectious diseases, such as atopic dermatitis, eczema, rosacea, psoriasis, and acne has been noted (Holland et al. 1977 Br J Dermatol. 96(6):623-6; Thomsen et al. 1980 Arch. Dermatol.

116:1031-1034; Till et al. 2000 Br. J. Dermatol. 142:885-892; Paulino et al. 2006 J. Clin. Microbiol. 44:2933-2941). Furthermore, the resident microbiota may also become pathogenic in response to an impaired skin barrier (Roth and James 1988 Annu. Rev. Microbiol. 42:441-464). Bacterial vaginosis is caused by an imbalance of the naturally occurring vaginal microbiota. While the normal vaginal microbiota is dominated by *Lactobacillus,* in grade 2 (intermediate) bacterial vaginosis, *Gardnerella* and *Mobiluncus* spp. are also present, in addition to *Lactobacilli.* In grade 3 (bacterial vaginosis), *Gardnerella* and *Mobiluncus* spp. predominate, and *Lactobacilli* are few or absent (Hay et al., Br. Med. J., 308, 295-298, 1994)

Other conditions where a microbial link has been noted include rheumatoid arthritis, multiple sclerosis, nervous system, Parkinson's disease, Alzheimer's disease, muscular dystrophy, fibromyalgia and cystic fibrosis (Cani et al., Molecular Metabolism 5:743-752 (2016), Sampson et al., Cell 167:1469-1480 (2016)). Evidence suggests a link between commensal gut microbiota and the central nervous system. Bravo *et al* showed that ingestion of *Lactobacillus* strain regulates emotional behavior and central GABA receptor expression in a mouse via the vagus nerve (Bravo et al., Proc Natl Acad Sci U S A. 108(38):16050-5(2011)).

Probiotic bacteria, *Lactobacillus fermentum* and *Bifidobacterium lactis,* appears to inhibit permeability caused by gliadin and therefore to reduce gliadin-induced cellular damage in the gut.

### IV. Applications

*In vitro* to *in vivo* translation (IVIVT) is an interpretive step that compares and validates MPS results to clinically-relevant outcomes. The disclosed apparatus may be applied with the IVIVT method in assessing additional factors such as endogenous growth factor, inflammatory and hormone signals in the prediction of pharmacokinetics and pharmacodynamics (PK and PD). Compared with *in vivo* to *in vitro* correlation (IVIVC) and *in vivo* to *in vitro* extrapolation (IVIVE) methods in the prediction of PK, IVIVT goes a step further to include analysis of these additional factors and thus additionally predict PD, clinical toxicology, biomarkers, and patient stratification using information from MPS technologies. Combined with physiologically-based PK (PBPK) models for IVIVT, the disclosed apparatus provides an improved quantitative forecast on human responses to test agents, taking into accounts missing organs, organ and media size mismatches, and drug exposure.

In some embodiments, the system can also be used to exemplify diseases or disorders. For example, the apparatus may be used to establish micro-metastases in the context of a relatively large ($\geq$ 1 million cells) mass of liver cells, and then to analyze complex cell-cell communication network signatures using both measurements that can be routinely made in patients (on the circulating medium) as well as measurements that cannot also be made on patients - the kinetics of tumor cell growth and death.

### A. Preclinical drug discovery

MPS supports survival and functional culture of one or more organs on the chip for an extended period of time such as two, three, four, five weeks, two months, three months, or longer. Long-term multi-organ cultures are particularly advantageous for studying the pharmacology of low-clearance drugs, supporting repeated drug exposures, analyzing drug-drug interactions, and modeling chronic diseases.

The platform can be used for target identification and validation, target-based screening, phenotypic screening, and other biotechnological applications.

Cell and media volumes provide enough signal for commercial assays such as ELISAs and high-content, multiplexed assays.

Multiple -omics measurements across the scales of information flow in cells, from DNA to RNA to protein, protein activity states, and metabolites, as well as similar types of analysis of patient-derived immune cell function.

Although standard culture systems are reasonably effective for most small molecule drug PK assays, a vast number of diseases lacking adequate therapies have inflammation implications and are not well represented or modeled in standard culture systems. The apparatus may be particularly suitable for later stages of drug development that generally involves the immune system. The apparatus has been shown to recapitulate a complex immunologically-based drug-drug interaction between the anti-IL6 receptor antibody, tocilizumab, and the metabolism of simvastatin - a phenomenon that could not be reproduced in standard cultures (Long T, et al., Drug Metab Dispos 44, 1940-1948 (2016)).

A wide range of drug agents (small molecules, peptide, proteins, nucleic acid, etc.) may be tested in the disclosed apparatus for medicinal, cosmetic, or scientific applications. Generally addition to the mixing well mimics an intravenous dosage, and addition to the gut well mimics an oral dosage.

Agents are selected based on the disease or disorder to be treated or prevented.

**B. Disease and disorder to be modeled**

The multi-organ apparatus is a useful tool for disease modeling and drug development, especially in identifying and defining the appropriate "minimal set" of interacting organ systems to represent a disease state.

Drug development for a variety of diseases and/or disorders may be improved utilizing the disclosed multi-organs on a chip apparatus by culturing relevant tissues or cell types for systemic studies. Complex individual organs-on-chips that capture the local features of disease, especially inflammation, are preferably applicable for modeling systemic diseases or diseases that are associated with multiple organs or involve multiple types of cells. The diseases and/or disorders that may be modeled in the disclosed bioreactor include but are not limited to cancers/tumors (e.g., tumors in the breast, bones, liver, lungs, and brain), chronic inflammatory diseases (e.g. diabetes, arthritis, endometriosis, and Alzheimer's), non-malignant growth of endometrium outside the uterus (endometriosis) or displaced into the uterine muscle (adenomyosis), abnormal liver functions such as those caused by non-alcoholic fatty liver disease,

The system provides a means for exposing the cells to an agent to determine its effect on the cells administering the agent in different dosages, in a different dosing regimen, or in combination with one or more other agents and determining its effect on the cells, as well as wherein the agent is administered to different cell types or cell types associated with one or more diseases or disorders. This allows one to test agents *in vitro* with human cells under conditions mimicking a human, at least in part, under controlled conditions, looking for effects on other cell types, as well as on the cells one wants to monitor for an effect. This is more rapid, more controlled, and yet not restricted to a single class of cells or tissues.

**Examples**

The present invention will be further understood by reference to the following non-limiting examples. Examples 1-5 are provided for background reference and are from US2017/0227525.

***Example 1. Perfused, Single-Organ Microphysiological Systems (MPSs) On The Chip.***

**(1) Liver: Perfused, Coculture of Hepatocyte-Kupffer to Three Weeks.**

Materials and Methods

Metabolic and immunologically competent 3D cryopreserved human hepatocytes and kupffer cells were cocultured. Multiple hepatocyte and Kupffer cell donors have been qualified in the MPS. Co-cultures were responsive to Lipopolysaccharide (LPS) stimulus down to 0.01 $\mu$g/ml.

Results

Table 1 shows the comparison of hepatocytes only and coculture of hypatocyte and Kupffer cells at a 10:1 ratio over 7 days in a perfused MPS platform.

**Table 1 Biological function of liver cells vs. immune-competent liver MPS.**

| Function at Day 7 (n=3) | Hepatocyte Only | Hepatocyte + Kupffer (10:1) |
|---|---|---|
| Albumin ($\mu$g/day/mg) | 35±11 | 53±32 |
| Urea ($\mu$g/day/mg) | 175±75 | 184±25 |
| CYP3A (pmol/min/mg) | 2.9±0.5 | 2.0±0.7 |

The secretions of interleukin 6 (IL-6) and tumor necrosis factor alpha (TNF$\alpha$) of the cocultured liver MPS were measured. The reproducibility of IL-6 response to LPS stimulus was determined. A physiologically-relevant (relatively low) level of cortisol was used in the common media. Hydrocortisone (cortisol) enhanced differentiated function, but suppressed inflammatory response.

The duration of cryopreserved human hepatocytes and kupffer cell co-cultures on the perfused MPS platform was extended to 3 weeks.

Expression of 84 hepatic genes remained stable between day 7 and 21. Kupffer cells remained inactivated for 21 days, until stimulated with LPS. Cell death marker LDH declined after seeding and remained at a low constant level. Hepatic phenotypic activity, including albumin and CYP450 remained measurable and superior to 2D cultures for 21 days. CYP450 activity was sensitive to hydrocortisone levels in the cultures.

**(2) Lung: Primary Human Tracheobronchial Epithelium Differentiation.**

Materials & Methods

A tracheobronchial module was developed in a TRANSWELL on the MPS platform. Primary basal epithelial cells (all CK5+) were differentiated at the air-liquid interface into a full subset of epithelial cell types. Different metrics were evaluated including transepithelial electrical resistance (TEER), mucus production, differentiated cell populations, and basal IL-8 production.

Results

Fluorescent microscopic images were taken and confirmed the expressions of differentiation and functional markers: Tubulin (ciliated), Ck5 (basal), Muc5Ac (goblet), and phalloidin (actin).

**Table 2 Comparison of estimated and measured percentages of differentiated cells on the lung MPS platform.**

| Cell Sub-type | Physiological Estimates | MIT - Donor Z Lung MPS |
|---|---|---|
| Basal | 20 % | $28 \pm 3$ % |
| Goblet | 1-5% | $1 \pm 0.5$% |
| Ciliated | 30 - 50 % | $46 \pm 11$% |
| Clara | < 1 % | Not Determined |

Table 2 confirms the lung MPS model supported differentiation of cells, to a degree that aligned well with physiological estimates, which was indicative of its function of primary human tracheobronchial epithelial model.

**(3) Endometrium: Half-primary Coculture of Epithelium-Stroma is Stable and Functionally Secretes Glycoprotein.**

Materials & Methods

In a menstrual cycle, human endometrium undergoes a proliferative phase, marked by an increased level of estrogen, and a secretory phase, marked by an increased level of progesterone. In the secretory phase, endometrium secretes characteristic proteins such as glycodelin, prolactin, and insulin-like growth factor-binding protein 1 (IGF-BP1).
An exemplary endometrium model of cell culture system in a TRANSWELL® on the MPS platform includes hydrogel encapsulating stromal cells and epithelial cells on top surface of the hydrogel were cultured on the apical side of the TRANSWELL®. The epithelial cell source was primary human endometrial epithelial cells, which were readily obtained from endometrial biopsies, had limited expansion and lifespan in culture, exhibited functional differences between harvest in proliferative and secretory phase, and supported robust glycodelin secretion (secretory phase cells). The cell line used was Ishikawa human stage 1 adenocarincoma cell line, which were estrogen and progesterone receptor positive, polarized in matrigel (Chitcholtan et al., Exp Cell Research, (2013)) or functionalized PEG gels, and had low/variable secretion of glycodelin. The stromal cell source was primary human endometrial stromal cells, which were readily obtained from human biopsies, had well-established *in vitro* expansion protocols, and showed functional difference between harvest in proliferative and secretory phase. The cell line used was human tert-immortalized cell line (tHESC), which was highly proliferative and stable, had low/variable secretion of prolactin or IGF-BP1 without strong decidualization cues, and could be quiesced in PEG gels.

Results

With Ishikawa epithelial cells, the apical medium contained estradiol and progesterone. Ishikawa glycodelin secretion was below detection limit. Off-platform culture of tHESC had produced IGF-BP1 right at the detection limit and a borderline detectable amount of prolactin (PRL) from primaries (likely due to a dilution effect). On-platform co-culture of these "half-primary" cell lines were stable and had detectable functions from apical sampling.

**(4) Gut/Immune: Coculture for Two Weeks Forming Intact Barrier, and Drug-induced Leakiness Triggering Immune Response.**

Materials & Methods

Physiological gut system features absorption and metabolism, intestinal immune system, interactions between microbiome and mucosal interactions, immune interaction between cell and microbiome, and drug-immune interactions. An exemplary immune-competent gut model with cell culture in a TRANSWELL® on the MPS platform included enterocytes and goblet cells were cocultured on the apical side of the TRANSWELL and immune cells on basal side of the TRANSWELL membrane. The cell lines used were Caco2 (enterocytes), HT29-MTX (goblet cells), and dendritic cells (immune cells), where enterocytes: goblet cells were cultured at a 9:1 ratio (mimicking small intestine) to maturation off platform for 2 weeks and transferred to perfusion platform with added immune cells on the basal side of the TRANSWELL membrane.

Results

When cultured off-platform (static medium), immune cells at 14 days had much less survival than were cultured on-platform with basolateral flow, as confirmed via immunofluorescent microscopy. On-platform cultures at 14 days supported the function of gut barrier cells and their differentiation.

*Example 2. Assessment of Drug Toxicity In Individual Or 2-Way MPS On The Chip.*

**(1) Liver/Immune: Toxicities of Diclofenac and Tolcapone.**

An immune-competent liver MPS model was prepared and studied. Diclofenac impaired liver functions while cell death was minimal. Tolcapone decreased mitochondrial activity and caused cell death.

**(2) Gut/Immune: Toxicities of Diclofenac and Tolcapone.**

An immune-competent gut MPS model was prepared and studied. Diclofenac reduced epithelial barrier integrity, causing leaky gut with a minimal cell death. Tolcapone led to severe cellular death, hence a complete loss of epithelial function.

**(3) Endometrium MPS: Toxicities of Diclofenac and Tolcapone.**

An endometrium MPS model was prepared and studied. Diclofenac-induced loss of function correlating with cellular death. Tolcapone induced loss of function correlating with cellular death.

**(4) Gut-Liver 2-Way: Administration of Tolcapone to Gut ("oral") Results In Gut-Specific Toxicity.**

Materials & Methods

An immune-competent gut-liver interacted MPS model was prepared (details similarly shown in Example 3) and studied. Tolcapone was added to the gut MPS to mimic "oral" administration. Metrics were an volume-weighted average from the 3 media compartments: $Signal_{systemic} = Signal_{apical, gut} * V_{apical, gut} + Signal_{basal, gut} * V_{basal, gut} + Signal_{liver} * V_{liver}$.

Results

In the presence of tolcapone, gut and liver suffered MPS-specific loss of function, which was indicative of MPS-specific toxicity of tolcapone even delivered "orally". Gut and liver also suffered from MPS-specific cell death markers whereas generic marker was insensitive to tolcapone, which indicated site of toxicity of tolcapone. Intestinal Fatty Acid Binding Protein (I-FABP) was used as a clinical biomarker of enterocyte damage for various disease states.

*Example 3. Inflammatory Cytokine/Chemokine Crosstalk in Gut-Liver 2-way MPS.*

Immune-competent human liver (hepatocytes and Kupffer cells) combined with intestinal (enterocyte, goblet cells, and dendritic cells) microphysiological systems is studied in this *in vitro* platform, to examine gut-liver interactions under normal and inflammatory contexts.

The liver is situated downstream from the gut; as such, it is constantly exposed to gut-derived factors, including metabolites, microbial antigens and inflammatory mediators. However, a quantitative understanding of how these multicellular tissues communicate and contribute to overall (patho)physiology is limited.

Background

Gut-liver crosstalk is an integrable part of normal physiology and their dysregulation is a common denominator in many disease conditions (Marshall JC, Host Defense Dysfunction in Trauma, Shock and Sepsis: Mechanisms and Therapeutic Approaches, eds Faist E, Meakins JL, & Schildberg FW (Springer Berlin Heidelberg, Berlin, Heidelberg), 243-252 (1993)). Furthermore, gut and liver are major organs involved in drug absorption and metabolism; changes to their functional interaction can impact their response to therapeutic intervention (Morgan ET, Drug Metab Dispos 29(3):207-212 (2001); Deng X, et al., Pharmacological Reviews 61(3):262-282 (2009); Long TJ, et al. Drug Metabolism and Disposition 44(12):1940-1948 (2016)). Gut and liver functions are intimately linked by virtue of their anatomical proximity. The liver receives 70% of its blood supply from the gut via portal circulation; as such, it is constantly exposed to gut-derived factors, including metabolites, microbial antigens and inflammatory mediators. The gut-liver axis contributes considerably to the overall immunological state of the body, with the gut being the largest immune organ and the liver harboring over 70% of the total macrophage population in the body. Interspecies differences often hinder the accurate prediction of human responses in animal models; the discrepancy is especially evident in processes involving the immune system (Mestas J, et al., The Journal of Immunology 172(5):2731-2738 (2004); Giese C et al., Adv Drug Deliver Rev 69:103-122 (2014)). For instance, few of the clinical trials for sepsis treatment have led to drug approval (Seok J, et al. Proc Natl Acad Sci U S A 110(9):3507-3512 (2013). Fink MP Virulence 5(1):143-153 (2014)). In sepsis, gastrointestinal and hepatic injury have been associated with increased disease severity (Rowlands BJ, et al., British Medical Bulletin 55(1):196-211 (1999); Nesseler N, et al., Crit Care 16(5):235 (2012)). Acute liver failure in the first 72 hours following onset of sepsis was highly correlated with poor prognosis in septic patients. However, the lack of specific and predictive biomarkers precludes early diagnosis and patient stratification for effective intervention (Pierrakos C et al., Crit Care 14(1):R15 (2010)). Though the gut-liver axis has been implicated in the escalation of a septic response, the mechanisms and molecular players involved are poorly defined. Therefore, a fundamental understanding of gut-liver crosstalk is critical not only to the prediction of drug disposition, efficacy and toxicity, but also the elucidation of (patho)physiological mechanisms.

Materials & Methods

*In vivo,* the liver receives a dual blood supply, from the hepatic artery and the portal vein (Liaskou E, et al., Mediators Inflamm 2012:949157 (2012); Brown RP, et al., Toxicol Ind Health 13(4):407-484 (1997)). Correspondingly, the flow from the mixer well was partitioned into the gut and liver compartments to be 75% and 25%, respectively, scaled proportional to physiological cardiac output and hepatic blood flow, as shown below in Tables 3 and 4. Output from the gut module fed into the liver, representing portal circulation. A systemic recirculation flow rate of 15 mL/day was used to ensure efficient distribution of exogenous and endogenous factors.

**Table 3 Exemplary controlled flow rates in gut-liver MPS.**

| Compartments | | Flow rates ($\mu$L/s) |
| --- | --- | --- |
| Mixer | self-circ | 1.0 |
| | mixer-gut | 0.13 |
| | mixer-liver | 0.043 |
| Liver | self-circ | 1.0 |
| Gut | self-circ, basal | 0.25 |

**Table 4 Exemplary controlled volume in gut-liver MPS.**

| Compartments | Volume (mL) |
| --- | --- |
| Mixer | 1.0 |
| Liver | 1.6 |

(continued)

| Compartments | | Volume (mL) |
|---|---|---|
| Gut | Apical | 0.5 |
| | Basal | 1.5 |

The liver and gut tissue constructs in this study were multicellular and (innate) immune-competent, designed to encompass multiple key functions, including metabolic, barrier and immune functions. The liver microtissue comprised a co-culture of human primary cryopreserved hepatocytes and Kupffer cells at physiological 10:1 ratio, maintained in a culture medium permissive for retention of inflammation responses, as previously described (Long TJ, et al. Drug Metabolism and Disposition 44(12):1940-1948 (2016); Sarkar U, et al. Drug Metabolism and Disposition 43(7):1091-1099 (2015)). The gut tissue was engineered to mimic the small intestine, with the epithelial monolayer derived from 9:1 ratio of absorptive enterocytes (Caco2-BBE) and mucus-producing goblet cells (HT29-MTX), and the immune compartment containing primary monocyte-derived dendritic cells.

Human primary hepatocytes and Kupffer cells were purchased from Life Technologies (HMCPMS, HUKCCS). Scaffolds were washed 15 min in 70% EtOH, rinsed twice in PBS, incubated for 1 hour @RT in 30 ng/mL rat tail collagen in PBS, left to dry overnight at room temperature, and punched into platforms (filter under scaffold under retaining ring). At day(-3) to experiment start, 10:1 ratio of hepatocytes and Kupffer cells were thawed into warm Cryopreserved Hepatocyte Recovery Medium (CHRM, Invitrogen), spun at 100g for 8 min, and seeded at $6*10^5$ and $6*10^4$ cells/well, respectively, in cold hepatocyte seeding medium (250 mL Advanced DMEM + 9 mL Gibco Cocktail A + 12.5 mL FBS). After one day, the media was changed to D(-2) medium (250 mL Advanced DMEM + 10 mL Cocktail B). After two more days, the medium was changed to common medium for the duration of the interaction experiment.

The common medium used in this study consisted of 500 mL Williams E medium + 20 mL Gibco Cocktail B + 100 nM hydrocortisone + 1% Penicillin-Streptomycin (P/S)).

Caco2 (clone: C2BBe1, ATCC, passage 48-58) and HT29-MTX (Sigma, passage 20-30) cell lines were used for the intestinal epithelial cultures. Both cell lines were passaged twice post thawing before their use for TRANSWELL seeding. Cell lines were maintained in DMEM (Gibco™ 11965-092) supplemented with 10 % Fetal Bovine Serum (Atlanta Biologicals S11150, heat inactivated (HI) at 57 °C for 30 minutes), 1x GlutaMax (Gibco™ 35050-061), 1x Non-Essential Amino Acids (Gibco™ 15140-148), and 1% Penicillin-Streptomycin (Gibco™ 15140-148). Caco2 at ~70-80% confluence and HT29-MTX at ~80-90% confluence were harvested using 0.25 % Trypsin-EDTA (Gibco™ 25200-056) and mechanically broken up into single cells for TRANSWELL seeding. In seeding the cells into TRANSWELL®, the apical and basal side of TRANSWELL membrane were coated with 50 μg/mL Collagen Type I (Corning 354236) overnight at 4 °C. The inserts were washed with PBS-/- to remove unbound protein. 9:1 ratio of C2BBe1 to HT29-MTX was seeded onto 12-well 0.4 μm pore size TRANSWELL® inserts (Costar 3460) at a density of $10^5$ cells/cm². Seeding media contained 10% heat-inactivated FBS, 1x GlutaMax, 1% P/S in Advanced DMEM (Gibco™ 12491-015). The apical media was replaced 1 day post seeding to remove any unattached cells. The top and bottom compartments of the TRANSWELL plate are fed with 0.5 mL and 1.5 mL of seeding medium every 2-3 days. After 7 days, medium was switched to a serum-free gut medium by replacing FBS with Insulin (5 μg/ml)-Transferrin (5 μg/ml)-Sodium Selenite (5 ng/ml) (Roche 11074547001).

To evaluate long-term functional viability in the gut-liver interaction, corresponding single tissue controls on platform were assayed with identical media volumes, flow rates and flow partitioning. All conditions were tested in a defined, serum-free common media that supported maintenance of gut and liver functions. The liver cells (10:1 hepatocyte: Kupffer cell) were seeded on platform 3 days prior to the start of the interaction experiment to allow for tissue formation and recovery from seeding-related stress responses. The gut MPS was differentiated for 3 weeks off-platform prior to the start of the interaction experiment. During long-term operation, the common culture medium in the system was replaced every 3 days.

To evaluate the health of the liver, samples from all compartments were taken at every media change (every 72 hours) and assayed for albumin via ELISA (Bethyl Laboratories, E80-129).

Various Cytochrome P450 (CYP) enzyme activities were measured using a developed CYP cocktail assay (Pillai VC, et al., J Pharm Biomed Anal 74:126-132 (2013)). Briefly, a cocktail of CYP substrates was added to liver compartment for a one hour incubation, and the supernatant was collected for downstream processing. Substrate-specific metabolite production was analyzed using mass spec.

Monocyte-derived dendritic cells were used as the immune component of the gut MPS. Briefly, peripheral blood mononuclear cells (PBMCs) were processed from Leukopak (STEMCELL Technologies, 70500) and stored in liquid nitrogen. For each experiment, PBMCs were thawed and monocytes were isolated using the EasySep Human Monocyte Enrichment Kit (STEMCELL Technologies, 19058). The monocytes were differentiated to dendritic cell in Advanced RPMI medium (Gibco™ 12633-012) supplemented with 1x GlutaMax, 1% P/S, 50 ng/mL GM-CSF (Biolegend 572903), 35 ng/mL IL4 (Biolegend 574004) and 10nM Retinoic acid (Sigma R2625). After 7 days of differentiation (at day 19-20 of gut epithelial cell maturation), immature dendritic cells were harvested using Accutase (Gibco™ A11105-01) and seeded on to the basal side of the inverted gut TRANSWELLs® for 2 hours. After 2 hours, cells were returned to culture plate and fed with gut media.

One-day post dendritic cell seeding, gut barrier function was assessed. Gut MPS with transepithelial electrical resistance values of at least 250 Ohm∗cm2 were considered acceptable for experiment. For all interaction experiments on platform, the gut MPS was maintained in common media.

TEER measurement was performed using the EndOhm-12 chamber with an EVOM2 meter (World Precision Instruments). The samples and the EndOhm chamber were kept warm at ~37°C on a hot plate. Temperature was rigorously maintained during TEER measurement to minimize variability.

Secreted mucin was measured in apical gut compartment using an Alcian Blue assay. The mucin quantification protocol was adapted from (5). Briefly, media from apical was collected in low-binding tubes, and spun down at 10,000 g for 5 minutes, and the supernatant was collected and stored at -80 °C for subsequent analysis. Mucin secretion was quantified against a standard of mucin (Sigma-Aldrich M3895) dissolved in culture medium. Samples and standards were incubated in a 96-well plate in a 3:1 mix of sample to Alcian Blue solution (Richard Allen Scientific) for two hours. After incubation, plates were centrifuged at 1640g for 30 minutes at room temperature. Supernatant was removed by inverting the plates. Samples were rinsed twice with wash buffer (40% (v/v) of ethanol and 60% (v/v) of 0.1M sodium acetate buffer containing 25mM $MgCl_2$ at pH 5.8), with a 10-minute centrifugation step after each rinse. After second spin, supernatant was removed and samples were dissolved with 10% SDS in distilled water. Plates typically required shaking or pipetting to fully resuspend samples. If bubbles formed during resuspension, plates were centrifuged again for about 5 minutes prior to absorbance measurement on a Spectramax m3/m2e at 620nm.

Cytokine levels were measured using multiplex cytokine assays, 37-plex human inflammation and 40-plex panel chemokine panels (Bio-Rad Laboratories, Inc., Hercules, CA, USA). Briefly, media samples were collected in low-binding tubes, spun down at 10,000 g for 5 mins to remove cell debris, and the supernatant was stored in -80 °C. Samples were measured at multiple dilutions to ensure the measurements were within the linear dynamic range of the assay. To minimize non-specific binding to beads, bovine serum albumin (BSA) was added to achieve a final concentration of 5 mg/mL in all samples. The protein standard was reconstituted in the same media and the protein stock serially diluted to generate an 8-point standard curve. Assays were run on a Bio-Plex 3D Suspension Array System (Bio-Rad Laboratories, Inc.). Data were collected using the xPONENT for FLEXMAP 3D software, version 4.2 (Luminex Corporation, Austin, TX, USA). The concentration of each analyte was determined from a standard curve, which was generated by fitting a 5-parameter logistic regression of mean fluorescence on known concentrations of each analyte (Bio-Plex Manager software).

To obtain the total production amount per platform, the concentration values were normalized by compartmental volume and added up across all compartments (mixer, gut, liver) in each platform.

For both the baseline and inflamed conditions (at Day 3, n=4), intestinal and hepatic tissues were taken out of the platforms, and mRNA was extracted using the PureLink RNA mini kit (ThermoFisher, 12183018A). Total RNA was analyzed and quantified using the Fragment Analyzer (Advanced Analytical), and cDNA was generated using the SMART-Seq v3 kit (Clontech). After cDNA fragmentation (Covaris S2), cDNA was end-repaired and adaptor-ligated using the SPRI-works Fragment Library System I (Beckman Coulter Genomics). Adaptor-ligated cDNA was then indexed during PCR amplification, and the resulting libraries were quantified using the Fragment Analyzer and qPCR before being sequenced on the Illumina HiSeq 2000. 40-50 nt single-end read with an average depth of 15-20 million or 5 million reads per sample were sequenced for the baseline and inflamed conditions respectively.

The FASTQ files were generated from the sequencing runs. The resultant reads were aligned to the human reference genome (GRch37/hg19) using Tophat (version 2.0.12) (Kim D, et al. Genome Biol 14(4):R36 (2013)) to identify reads that map to known transcripts, accounting for splice junctions. HTSeq was used to determine the number of read counts uniquely overlap with known genomic features (Anders S, et al., Bioinformatics 31(2):166-169 (2015)).

To identify significantly altered genes in isolation vs interaction conditions, differential gene analysis of count data was performed using DESeq2 (Version 1.12.3) in R (Love MI, et al., Genome Biol 15(12):550 (2014)). Only genes with greater than 1 cpm (count per million) in at least 4 replicates, were included in the analysis. Multiple testing correction was performed using the procedure of Benjamini and Hochberg. Genes with an adjusted P-value below a FDR cutoff of 0.05 were considered significant.

GOSeq R packages (Young MD, et al., Genome Biol 11(2):R14 (2010)) was used to determine the over-represented biological of the differentially expressed genes (FDR-adjusted P-values<0.05).

GSEA (version 2.2.3) was performed to identify differentially regulated gene sets in isolation versus interaction, as describe in (Subramanian A, et al. (2005) Proceedings of the National Academy of Sciences 102(43):15545-15550). To stabilize variance, the normalized count data were processed using a regularized logarithm transformation in DESeq2. The signal-to-noise metric was used to generate the ranked list of genes. Canonical pathway gene sets from Molecular Signatures Database (c2.cp.v5.2) were used, which is a collection of curated genes sets from multiple databases (e.g.,

Reactome, KEGG, BioCarta, PID). The empirical P-values for each enrichment score were calculated relative to the null distribution of enrichment scores, which was computed via 1000 gene set permutations. Gene sets with nominal P-value <0.01 and q-value <0.05 were considered significant. Enrichment map (11), a Cytoscape plugin, was used to visualize the overlaps between significant gene sets and to facilitate the systematic interpretation of the interdependencies among different biological processes.

Results

1. Baseline liver- and gut-specific functions were maintained for a relatively long term (> 2 weeks) in gut-liver interactome.

Hepatic and intestinal functions assessed over two weeks of culture were comparable for MPS maintained in communication or in isolation, as assessed by measurements of albumin production, gut barrier integrity, and gut mucus production. To evaluate liver metabolic function at the end of the 2-week experiment, the liver tissues from isolation and interaction conditions (in the absence of gut) were dosed with a cocktail of drug substrates targeting specific CYP450 enzymes. Drug-specific metabolite production in the media was measured using mass spectrometry to determine the cytochrome P450 activity of the different isoforms. Overall, the liver metabolic function was largely maintained, with modulation of select cytochrome P450 activities observed in gut-liver interaction. In particular, Cyp2C9 activity was significantly enhanced, while Cyp3A4/5 activity was depressed. Gut-specific functions, including barrier integrity and mucus production, were not sizably altered between interaction and isolation controls. Subtle but significant modulation of cytochrome P450 activities (e.g., CYP3A4 and CYP2C9) were observed after 2 weeks of interaction.

2. Bile acid synthesis pathway was modulated in bi-directional gut-live crosstalk.

RNA sequencing was performed to profile the global transcriptomic changes in the gut and liver tissues after 3 days of interaction, with corresponding isolation controls (i.e., gut-only and liver-only). 105 genes were significantly (FDR-adjusted P<0.05) altered in the liver during interaction relative to isolation controls, of which 70 were upregulated and 35 were downregulated. For the gut, 6 genes were significantly differentially expressed, of which 2 were upregulated and 4 were downregulated. To understand the functional implications of these molecular changes, Gene Ontology (GO) analysis was performed to identify overrepresented biological processes that were altered under interaction. Only significantly altered genes (FDR-adjusted P< 0.05) were used for GO analysis. The up-regulated biological processes in the liver primarily involved cell division-related processes are shown in Table 5.

**Table 5 Biological processes up-regulated in liver under gut-liver interaction.**

| GO ID | Biological Processes | P-value | Adj. P-value |
|---|---|---|---|
| GO:0051302 | regulation of cell division | $0.0\times10^{+00}$ | $0.0\times10^{+00}$ |
| GO:0000070 | mitotic sister chromatid segregation | $0.0\times10^{+00}$ | $0.0\times10^{+00}$ |
| GO:0007059 | chromosome segregation | $0.0\times10^{+00}$ | $0.0\times10^{+00}$ |
| GO:0007049 | cell cycle | $9.6\times10^{-18}$ | $1.1\times10^{-14}$ |
| GO:0006996 | organelle organization | $7.3\times10^{-10}$ | $3.6\times10^{-07}$ |
| GO:0008283 | cell proliferation | $3.4\times10^{-09}$ | $1.4\times10^{-06}$ |
| GO:0007017 | microtubule-based process | $4.9\times10^{-08}$ | $1.4\times10^{-05}$ |

Induction of cell cycle genes in liver may indicate an adaptive response to gut-derived signals, although the soluble factors involved are unknown. On the other hand, the down-regulated biological processes in the liver were mainly metabolic processes including bile acid biosynthesis, lipid metabolism and xenobiotic metabolism (Table 6).

**Table 6 Biological processes down-regulated in liver under baseline gut-liver interaction.**

| GO ID | Biological Processes | P-value | Adj. P-value |
|---|---|---|---|
| GO:0006694 | steroid biosynthetic process | $2.2\times10^{-05}$ | $1.5\times10^{-01}$ |
| GO:0006579 | amino-acid betaine catabolic process | $2.8\times10^{-05}$ | $1.5\times10^{-01}$ |
| GO:0008202 | steroid metabolic process | $4.7\times10^{-05}$ | $1.5\times10^{-01}$ |
| GO:1901617 | organic hydroxy compound biosynthetic process | $1.0\times10^{-04}$ | $2.6\times10^{-01}$ |

(continued)

| GO ID | Biological Processes | P-value | Adj. P-value |
|-------|---------------------|---------|--------------|
| GO:0044283 | small molecule biosynthetic process | $1.8 \times 10^{-04}$ | $3.8 \times 10^{-01}$ |
| GO:0015914 | phospholipid transport | $2.2 \times 10^{-04}$ | $3.9 \times 10^{-01}$ |
| GO:0044281 | small molecule metabolic process | $3.3 \times 10^{-04}$ | $4.8 \times 10^{-01}$ |

Specifically, a mediator of the bile acid metabolism, CYP7A1, was down-regulated, which was indicative of a physiological coupling of gut-liver functions, e.g., bile acide-mediated enterohepatic crosstalk. CYP7A1 is an enzyme central to bile acid synthesis; and its feedback inhibition via FGF19 enterohepatic communication is well established (Ding L, et al., Acta Pharm Sin B 5(2):135-144 (2015)). The result on CYP7A1 was consistent with previous findings that perfusion of precision-cut rat intestinal and hepatic tissues in a microfluidic device for 7 hours resulted in bile acid-mediated CYP7A1 inhibition (van Midwoud PM, et al., Lab Chip 10(20):2778-2786 (2010)). Though the number of significant genes in the gut samples was insufficient for GO analysis, PCSK9, one of the differentially expressed genes, was found to play a key role in cholesterol and lipid homeostasis. In fact, cholesterol and various types of bile acids have been shown to suppresses PCSK9 mRNA expression in Caco2 intestinal cultures (Leblond F, et al. Am J Physiol Gastrointest Liver Physiol 296(4):G805-815 (2009)). The studies showed the convergence on cholesterol and bile acid metabolism pathways was indicative of transcriptional rewiring due to inter-MPS communication.

3. Coordinated transcriptomic changes and tissue-specific transcriptomic changes were observed in inflammatory gut-liver crosstalk.

A large number of immune cells reside in the gut and liver during homeostasis and their activation in disease can contribute to systemic pathophysiology. Liver dysfunction associated with idiosyncratic adverse drug reactions has been linked to inappropriate immune activation (Cosgrove BD, et al. Toxicology and Applied Pharmacology 237(3):317-330 (2009)). This study complemented parenchymal tissue models with immune cells in both the gut and liver to provide a more physiologically-relevant culture platform for disease modeling and drug testing. Reciprocal immune-epithelial cell communication drives systemic inflammation.

In an inflammatory context mimicking endotoxemia, 2 ng/mL lipopolysaccharide (LPS) was added in the circulating media from day 0 (Gut MPS on platform) to day 3 (RNAseq was performed) while the operation of the system and the isolation controls were tested in a similar way to the baseline studies above. The LPS concentration was chosen based on clinically-relevant range of plasma endotoxin (2-10 ng/mL) reported in patients with inflammatory diseases (Guo S, et al, Am J Pathol 182(2):375-387 (2013)).

RNA sequencing was performed to assess the global molecular changes associated with inflammatory gut-liver crosstalk. For the liver, 2548 genes were significantly altered in the interaction, of which 1137 genes were upregulated and 1411 genes were downregulated. GO analysis of the differentially expressed genes showed upregulation of cytokine response and antigen processing and presentation pathways, and downregulation of lipid and xenobiotic metabolism pathways (Tables 7 and 8).

**Table 7 Biological processes up-regulated in liver under inflammatory gut-liver interaction.**

| GO ID | Biological Processes | P-value | Adj. P-value |
|-------|---------------------|---------|--------------|
| GO:0006955 | immune response | $1.7 \times 10^{-28}$ | $2.3 \times 10^{-24}$ |
| GO:0006952 | defense response | $1.5 \times 10^{-27}$ | $1.0 \times 10^{-23}$ |
| GO:0019221 | cytokine-mediated signaling pathway | $2.5 \times 10^{-25}$ | $4.6 \times 10^{-22}$ |
| GO:0060337 | type I interferon signaling pathway | $2.0 \times 10^{-25}$ | $4.4 \times 10^{-22}$ |
| GO:0051707 | response to other organism | $8.8 \times 10^{-22}$ | $7.6 \times 10^{-19}$ |
| GO:0019882 | antigen processing and presentation | $3.1 \times 10^{-06}$ | $2.9 \times 10^{-04}$ |
| GO:0002250 | adaptive immune response | $1.0 \times 10^{-07}$ | $1.2 \times 10^{-05}$ |
| .. see further list in Table 11 and below. | | | |

**Table 8 Biological processes down-regulated in liver under inflammatory gut-liver interaction:**

| GO ID | Biological Processes | P-value | Adj. P-value |
|---|---|---|---|
| GO:0044281 | small molecule metabolic process | $7.8 \times 10^{-97}$ | $1.0 \times 10^{-92}$ |
| GO:0006082 | organic acid metabolic process | $5.4 \times 10^{-78}$ | $1.8 \times 10^{-74}$ |
| GO:0055114 | oxidation-reduction process | $5.4 \times 10^{-69}$ | $1.4 \times 10^{-65}$ |
| GO:0044710 | single-organism metabolic process | $2.3 \times 10^{-56}$ | $5.0 \times 10^{-53}$ |
| GO:0032787 | monocarboxylic acid metabolic process | $4.0 \times 10^{-54}$ | $7.4 \times 10^{-51}$ |
| GO:0006629 | lipid metabolic process | $7.4 \times 10^{-53}$ | $9.7 \times 10^{-50}$ |
| GO:0006805 | xenobiotic metabolic process | $1.3 \times 10^{-23}$ | $5.7 \times 10^{-21}$ |
| .. see further list in Table 12 and below. | | | |

For the gut, 780 genes were significantly altered during interaction, of which 290 genes were upregulated and 490 genes were downregulated. Similarly, GO analysis revealed upregulation of defense response, antigen processing and presentation pathways and protein translation; down-regulated pathways included alcohol biosynthesis, steroid and lipid metabolism (Tables 9 and 10).

**Table 9 Biological processes up-regulated in gut under inflammatory gut-liver interaction.**

| GO ID | Biological Processes | P-value | Adj. P-value |
|---|---|---|---|
| GO:0006952 | defense response | $4.5 \times 10^{-20}$ | $5.3 \times 10^{-16}$ |
| GO:0060337 | type I interferon signaling pathway | $1.2 \times 10^{-19}$ | $5.3 \times 10^{-16}$ |
| GO:0002376 | immune system process | $1.4 \times 10^{-13}$ | $2.2 \times 10^{-10}$ |
| GO:0034097 | response to cytokine | $9.2 \times 10^{-13}$ | $1.1 \times 10^{-09}$ |
| GO:0006082 | organic acid metabolic process | $9.8 \times 10^{-10}$ | $3.5 \times 10^{-07}$ |
| GO:0019882 | antigen processing and presentation | $6.0 \times 10^{-07}$ | $1.4 \times 10^{-04}$ |
| GO:0006418 | tRNA aminoacylation for protein translation | $6.6 \times 10^{-10}$ | $2.7 \times 10^{-07}$ |
| .. see further list in Table 11 and below. | | | |

**Table 10 Biological processes down-regulated in gut under inflammatory gut-liver interaction.**

| GO ID | Biological Processes | P-value | Adj. P-value |
|---|---|---|---|
| GO:0046165 | alcohol biosynthetic process | $5.5x\ 10^{-16}$ | $7.0\times\ 10^{-12}$ |
| GO:0008202 | steroid metabolic process organic hydroxy compound metabolic | $2.6x\ 10^{-14}$ | $1.0\times\ 10^{-10}$ |
| GO:1901615 | process | $3.2 \times 10^{-14}$ | $1.0 \times 10^{-10}$ |
| GO:0044281 | small molecule metabolic process monocarboxylic acid metabolic | $4.4 \times 10^{-12}$ | $4.3 \times 10^{-09}$ |
| GO:0032787 | process | $4.8 \times 10^{-11}$ | $3.8 \times 10^{-08}$ |
| GO:0006629 | lipid metabolic process | $7.8 \times 10^{-11}$ | $5.9 \times 10^{-08}$ |
| GO:0055114 | oxidation-reduction process | $5.0 \times 10^{-8}$ | $2.4 \times 10^{-05}$ |
| .. see further list in Table 12 and below. | | | |

In addition to gene-based GO analysis that focused only on the significantly altered genes determined by an arbitrary statistical cut-off, Gene Set Enrichment Analysis (GSEA) was also performed to uncover coordinated changes in groups of genes that are functionally related. GSEA can reveal more nuanced pathway regulation that might have been masked by strict cutoffs in gene-based approach. Generally, GSEA results were largely consistent with GO analysis outcomes, but with greater interpretability and generality. Consensus clusters of gene sets from different databases were obtained, which contained overlapping but distinct groups of genes that define major biological processes. Specifically, inflammation-related pathways centered around IFN$\alpha$/$\beta$/$\gamma$ signaling were up-regulated whereas metabolic processes involving cholesterol and lipid metabolism were down-regulated in both the gut and liver in interaction (Table 11). The pronounced alteration in inflammatory processes and lipid metabolism was characteristic of a sepsis response.

**Table 11 Gene sets commonly up-regulated in both gut and liver in the gut-liver MPS**

| Pathways | | Liver: qval | Gut: q-val |
|---|---|---|---|
| IFN signaling | Reactome_Interferon_alpha_beta_signaling | 0.0+00 | 0.0+00 |
| | Reactome_Interferon _gamma_signaling | 0.0+00 | 0.0+00 |
| | Reactome_Interferon _signaling | 0.0+00 | 0.0+00 |
| Cytokine signaling | Reactome_cytokine_signaling_in_immune_syste m | 0.0+00 | $3.0 \times 10^{-3}$ |
| Antigen processing | Kegg_antigen_processing_and_presentation | $1.0 \times 10^{-03}$ | $1.0 \times 10^{-03}$ |
| | Reactome_antigen_presentation_folding_assembl y_ and_peptide_loading_of_class_I_MHC | $2.0 \times 10^{-3}$ | $3.0 \times 10^{-3}$ |
| | Reactome_antigen_processing_cross_presentation | $6.0 \times 10^{-03}$ | $2.5 \times 10^{-02}$ |
| | Reactome_ER_phagosome_pathway | $7.0 \times 10\text{-}03$ | $4.0 \times 10^{-03}$ |
| Immune processes | Kegg_intestinal_immune_network_for_IGA_prod uction | $1.8 \times 10^{-02}$ | $2.5 \times 10^{-02}$ |
| | Reactome_immunoregulatory_interactions_ between_ a_lymphoid_and_a_non_lymphoid_cell | $4.0 \times 10^{-3}$ | $2.4 \times 10^{-02}$ |
| | Kegg_allograft_rejection | $1.0 \times 10^{-03}$ | 0.0+00 |
| | Kegg_autoimmune_thyroid_disease | $2.0 \times 10^{-3}$ | 0.0+00 |
| | Kegg_viral_myocarditis | $2.0 \times 10^{-3}$ | $4.0 \times 10^{-3}$ |
| | Kegg_graft_versus_host_disease | $3.0 \times 10^{-3}$ | 0.0+00 |
| | Kegg_Type_I_diabetes_mellitus | $7.0 \times 10^{-03}$ | 0.0+00 |

**Table 12 Gene sets commonly down-regulated in both gut and liver in the gut-liver MPS.**

| Pathways | | Liver: q-val | Gut: q-val |
|---|---|---|---|
| Endogeneous and xenobiotic metabolism | Reactome_cytochrome_p450_arranged | 0.0+00 | $3.3 \times 10^{-02}$ |
| | Reactome_phase | 0.0+00 | $4.4 \times 10^{-02}$ |
| | Kegg_metabolism_of_xenobiotics_by_ cytochrome_p450 | 0.0+00 | $4.6 \times 10^{-02}$ |
| Lipid metabolism | Kegg_PPAR_signaling_pathway | 0.0+00 | $4.0 \times 10^{-03}$ |
| | Reactome_lipid_digestion_mobilizatio | $4.0 \times 10^{-03}$ | $3.9 \times 10^{-02}$ |
| | Reactome_lipoprotein_metabolism | $1.0 \times 10^{-02}$ | $4.3 \times 10^{-02}$ |
| | Reactome_metabolism_of_lipids_and_1 ipoproteins | $1.0 \times 10^{-03}$ | $8.0 \times 10^{-03}$ |
| Steroid and bile acid metabolism | Kegg_steroid_hormone_biosynthesis | $1.1 \times 10^{-02}$ | 0.0+00 |
| | Reactome_bile_acid_and_bile_salt_me tabolism | 0.0+00 | $3.2 \times 10^{-02}$ |

In addition to the co-modulated pathways, tissue-specific regulation was also identified. Pathways involved in hypoxia and TGFβ/SMAD signaling were exclusively upregulated in the liver in interaction, suggestive of a pro-fibrotic response. Although the current study focused on acute inflammation, chronic liver inflammation has been linked to liver fibrosis.

In the gut, PI3K-mediated ERBB2 and ERBB4 signaling was upregulated, which was indicative of a wound healing or anti-apoptotic response, possibly serving as a protective mechanism. Previously, ERBB2 (Yamaoka T, et al. Proc Natl Acad Sci U S A 105(33):11772-11777 (2008); Zhang Y, et al., Lab Invest 92(3):437-450 (2012)) and ERBB4 (Frey MR, et al., Gastroenterology 136(1):217-226 (2009)) signaling have been shown, *in vitro* and *in vivo,* to protect against TNF-induced apoptosis in intestinal epithelial cells and provide pro-survival and pro-healing effects following intestinal injury.

Complete lists of gene sets involved in tissue-specific modulation are shown below:

Gene sets up-regulated uniquely in liver during inflammatory gut-liver crosstalk included Biocarta_TNFR2_pathway, St_tumor_necrosis_factor_pathway, PID_TNF_pathway, Reactome_chemokine_receptors_bind_chemokines, Kegg_cytokine_cytokine_receptor_interaction, Kegg_rig_i_like_receptor_signaling_pathway, Kegg_cytosolic_dna_sensing_pathway, Reactome_negative_regulators_of_rig_i_MDA5_signaling, Naba_secreted_factors, PID_CD40_pathway, PID_hif1_tfpathway, PID_hif2pathway, PID_il23_pathway, Kegg_primary_immunodeficiency, Reactome_antiviral_mechanism_by_ifn_stimulated_genes, Reactome_)O.o_linked_glycosylation_of_mucins, Reactome_regulation_of_hypoxia_inducible_factor_hif_by_oxygen, Reactome_rig_i_mda5_mediated_induction_of_ifn_alpha_beta_pathways, Reactome_signaling_by_tgf_beta_receptor_complex, Reactome_smad2_smad3_smad4_heterotrimer_regulates_transcription, Reactome_traf6_mediated_irf7_activation, Reactome_transcriptional_activity_of_smad2_smad3_smad4_heterotrimer, and St_fas_signaling_pathway.

Gene sets up-regulated uniquely in gut during inflammatory gut-liver crosstalk included PID_IL12_2pathway, Kegg_abc_transporters, Reactome_amino_acid_synthesis_and_interconversion_transamination, Kegg_aminoacyl_trna_biosynthesis, Reactome_cytosolic_trna_aminoacylation, Reactome_trna_aminoacylation, Kegg_cell_adhesion_molecules_cams, Kegg_histidine_metabolism, Reactome_activation_of_genes_by_atf4, Reactome_perk_regulated_gene_expression, Reactome_PI3 K_events_in_erbb2_signaling, and Reactome_PI3K_events_in_erbb4_signaling.

Gene sets down-regulated uniquely in liver during inflammatory gut-liver crosstalk included Biocarta_ami_pathway, Biocarta_intrinsic_pathway, Kegg_alanine_aspartate_and_glutamate_metabolism, Kegg_arachidonic_acid_metabolism, Kegg_arginine_and_proline_metabolism, Kegg_beta_alanine_metabolism, Kegg_biosynthesis_of_unsaturated_fatty_acids, Kegg_butanoate_metabolism, Kegg_citrate_cycle_tca_cycle, Kegg_complement_and_coagulation_cascades, Kegg_drug_metabolism_cytochrome_p450, Kegg_drug_metabolism_other_enzymes, Kegg_fatty_acid_metabolism, Kegg_glycine_serine_and_threonine_metabolism, Kegg_glycolysis_gluconeogenesis, Kegg_propanoate_metabolism, Kegg_glyoxylate_and_dicarboxylate_metabolism, Kegg_histidine_metabolism, Kegg_linoleic_acid_metabolism, Kegg_lysine_degradation, Kegg_oxidative_phosphorylation, Kegg_parkinsons_disease, Kegg_peroxisome, Kegg_proximal_tubule_bicarbonate_reclamation, Kegg_pyruvate_metabolism, Kegg_retinol_metabolism, Kegg_tryptophan_metabolism, Kegg_tyrosine_metabolism, Kegg_valine_leucine_and_isoleucine_degradation, PID_hnf3b_pathway, Reactome_biological_oxidations, Reactome_branched_chain_amino_acid_catabolism, Reactome_citric_acid_cycle_tca_cycle, Reactome_fatty_acid_triacylglycerol_and_ketone_body_metabolism, Reactome_formation_of_fibrin_clot_clotting_cascade, Reactome_metabolism_of_amino_acids_and_derivatives, Reactome_peroxisomal_lipid_metabolism, Reactome_phase_ii_conjugation, Reactome_pyruvate_metabolism_and_citric_acid_tca_cycle, Reactome_respiratory_electron_transport, Reactome_respiratory_electron_transport_atp_synthesis_by_chemiosmotic_ coupling_and_heat_production_by_uncoupling_proteins_, Reactome_synthesis_of_bile_acids_and_bile_salts, Reactome_synthesis_of_bile_acids_and_bile_salts_via_7alpha_hydroxychol esterol, and Reactome_tca_cycle_and_respiratory_electron_transport.

Gene sets down-regulated uniquely in gut during inflammatory gut-liver crosstalk included Biocarta_TNFR2_pathway, Kegg_DNA_replication, Kegg_pantothenate_and_coa_biosynthesis, Kegg_pentose_and_glucuronate_interconversions, Kegg_steroid_biosynthesis, Kegg_terpenoid_backbone_biosynthesis, PID_aurora_b_pathway, PID_hif1_tfpathway, Reactome_activation_of_atr_in_response_to_replication_stress, Reactome_activation_of_the_pre_replicative_complex, Reactome_cholesterol_biosynthesis, Reactome_deposition_of_new_cenpa_containing_nucleosomes_at_the_centr omere, Reactome_DNA_strand_elongation, Reactome_e2f_mediated_regulation_of_dna_replication, Reactome_fatty_acyl_coa_biosynthesis, Reactome_formation_of_tubulin_folding_intermediates_by_cct_tric, Reactome_g1_s_specific_transcription, Reactome_g2_m_checkpoints, Reactome_transport_of_vitamins_nucleosides_and_related_molecules, and Reactome_triglyceride_biosynthesis.

4. Systemic inflammation suppressed hepatic detoxification function.

Hepatic clearance of endogenous and xenobiotic compounds is mediated by two mechanisms, i.e., metabolism and bile elimination. The results revealed inflammatory crosstalk negatively affected both of these pathways and might lead to the buildup of toxic by-products. Collectively, CYP1A2, CYP2C9, CYP2C19, CYP2D6, an CYP3A4 and 3A5 are responsible for the metabolism of over 90% of known drugs (Jacob A, et al., Int J Clin Exp Med 2(3):203-211 (2009); Ebrahimkhani MR, et al., Adv Drug Deliv Rev 69-70:132-157 (2014)). All of these were suppressed in the liver in the integrated system, likely due to accumulation of inflammatory mediators, such as IL6, TNFα, and/or type I interferons (Long TJ, et al. Drug Metabolism and Disposition 44(12):1940-1948 (2016); Huang SM, et al. Clin Pharmacol Ther 87(4):497-503 (2010)).

In short, lipid metabolism and inflammation were the dominant pathways altered during gut-liver interaction. Lipoprotein binding to LPS can redirect the LPS uptake from Kupffer cells to hepatocytes, thereby attenuating immune activation and facilitating bile clearance of LPS (Khovidhunkit W, et al., J Lipid Res 45(7):1169-1196 (2004)). Peroxisome proliferator-activated receptors (PPARs), master regulators of lipid metabolism, have been shown to exert anti-inflammatory effects (Varga T, et al., Biochim Biophys Acta 1812(8):1007-1022 (2011)). Taken together, the suppression of apolipoprotein synthesis and PPAR signaling observed during inflammatory gut-liver crosstalk indicates a potential loss of a protective mechanism, thereby intensifying inflammation in immune and epithelial cells. The complexities in systemic response to perturbations motivate the need for multi-cellular and multi-organ experimental models.

Sepsis patients are susceptible to adverse drug reactions due to inflammation-induced suppression of liver metabolic function, specifically the activity of cytochrome P450 enzyme system (Kim TH, et al., Febs J 278(13):2307-2317 (2011)). The results demonstrated altered mRNA expression of Phase I and Phase II metabolic enzyme in inflammatory gut-liver crosstalk. Thus, accurate prediction of drug pharmacokinetics and pharmacodynamics necessitates the consideration for multi-organ interaction as well as the physiological context (i.e., health vs. disease). This is especially pertinent for drugs with a narrow therapeutic window because even modest changes to cytochrome P450 activities can precipitate toxicity.

5. Cytokine levels in the gut-liver integrated system deviates from the linear sum of individual, isolated systems.

The levels of secreted cytokines and chemokines were measured in the media at 6, 24, and 72 hours post stimulation to examine the temporal evolution of the inflammatory response. Pairwise hierarchical clustering was performed on the 72 hr. cytokine measurement to explore the correlations of cytokine responses among the analytes and conditions. Unsupervised principal component analysis (PCA) revealed that the over 96% of the covariance in the cytokine dataset can be captured by the first 2 principal components. PC1 accounted for 76.5% of the variability in the data, segregating the interaction versus isolation controls; PC2 accounted for 19.8% of the total variability and discriminated the gut and liver only conditions. The loading plot depicted the relative contribution of each analyte to the 1st and 2nd principal components. All analytes were positively loaded on PC1 and contributed to the cytokine level in the integrated system, whereas loadings on PC2 can help infer the primary tissues of origin of the circulating cytokines/ chemokines in the integrated system. While none of the soluble factors were unique to gut or liver, multivariate cytokine patterns can reveal tissue-specific signatures.

In order to accurately assess the contribution of inter-MPS crosstalk to the integrated inflammatory response, the measured cytokine levels in the interacting system were compared to the theoretical linear sum of the isolated conditions. The cytokine level observed in isolation accounted for cytokine output due to direct TLR4 activation and intra-MPS paracrine signaling. The actual (measured) cytokine levels in the integrated systems deviated significantly from the linear sum of the isolated systems, revealing non-linear modulation of cytokine production as a result of inter-MPS communication. Approximately 58% of the analytes were linearly additive, 23% were less than additive, and 19% were more than additive, some very markedly so. Interestingly, several cytokines exhibited similar temporal dynamics as CXCL6, which was linearly additive up to 24 hr., and then diverged from linear sum and became more than additive. This may suggest a thresholddependent regulation, where cytokine production is dependent on the accumulation of upstream inducer molecules during organ crosstalk.

6. Inflammatory-related CXCR3 ligand was greatly amplified in gut-liver interaction.

Table 13 shows a notable more than additive amplification of CXCR3 ligands, where CXCL10 (IP10) and CXCL11 (I-TAC) were most significantly more than additive and CXCL9 (MIG) was borderline significant. The fractions of total analytes that were additive, subadditive, and more than additive in terms of the level in the gut-liver MPS, compared to the linear sum of the levels in individual gut and individual liver, were 58%, 23%, and 19%, respectively. CXCR3 signaling has been implicated in autoimmunity, transplant rejection, infection, and cancer (Groom JR, et al., Immunol Cell Biol 89(2):207-215 (2011); Singh UP, et al., Endocr Metab Immune Disord Drug Targets 7(2):111-123 (2007)).

**Table 13. Cytokines/chemokines statistically different from linear sum (Adj. P-value <0.05) and the corresponding receptors.**

| Cytokines/ chemokines | | Receptors | Target cells |
|---|---|---|---|
| Subadditive | CCL21 | CCR7, CCR11 | thymocytes & activated T cells |
| | CCL1 | CCR8, CCR11 | monocytes, NK cell, B cells & DCs |
| | CCL11 | CCR3 | leukocytes, eosinophils |
| | CXCL12 | CXCR4, CXCR7 | lymphocytes, endothelial progenitors |
| | CHI3L1 | | |
| | CCL22 | CCR4 | lymphocytes, monocytes, DCs, NK cells |
| | MIF | CXCR2, CXCR4 | most hematopoetic cells & endothelial cells |
| | IFN-Y | IFNY-R | immune cells & epithelial cells |
| | CCL27 | CCR10 | memory T lymphocytes |
| | CXCL13 | CXCR5 | B lymphocytes |
| Synergistic | CXCL10 | CXCR3 | Th 1 cells, NK cells |
| | CXCL11 | CXCR3, CXCR7 | Th 1 cells, NK cells, monocytes, neutrophils |
| | CXCL6 | CXCR1, CXCR2 | neutrophils |
| | CCL20 | CCR6 | lumphocytes, DCs |
| | CCL2 | CCR2 | monocytes, basophils |
| | CX3CL1 | CX3CR1 | leukocytes |
| | CCL19 | CCR7 | lymphocytes, DCs, hematopoetic progenitors |
| | CXCL9 | CXCR3 | Th1 cells, NK cells |

These results showed that consideration of gut-liver crosstalk may be important for assessing systemic inflammatory processes and their potential influence on disease development.

RNA sequencing data showed activation of IFN$\alpha$/$\beta$/$\gamma$ signaling pathways in both the gut and liver during organ crosstalk. TNF$\alpha$ can magnify IFN-dependent production of CXCR3 ligands. PCA loadings revealed that TNF$\alpha$ was predominately gut-derived and IFN$\gamma$ was produced at comparable levels by both the gut and the liver. It was plausible that gut (dendritic cells)-derived TNF$\alpha$ interacted with tissue-specific IFN$\gamma$ signaling to drive CXCR3 ligand production in both the gut and liver. However, the relative contribution of epithelial and immune compartment to the integrated response was difficult to ascertain. Although immune cells are the principal responders to endotoxin due to higher expression of TLR4 as shown in Table 14, epithelial cells also contribute to inflammation indirectly via activation by immune cell-derived cytokines, such as TNF$\alpha$ and IL-1 (Nguyen TV, et al. Drug Metab Dispos 43(5):774-785 (2015); Yeruva S, et al., Int J Colorectal Dis 23(3):305-317 (2008); Dwinell MB, et al., Gastroenterology 120(1):49-59 (2001)).

**Table 14 TLR expression (Log10, normalized to GAPDH)**

| Cell types | TLR1 | TLR2 | TLR3 | TLR4 | TLR5 |
|---|---|---|---|---|---|
| Primary human hepatocytes (thawed) | 179.6 | 48.0 | 332.0 | 12.0 | 13.7 |
| Primary human hepatocyte after 4 days in culture | 299.2 | 104.2 | 314.4 | 50.4 | 13.2 |
| Primary Kupffer cells (thawed) | 3496.4 | 10713.5 | 83.7 | 2753.7 | 24.5 |

Exposure of rat hepatocytes to TNF$\alpha$ and IFN$\gamma$ *in vitro* promoted CXCL10 mRNA and protein expression (Hassanshahi G, et al., Iran J Allergy Asthma Immunol 6(3):115-121 (2007)). Combinations of IL-1$\alpha$/$\beta$, TNF$\alpha$ and IFN$\gamma$ have been shown to induce CXCR3 ligand gene expression and protein secretion in intestinal cell lines and human intestinal xenografts. To assess the epithelial contribution to the cytokine response, 5 ng/mL TNF$\alpha$, 5 ng/mL IFN$\gamma$, or both, was added for presence of 24 hours to stimulate the gut epithelium (Caco2-BBE/HT29-MTX) basally. Co-treatment of TNF$\alpha$ and IFN$\gamma$ on the gut epithelium, in the absence of immune cells, resulted in marked amplification of 4 out of the 8 chemokines identified in the integrated system, including CXCL9, CXCL10, CXCL11 and CX3CL1 (Table 11).

These results corroborated with the RNAseq findings and demonstrated that IFN$\gamma$ and TNF$\alpha$ signaling crosstalk was central to the chemokine production in the integrated system. These results showed epithelial cells are not passive bystanders during inflammatory gut-liver crosstalk, but contribute considerably to the overall immune milieu via paracrine interactions with immune cells.

Under inflammatory gut-liver interaction, more than additive amplification of chemokine production was detected from the disclosed integrated gut-liver MPS. This amplification was in part mediated by TNF$\alpha$ and IFN$\gamma$ signaling. Although immune cells were normally considered as the primary sensor of endotoxin, the results here showed epithelial cells responded to immune cells-derived signals to influence CXCL9/10/11 and CX3CL1 chemokine production. Exposure to TNF$\alpha$ and IFN$\gamma$ did not result in the amplification of CCL19, CCL20, CXCL6 and CCL2 in intestinal epithelial cells, which indicated the involvement of additional mechanisms, likely in different cell types.

The chemokine production observed in the integrated system can target cells of the innate and adaptive immune system. Potential immune cell recruitment can be inferred based on the chemokines and the corresponding receptors profile. Although adaptive immunity was not represented in the system, regulation of pathways linking innate and adaptive immunity were evident during organ crosstalk. For example, enrichment of the CD40 costimulatory process was identified. CD40 is a surface receptor ubiquitously expressed on immune cells as well as non-immune cells. CD40L is predominantly expressed by CD4$^+$ T cells and CD40-CD40L engagement mediates heterologous cellular communication (Danese S, et al., Gut, 53(7):1035-1043 (2004)). Taken together, CXCR3 chemokine production and CD40-CD40L regulation implicates a bias toward Th1 signaling.

***Example 4. 4-Way MPS On The Chip For Pharmacokineticl Pharmacodynamic (PK-PD) Prediction.***

**(1) 4-way MPS survival and functional for at least 2 week.**

Materials & Methods

Validation: Flow rates in thirteen 4-MPS platforms (n=9 pumps per platform) averaged from 0.82 to 1.12 $\mu$L/s without calibration, and had an average standard deviation of 0.07 $\mu$L/s. Software calibration factors were calculated from the flow rate measurement and entered to correct the pump rates to within $\pm 5\%$ of the target flow rates.

A systemic interaction flow rate of $Q_{mix}$ = 5 mL/day was used for the duration of the experiment. Flow was partitioned to each MPS from the mixer based on the relative percentages of cardiac output to each tissue type in humans; these numbers can be easily modified on the platform for different scaling strategies and MPS modules. Additionally, intra-MPS basal recirculation rates of 0.25 $\mu$L/s (gut, lung, and endometrium MPSs) and 1 $\mu$L/s (liver MPS and mixer) were used to provide well-mixed basal media in each compartment and oxygenate the liver tissue. Complete media changes were conducted every 48 hours. During media changes, samples were taken from each compartment to assess MPS function throughout the two-week interaction study. Biomarker metrics of healthy cell function were measured during a 2-week co-culture of 4-way MPS: liver, gut, lung, and endometrium, with a partitioning of flow. Every two days, secreted albumin and IGFBP-1 were measured from conditioned media. Barrier integrity of the Gut and Lung MPSs was quantified with trans-epithelial electrical resistance (TEER), measured off-platform using the commercial EndOhm systems. Simultaneously, functionality of each MPS in isolation was monitored.

Results

1. 4-way MPS supports cell viability and functions for at least two weeks.

Continuous functionality metrics from 4-MPS platform studies indicated the multi-organ MPS viability during the 2-week culture. Transient albumin secretion kinetics was observed of an initial increase in albumin secretion followed by a gradual decline by the conclusion of the experiment. Barrier integrity of the Gut and Lung MPSs was quantified with trans-epithelial electrical resistance (TEER). TEER values from the Gut MPS fluctuated in the early days of interaction studies before settling into a 150-250 $\Omega \cdot cm^2$ range for the remainder of the experiment. Lung MPS TEER values followed a similar trend of high TEER during the first few days, but eventually established stable values in the 600-800 $\Omega \cdot cm^2$ range. Endometrium MPS functionality, evaluated by secretion of insulin-like growth factor-binding protein 1 (IGFBP-1), remained in the 20-30 pg/day range throughout the study. Similar trends for each phenotypic metric in the isolation studies were observed, but IGFBP-1 secretion rate in the isolated endometrium MPS (off-platform) was lower than that of interaction studies.

2. Endogenously produced albumin from one organ was uniformly distributed to each compartment with the controlled systemic flow rate.

In the 4-MPS platform, the effect of systemic flowrate ($Q_{mix}$) on albumin (endogenously produced by liver MPS) secretion and distribution kinetics was characterized via collecting samples from each compartment and, then, the results were computationally model to assess the accuracy of the distribution. The albumin concentrations in each compartment and the mixing chamber were at day 2 ($Q_{mix}$ = 5 ml/day), day 4 ($Q_{mix}$ = 15 ml/day), and day 6 (Qmix = 30 ml/day).

With an increasing systemic flow rate, albumin was distributed more uniformly as demonstrated by experimental measurements, where the deviation between MPSs was considerably lower with higher flow rates. Similarly, the calculated albumin secretion rates show smaller standard deviations. However, one platform showed considerably lower albumin in all compartments for days 2-4. Furthermore, computationally generated albumin distribution profiles was compared with experimentally measured albumin concentrations. The ratios of both values indicated that the higher flowrate resulted in more deterministic molecular biodistribution in the 4-way MPS platform.

**(2) Gut-Liver/Lung/Endo 4-Way Platform: Independent Flow Rate Control Improves PK-PD Prediction For Complex Physiology.**

Background

In the study of modern medicine for human, interpretation of results from animal studies for the prospect of human treatment generally employs allometric scaling; and the interpretation of *in vitro* results for the prospect of *in vivo* efficacy is commonly referred to IVIV Correlation and Extrapolation. *In vitro* studies OF liver MPS pharmacokinetics (PK) is characterized by accounting for binding in media, drug uptake, and elimination. *In vivo* studies use known clinical data and physiological-based PK (PBPK)/ absorption/binding models to calculate comparable parameters.

Clinical PK data of seven drugs from Manvelian et al. 2012, Shimamoto et al. 2000, Yilmaz et al. 2011, Willis et al. 1979 were compared with *in vitro* liver data gathered from LIVERCHIP™ to assess the prediction accuracy of *in vitro* results from LIVERCHIP™. While PBPK *in vivo* for free diclofenac elimination per cell has a rate constant of $1.76 \times 10^{-10}$ (cell*min)$^{-1}$, scaled liver MPS from LIVERCHIP™ was studied to show a diclofenac elimination rate constant of $5.66 \times 10^{-9}$ (cell*min)$^{-1}$. Hence, *in vitro* drug PK data from LIVERCHIP™ overestimated *in vivo* drug elimination rate.

Materials & Methods

Following a diagram and flow partitioning (total Qmixing = 1 $\mu$L/s; liver/mixer recirculation = 1 $\mu$L/s; gut/lung/endometrium recirculation rate = 0.5 $\mu$L/s) (9 pumped flows: 5 self-circ, 4 mixing; 6 independent flow rates: 5 self-circ flows collapsed to 2 independent pneumatic duty cycles; 6 pump sets = 18 DOF), 4-way MPS interactome was studied, where addition of agents to the mixing chamber accounted for an intravenous dosage while addition to the gut chamber accounted for an oral dosage. Drug was added to the apical side of gut chamber for the experiment.

Results

Uniform drug distribution was calculated as time for downstream (Endometrium) MPS to reach 90% of the concentration in mixing chamber. Drug exposure was calculated as area under curve (AUC) from 0-48 hr. in downstream (Endometrium) MPS. Drug exposure and distribution were able to strongly drive selection of useful operational ranges: Qmixing > 15 mL/day for drug permeability greater than $10^{-6}$ cm/s, and Qmixing > 40 mL/day for AUC0-48hr of greater than $2*10^4$ ng/L*hr.

***Example 5. Operation of 7-Way MPS On The Chip***

Materials & Methods

Validation: Flow rates in ten 7-way platforms (n=17 pumps per platform) averaged 1.12 $\pm$ 0.10 $\mu$L/s. Software calibration factors were calculated from the flow rate measurement and entered to correct the pump rates to within $\pm$5% of the target flow rates (0.99 $\pm$0.056 $\mu$L/s).

A 7-way MPS platform was utilized and operated in a similar manner to the 4-MPS platform described in Example 4. The 7-way platform include gut (immune-competent), liver (immune-competent), lung, endometrium, cardiac, brain, and pancreas MPSs, and was assessed for survivability and function over a 3-week period. Each MPS was differentiated or matured in isolation prior to the interaction study. Platforms were run at a systemic flow rate of $Q_{mix}$ = 10 mL/day, with flow partitioning. During the medium changes, a basal common medium was used for the gut, lung, liver, and endometrium MPSs, while the new MPS were supplied with their preferred maintenance media. Each basal medium was then allowed to mix throughout the course of the interaction, with media changes at 48-hour intervals. Functionality of each MPS was evaluated every 2-4 days up to 3 weeks, in comparison to isolated MPSs to benchmark the non-interacting MPS functions. Due to the dramatic reduction in the functionality of isolated pancreas MPS, islets were replaced with the fresh islets at day 12 for both interaction and in-isolation studies.

Results

1. 7-way MPS supports cell viability and functions for at least three weeks.

Transient albumin secretion kinetics, sustained gut and lung TEER values, and IGFBP-1 secretion profiles were established. The functionality of cardiac MPS, which was monitored by beat frequency, was well maintained during the study. N-acetyl aspartate (NAA) and c-peptide release profiles revealed that both the brain MPS and the pancreas were also functional up to 3 weeks. The comparison of the interaction results with the isolation results showed no negative effect of interaction on the MPS functionality. Increased NAA secretion and more sustained c-peptide secretion were observed during the interaction. The long-term MPS viability and functionality could be maintained in the 7-MPS platform for at least extended culture periods of three weeks.

2. "Orally" administered drug and its metabolite were distributed across MPSs in concentrations consistent with model pharmacokinetics predictions.

Exogenous drug studies with clinically-relevant concentrations are important to translate *in vitro* results to clinical outcomes. Pharmacokinetics of diclofenac (DCF), a nonsteroidal anti-inflammatory drug, was analyzed in the 7-MPS platform. The maximum measured plasma concentration, Cmax, of oral diclofenac *in vivo* varies between 2-6 $\mu$M (Davies NM, et al., Clin. Pharmacokinet. 33, 184-213 (1997)). 4'-hydroxy-DCF (4-OH-DCF) is the common metabolite of DCF.

To recapitulate clinically observed Cmax from oral delivery in the platform, diclofenac was added to the apical side of the gut MPS. The measured concentrations of DCF and 4'-hydroxy-DCF media across different MPS compartments fitted respective pharmacokinetic model predictions. The DCF dose was absorbed across the gut epithelial barrier, distributed to the liver MPS and subsequently to the mixing chamber and all the other MPS compartments. Metabolite 4-OH-DCF was produced in the liver MPS, circulated across the 7-way MPS platform, and was detected in all the others MPS compartments. Physiologically based pharmacokinetic (PBPK) model predictions on both DCF and 4-OH-DCF concentrations aligned well with the measured data, which indicated the platform functions in a deterministic manner consistent with biology predictions. The unbound intrinsic clearance (CL_int(u); i.e., the ability of liver to remove drug in the absence of flow) was estimated to be 13.90 $\mu$L/min, and approximately 19% of this clearance was estimated to be towards the formation of the 4-OH-DCF metabolite.

### *Example 6. Bioreactor devices for Microbiome and Multi-organ Interaction Studies*

Growing the broad range of microbial flora found in the gut requires, among other factors, the maintenance of an anaerobic environment. This is in direct opposition to the higher oxygen tension required for traditional mammalian cell culture (oxygen partial pressure of about 20 kPa). To reconcile these two opposing requirements, a micro-bioreactor that interfaces with commercial transwell culture inserts was developed. The device isolates the apical compartment of the transwell and allows for precise control of the environment on the luminal side of the model. Deoxygenated media with or without microbes can be infused through the luminal compartment, establishing a stable and tunable $O_2$ gradient.

A robust and user-friendly *in vitro* model to study gut epithelium-microbiome-immune (GuMI) homeostasis is presented in Figures (44A-46). The system allows controlled and isolated apical and basolateral flow to support co-culturing of primary human intestinal model with a defined gut microbiome model on the apical side and immune component on the basolateral side. Importantly, this fluidic platform can be modified to accommodate multiple parallel "guts" for higher throughput and ultimately integrated with the existing platform to study multi-micro organs interaction.

Materials & Methods

The fluidic plate for this example includes an integrated pumping system for controlling 3 replicate experiments, each containing two transwell-based MPS (12- or 24-well transwells with use of adapter insert) as well as a perfused scaffold. There are three additional wells that can provide support functions, such as adding/removing media, drugs, samples, and metabolites to the basal side of the 3-MPS flow loop (Figure 47).

An apical insert assembly (Figures 44A and 44B) is used with the fluidic plate and allows for oxygen gradient to be established in the multiwell culture system (Figure 45).

Results

Figure 44A is a diagram showing an apical insert assembly **3000** for use with standardized transwells and a fluidic plate **100.** The apical insert assembly includes an inlet point **375** and an outlet point **377.** Figure 44B is a diagram showing the apical insert assembly **3000** with compression fittings **400** and **402** for 1/16" OD tubing, four 0-80 screws **405a, 405b, 405c,** and **405d,** an apical insert **407,** an o-ring **409,** a standard 12-well transwell **412,** and a lower ring **410.** The apical inserts is designed to fit standardized transwells and to provide adequate seal to prevent fluid evaporation and contamination. This assembly controls flow conditions and oxygen tension. When inoculated with gut cells, the gut apical flow module (GAFM) is incorporated in a microphysiological system to study organ interaction (e.g. gut-liver (see Figures 45 and 47).

Figure 45 is a diagram showing a simplified version of an apical insert assembly **3000** with a transwell **412** seeded with a monolayer of 9:1 of Caco2/HT-29 cells receiving a feeding medium inoculated with commensal bacteria with different oxygen tolerability. The feeding medium for the apical insert assembly **3000** is supplied through the inlet point **375** (apical feed), and removed through the outlet point **377** (apical effluent), and is sealed in with the o-ring **409.** The apical insert assembly **3000** is suspended in a basolateral compartment **500** containing a medium with immune cells. The medium at the basolateral compartment **500** is supplied through a basal feed port **502** and removed through a basal effluent port **504.**

Commensal bacteria with different oxygen tolerability (e.g. *L. reuteri, B. fragilis, V. parvula)* will be inoculated and co-cultured with the monolayer of 9:1 Caco2/HT-29 cells. The Caco2/HT-29 cells are allowed to mature before they are used in GAFM. Immune cells, introduced at the basolateral compartment, allow for gut epithelium-microbiome-immune (GuMI) studies.

Figure 46 is a line graph showing the oxygen consumption rate as oxygen partial pressure (kPa) over time (hours) for Caco2-HT29 mixtures seeded on a membrane exposed to physioxia on apical side and normoxia on basal side. The oxygen consumption was measured at inlet oxygen sensor ((1), top line) and outlet oxygen sensor ((2), lower line) over time. Oxygen consumption rate of cells remained at steady equilibrium. Flow rate may be used as a parameter to control cell metabolization.

A desired oxygen tension is established by bubbling inert gas in the source. Apical flow is driven by pneumatic actuation. Real-time monitoring of oxygen tension at inlet and outlet measures $O_2$ consumption rate. Transmembrane pressure between apical and basal sides is minimized to regulate paracellular transport.

Figure 47 is a floor plan view of a three-organ culture system. The system includes a pneumatic control plate **3200** and a fluid handling plate **3100.** The platform allows 1, 2, or 3-organ interactions using perfused tissues or transwell-based models of varying size. Each platform houses 3 sets of interactions, each with dedicated reservoirs for automated media feeding and waste removal, as well as drug dosing. Delivery of drugs or hormones to the system can be automated to generate a desired concentration profile, and the component materials of the device are low-binding, making it more attractive than PDMS microfluidics for studies involving highly lipophilic or hydrophobic compounds. The reservoirs are programmable for automated feeding, drug dosing, sampling, waste removal.

***Example 7: Validation of capacitive sensing and feedback of fluid height changes.***

A linearity range of capacitance measurements achieved with the physical embodiment were determined. Comparative results from dynamic fluid tracking with its corresponding capacitance measurements demonstrated functionality in an archetypical fluid reservoir or square crosssection (L=10 mm). The data showed The data showed linearity of capacitance measurements. The entirety of the experiment follows the input and output of 1 mL of water with a total fluid height range of 10 mm.

The height measurement data was obtained during a constant flowrate experiment (Q=0.1 mL/min) in a closed-loop, feedback controlled system containing one gravity-driven pump and a sensor as shown in Figure 52. The slope of the fluid level increase over time was used to calculate the flow rate in the system without using an additional flow-through sensor.

The fluid height measurement data was obtained during a decreasing flow-rate ramp experiment (initial input flow rate Qo = 0.5 mL/min; a final input flow rate $Q_f$=1 uL/min) in a closed-loop, feedback controlled system containing one gravity-driven pump and a sensor as shown in Figure 52. Analysis of fluid height trends over different time sections allows for extraction of instantaneous flow measurements in the system without using an additional flow-through sensor. Here, the flow rate directed to the reservoir (e.g., to replenish fluid supply reservoir) is set/input by the user; and by monitoring the fluid level changes in the reservoir, the output flow rate is extracted.

The fluid height measurement data was obtained during an increasing flow-rate ramp experiment ($Q_0$ = 0.1 uL/min to $Q_f$ = 0.25 mL/min) in a closed-loop, feedback controlled system containing one gravity-driven pump and a sensor as shown in Figure 52. Analysis of fluid height trends over different time sections allows for extraction of instantaneous flow measurements in the system.

***Example 8: Hydraulic permeability measurement on a porous material within a meso- or microfluidic device.***

Direct measurement of hydraulic permeability of a porous material within a meso- or microfluidic device that is connected to a closed-loop, feedback controlled gravity-driven pump system was determined. The porous material can be synthetic or biological structures, such as biomimetic hydrogels, scaffolds and cells or bacteria from any origin, and any other material assembly exhibiting a defined hydraulic permeability. Assessment of hydraulic permeability in biomimetic hydrogels is of particular interest for cellular growth, matrix remodeling, and/or vascularization.

Hydraulic permeability data was collected through dynamic measurement of fluid height using a non-contact fluid level sensor (e.g., the one described in Figure 50) in conditions of passive gravity-driven flow with maintained base pressure at the outlet of the meso- and/or microfluidic device through passive or active means. A passive means can be a spillway, whereas an active means can be another pump. The base pressure is defined by the height Ho, which is the average vertical distance from the fluidic device midline to the gas/liquid interface in the secondary gravity-driven reservoir Ho. In other embodiments including a unidirectional gravity-driven pump arrangement, this distance is zero. Nonetheless, height decrease or changes is measured using a capacitive fluid-level sensor, followed by calculation of the desired permeability coefficient by a processing unit using direct computation or fitting to a negative exponential function as:

$$\Delta H(t) = \Delta H_0 e^{-ct},$$

Where $\Delta H(t)$ is the change in fluid height in the supply reservoir over time, $\Delta H_0$ is the initial delta height, t is time, and c is the slope calculated from the solution to the homogeneous differential equation of regression:

$$c = \frac{1}{t} \ln\left(\frac{\Delta H}{\Delta H_0}\right).$$

Data from an an exemplary measured fluid height decrease (delta z (m)) over time in the supply reservoir during a hydraulic permeability measurement of a porous material were obtained.

The flow of a fluid through a porous medium follows Darcy's law. At constant elevation, the instantaneous fluid discharge rate, Q ($m^3$/s), through a porous medium is show below:

$$Q = \kappa \times A \times \Delta P \div (\mu L),$$

Where $\kappa$ is the permeability of the medium ($m^2$), A is the cross-sectional area normal to the flow of the porous medium, $\Delta P$ is the total pressure drop over a length L of the porous medium, and $\mu$ is the viscosity of the fluid. And the $\kappa$ is derived to be:

$$k = \frac{\mu L A_r}{\rho g A} c,$$

that is

$$k = \frac{\mu L A_r}{\rho g A} \frac{1}{t} \ln\left(\frac{\Delta H}{\Delta H_0}\right),$$

where p is the density of the fluid, g is acceleration due to gravity, $A_r$ is the cross-sectional area in the x-y direction (normal to gravitation) of the gravity-dominated fluid reservoir.

Figures 57A-57C schematically illustrate the multifunctional aspects of a closed-loop, feedback controlled gravity-driven pump system, with corresponding exemplary fluid height changes shown in Figure 57D.

One aspect is the provision and the measurement of constant gravity-driven flow profile in either direction, shown in Figure 57A. To produce a constant gravity-driven flow rate, the fluid height of the main and secondary driving fluid reservoirs of the pump are maintained at a constant height using closed-loop feedback control.

Another aspect is assessing hydraulic permeability within the mesoand/or microfluidic device via measurement of the fluid height in the driving reservoir, shown in Figure 57B. To perform a hydraulic permeability assessment of at least one meso- and/or microfluidic channel, only the secondary driving fluid reservoir of the pump is maintained at a constant height using closed-loop feedback control, while the decrease in the fluid height in the primary driving reservoir over time is used to compute the permeability value of a porous material.

A third aspect is the provision and the measurement of dynamic gravity-driven flow profile in either direction, depicted in Figure 57C. Fully controllable bidirectional flow is achieved by actively modifying the fluid heights of both the primary and the secondary fluid reservoirs of the gravity-driven pump using a closed-loop feedback control.

### Example 9: Applications in cell-culture devices.

The placement of a flexible capacitive fluid-level sensor corresponding to the design in Figure 50 was to a multiwell biomimetic tissue culture plate.

Figures 58A and 58B depict the application of a miniaturized capacitive fluid level sensor according to Figure 50 for each well in a multiwell cell culture plate for high throughput assays, where a miniaturized, closed-loop feedback gravity-driven pumping system containing two driving reservoirs occupies two of every three-well set. This system uses a modified microtiter plate as substrate **4081,** in which multiple wells act as reservoirs to impose closed-loop control gravity-driven flows. In this configuration three adjacent wells are used, where one well as the main/primary driving reservoir **4082,** a second well as region **4083** for a meso- and/or microchannel **4084** (where gel, cells and or any porous material may be deposited), and at least one non-contact fluid-level sensor **4085** for monitoring and sending feedback. Multiple fluid level sensors may be integrated through the use of multiplexed electrodes **4086** allowing for closed-loop feedback control using minimal instrumentation such as a CDC **4087.** This instrumentation arrangement can be placed in the back **4088** of the microtiter plate substrate via electric trace printing to allow for low-cost manufacturing. Once fluid levels are assessed in the microtiter plate wells, fluid levels may be adjusted through micro pipetting (e.g., robotic pipetting) or other automated fluid handling systems.

### Example 10. Manufacture and assembly of closed-loop feedback control system with automated capacitive fluid height sensing.

This example provides an optimized self-shielded coplanar capacitive sensor design and automated control system to provide submillimeter fluid-height resolution (-250 $\mu$m) and control of small-scale open reservoirs without the need for direct fluid contact. Results from testing and validation of the sensor and system also suggest that accurate fluid height information can be used to robustly characterize, calibrate and dynamically control a range of microfluidic systems with complex pumping mechanisms, even in cell culture conditions. Capacitive sensing technology provides a scalable and cost-effective way to enable continuous monitoring and closed-loop feedback control of fluid volumes in small-scale gravity-dominated wells in a variety of microfluidic applications.

Materials and Methods

### Feedback-controlled gravity-driven pump setup

A schematic of the assembled closed-loop controlled gravity-driven microfluidic setup with capacitive fluid-level sensing is shown in Figure 2 of 62/556,595. The components of the setup were prepared as follows.

### Hydrostatic fluid chamber

The assembled hydrostatic chamber with capacitive sensing used in the feedback-controlled gravity-driven setup used a sterile 1mL PLASTIPAK® graduated syringe barrel (Becton Dickinson, Rutherford, USA) is oriented vertically and connected to a 1/16" polypropylene barbed quick-turn coupling socket (McMaster Carr, Robbinsville, USA) within a 3D printed structure to form a biocompatible and sterile reservoir with dimensions ($H_{total}$=60 mm, ID=4.78 mm). A 0.22 $\mu$m pore FISHERBRAND® filter (Thermo Fisher Scientific, Waltham, USA) was placed on top of the assembly to allow for air flow while maintaining sterility. This hydrostatic chamber was connected to the pumping and recirculation circuits through 1/16" ID polypropylene tubing and a compatible nylon tube-to-tube wye connector (McMaster Carr, Robbinsville, USA). Gravity-driven flow was determined by the height of the fluid column and the downstream resistance of the system. The microfluidic device was connected using standard tubing and located within the device holder to reliably control its vertical position with respect to the bottom of the fluid column. The non-contact capacitive fluid-level sensor was in close proximity to the fluid (~2mm) in the 3D printed structure so as to monitor fluid height.

Therefore, individual components used for the assembly of the monitored hydrostatic chamber used for the feedback-controlled gravity-driven setup were as follows. For assembly, the syringe barrel (of 1 mL syringe), barbed socket (syringe tubing connector), tubbing and air filter were connected together to be sterilized and then assembled onto the 3D-printed structure (reservoir and sensor holder). The capacitive sensor was inserted into a thin slot at the back of the 3D-printed structure that positions the sensor in close proximity to the syringe barrel and thus the fluid. The sensor can be permanently attached to the 3D-printed structure with resin or adhesive to avoid mechanical induced drift.

*Capacitive fluid-level sensor for microfluidics*

The capacitive fluid-level sensor for microfluidic applications consisted of self-shielded coplanar electrodes connected to an AD7746 24-Bit $\Sigma$-$\Delta$ capacitance-to-digital converter in differential mode (Analog Devices, Norwood, USA). Figure 49 illustrates the layout of this sensor in conjunction with the monitored fluid reservoir. In this design, two pairs of excitation electrodes (diagonal pattern in Figures 49 and 50) are positioned around two sensing electrodes (dotted pattern) to measure fringing capacitance in the direction of the fluid as shown in Figure 51. This electrode design is referred to as excitation-sensing-excitation/interdigitated arrangement (ESE-ID).

Two symmetrical gaps separate the excitation electrodes from the central sensing electrodes. The gap length is a parameter that affects the penetration depth of the most sensitive fringing pathways in other types of capacitive sensors using coplanar electrode arrangements. Thus, $L_{gap}$ can be iteratively adjusted to achieve optimal sensing in other applications with different fluid-sensor wall thicknesses. For the assembled hydrostatic chamber prototype, $L_{gap}$=0.75mm was heuristically determined (from several design iterations) as sufficiently small to allow for capacitive sensing using the electrode geometry.

The AD7746 chip was connected to the front sensing electrode via the $C_{sense}$(+) terminal, while the back sensing electrode is connected to the reference terminal $C_{ref}$(-) to allow for differential measurements (a schematic is presented in Figures 50 and 52). The excitation electrodes of both sensing and reference planes were connected to the same excitation (EXC) terminal. The differential mode of the AD7746 chip was selected to maximize the robustness of capacitance measurements, while the addition of a symmetrical reference arrangement at the back of the sensing layout was designed to maximize signal-to-noise ratio. This effect can be explained by referring to the expected fringing capacitance pathways on the presented mirrored electrode design (Figure 49), which shows a self-shielding effect within the sensor.

In commonly used capacitive sensing instruments, the region separating the electrodes from a target fluid is usually made of materials with low electrical permittivity ($\varepsilon$) such as plastic, glass or air. This renders the capacitance due to the plastic and air small as along as $\varepsilon_{Air} < \varepsilon_{Plastic} \ll \varepsilon_{Fluid}$ is maintained. Thus, the capacitance attributable to the region next to the fluid can be approximated to the total capacitance detected at the $C_{sense}$(+) terminal. Furthermore, in previously described coplanar sensor designs (Walker, C.S. Capacitance, inductance, and crosstalk analysis. Artech House (1990)), the reference electrodes were usually situated in the same plane as the main sensing electrode (next to a region constantly filled with fluid). This traditional configuration simplifies the sensor compensation for different kinds of fluids and temperature changes, but it also brings several limitations in terms of footprint, minimum detectable fluid volume and achievable signal-to-noise ratio.

The exemplary sensor prototype was implemented using a 3-layer flexible printed circuit board (Flex-PCB) with total thickness of 0.2 mm. Electrodes were defined as 0.5 oz copper layers with 17.5$\mu$m thickness, while a 55 $\mu$m polyimide film was used as dielectric. A layer of dielectric film was between the mirrored electrodes, as well as at the top and bottom of the sensor to protect the conductive material from corrosion (Figure 52). Connection traces between sensing circuit and electrodes used 0.127 mm traces. The electrodes were made with an original length Ltotal = 20 cm, and then were cut at the top end to fit the 6 cm fluid reservoir. This design allowed for use of the sensor in longer hydrostatic chambers with minimum modification of the sensor design. The separation of the mirrored reference electrode from the main sensing plane was achieved using a 75 $\mu$m adhesivepolyimide-adhesive dielectric layer. The charge distribution imposed by this design directed the fringing fields of the sensing electrode plane preferably towards the fluid, while directing the fields of the reference electrode plane towards the back of the sensor. This configuration was more compact than traditional coplanar capacitive sensors, and enabled the detection of smaller increases in fluid height and more effective compensation of external parasitic capacitances (e.g. user's movement). For this configuration, the capacitance associated with the sensing and reference coplanar electrodes can be approximated as:

$$C_{sense}(+) \approx \frac{\pi * \varepsilon_o * \varepsilon_s}{\ln\left(\frac{\pi(d-w)}{w+t_c}+1\right)} H_{Fluid}$$

Equation 4.1

$$C_{ref}(-) \approx \frac{\pi * \varepsilon_o * \varepsilon_R}{\ln\left(\dfrac{\pi(d-w)}{w+t_c}+1\right)} L_{sensor}$$

Equation 4.2

where $C_{sense}(+)$ and $C_{ref}(-)$ are the capacitances of the sensing and reference electrodes respectively, $d$ is the average diameter of the fringing arcs between the sensing and the excitation electrodes. $L_{sensor}$ is the total length of the sensor, $H_{Fluid}$ is the fluid height, $t_c$ is the thickness of the electrode conductor (assumed to be constant across all electrodes), and $\varepsilon_o$ is the permittivity of free space ($\varepsilon_o \approx 8.9 \times 10^{-12}$ F/m). Due to symmetry, equations (4.1) and (4.2) are only valid for the case in which the width of the sensing electrode (w) is approximately equal to the added width of both excitation electrodes. The relative permittivity associated with the sensing region ($\varepsilon_s$) and the reference region ($\varepsilon_R$) in equations (4.1) and (4.2) can be determined by examining the ratio between the average diameter of fringing arcs (d) and the thickness of the material separating the fluid from the conductor *(tw)*. The thickness *tw* considers the flex-PCB dielectric and plastic wall and is only used to determine $\varepsilon_s$ and $\varepsilon_R$ according to the following rules: For $d/t_w \gg 1$, $\varepsilon_s = \varepsilon_R \approx 1$; whereas for $d/t_w \approx 1$, $\varepsilon_s = (1+ \varepsilon_{fluid})/2 \approx 40$ and $\varepsilon_R = (1+ \varepsilon_{plastic})/2 \approx 1$.. All previous approximations assume a relative permittivity for the fluid ($\varepsilon_{fluid}$) around 80 at 20 °C under a 1 kHz excitation, while $\varepsilon_{plastic} \approx 2$ for a bulk plastic dielectric material. Since the testing setup has $d = 2.5$ mm and $t_w = 2$ mm, it follows that $d/t_w = 1.25 \approx 1$ confirming that the second ratio condition applies. Dividing $C_{ref}(-)$ from $C_{sense}(+)$ and reordering terms toapproximate the fluid level in the reservoir as follows:

$$\Delta H_{Fluid} \approx \left(\frac{\varepsilon_R}{\varepsilon_S}\right)\frac{C_{sense}(+)}{C_{ref}(-)} L_{sensor} - H_{offset} \quad \text{Equation (4.3)}$$

where $\varepsilon_R/\varepsilon_S = (1+ \varepsilon_{plastic})/(1+ \varepsilon_{fluid})$ is just the proportionality constant in equation (4.3), which depends on the target fluid and the bulk dielectric material. This constant can be calculated for compensation purposes by performing a single capacitance measurement at a known fluid height. Finally, an additional compensation offset ($H_{offset}$) may be used to correct for mechanical inaccuracy due to sensor placement and to adjust for absolute height according to a common height reference as seen in equation (4.3).

*Electronic feedback-control hardware*

After acquisition of capacitance readings by the AD7746 chip, the digitized measurements were transmitted to a microcontroller board HUZZAH® ESP8266 Feather (Adafruit Industries, New York, USA) via a ZIF connector and I2C serial communication terminals to perform feedback control computations. This board was selected as the control hardware due to its low-cost, ease of programming (using ARDUINO® syntax, Arduino AG Corporation, Cham, Switzerland), availability of open-source design files, power efficiency and integrated wireless capabilities. This microcontroller board was then connected to a stackable custom-made active pumping board, and a FeatherWing OLED I/O interface board (Adafruit Industries, New York, USA) with assembled push buttons and a screen for offline parameter visualization.

*Bidirectional supply pumps*

In order to actively control fluid height within the hydrostatic fluid chamber, two Bartels Microtechnik mp6 piezoelectric pumps (Servoflo, Lexington, USA) are driven in a bidirectional configuration by the pumping board using two commercially available mp6-OEM driver circuits (Servoflo, Lexington, USA). The flow-rate of these piezoelectric pumps was controlled using a pulse-width modulated (PWM) signal generated by two independent output channels from the HUZZAH® ESP8266 Feather. This type of pump was selected due to its small size, fast response, high dynamic range, and chemical inertness. Other active bidirectional pumps may be used as long as their response time is faster than the characteristic time constant of the fluidic plant to be controlled.

*Control and user-interface software*

The AD7746 acquisition routine and a proportional-integral (PI) control law were implemented using the ARDUINO® in-system programmer (ISP) on the ESP8266 microcontroller unit. The ARDUINO® and ADAFRUIT® ESP8266 (Fried, Limor DBA Adafruit Industries, New York, New York, USA) libraries were also used to facilitate integration of this platform. Capacitance measurements were obtained every 10 milliseconds using a timer-driven interrupt. A sensor calibration routine was also implemented, so as to allow the user to record a base capacitance offset as well as the calculation of the fluid-dependent proportionality constant ($\varepsilon_R/\varepsilon_S$). Routines to follow constant and pre-programmed dynamic fluid height profiles were also implemented in non-volatile memory of the ESP8266 (EEPROM). Acquired data and system control parameters were transmitted via USB and wirelessly via Wi-Fi to a laptop and then converted to CSV format for analysis.

*Microfluidic devices and fluidic circuit*

Two simple microfluidic devices were used to test usability and validate functionality of the feedback-controlled gravity-driven setup. These microfluidic devices were: 1) A custom fabricated single channel polydimethylsiloxane (PDMS) chip, and 2) A commercially available chip for cell culture as described below. For most of the tests the output channel of the driven microfluidic device was connected to a dripping recirculation pathway that maintained the outlet of the microfluidic channel at atmospheric pressure as depicted in Figure 49. It is also possible to use two of these hydrostatic pressure chambers at both ends of the microfluidic chips to allow for bidirectional gravity-driven flow, as well as regulating the device average pressure.

*Structural and other components*

Structural elements such as the sensor/chamber holder, controller box and microfluidic device holder were fabricated from stereolithographic (SLA) resin using a FORM 2® 3D printer (Formlabs, Somerville, USA). The hydrostatic chamber and sensor holder were made from clear resin to allow for direct optical visualization of the monitored fluid front for validation purposes.

Results

***Accurate capacitance fluid sensing and fluid tracking***

The results of the initial characterization experiments used to assess the accuracy of capacitance readings and basic fluid level tracking capabilities of the sensing circuit are shown in Figures 53A-53C. Measured values of known capacitors directly connected to the $C_{sense}(+)$ and $C_{EXC}$ terminals in the sensing circuit appear to match those of the E4981A capacitance meter within <0.1 pF error (Figure 53A). Readings from the capacitive sensing circuit also appear to be linearly related to those obtained from the calibrated reference meter suggesting appropriate implementation of the sensor at the PCB level. Figure 53B shows the aggregated results for three basic fluid-level tracking challenges using the fluid chamber with the proposed excitation-sensing-excitation inter-digitating capacitive sensor arrangement (ESE-ID). The dashed line in Figure 53B is the known fluid-height as imposed by the calibrated syringe pump. Solid points refer to the averaged capacitive sensor output for the three replicates at each time point. The average standard deviation across all samples in this experiment was

<250 $\mu$m when compared to the fluid height imposed by the calibrated syringe pump. Figure 53C shows both measured and estimated flow-rates in the gravity-driven pump as a function of set fluid height. Experimental measurements closely followed the theoretical values predicted by Poiseuille's equation.

***Example 11. Validation of closed-loop feedback control system with automated capacitive fluid height sensing.***

Materials and Methods

***System testing and validation***

*Validation of capacitance measurements*

In order to characterize the accuracy of the capacitance measurements obtained by the sensor, the AD7746 circuit of the Flex-PCB was isolated. Capacitors of known values were placed between the sensor's $C_{sense}(+)$ and EXC terminals to record their values. The tested capacitances were verified using a calibrated E4981A capacitance Meter (Keysight Technologies, Santa Rosa, USA) for comparison purposes. The results of this test are shown in Figure 53A.

*Basic fluid-height tracking*

After characterization of the sensing circuit, a basic fluid height "challenge" within an isolated hydrostatic chamber with an embedded capacitive sensor was performed in triplicate using a 1x phosphate buffered saline (PBS) solution.

The fluid level in this reservoir was controlled using a calibrated syringe pump 11-PicoPLUS Elite (Harvard Apparatus, Holliston, USA). This pump was programmed to produce a dynamic oscillating change in fluid volume over the reservoir's entire height range (6 cm) during a 20 s period. Readings from the capacitive sensor were taken every 10 ms (without averaging) and compared to the fluid volume supplied by the syringe pump. Fluid height in the chamber was also verified using video recordings and image processing to track the fluid-air interface visualized through the translucent regions of the fluid reservoir. The results are shown in Figure 53B.

*Gravity-Driven Flow Rate Validation*

To validate flow rates established using a given hydrostatic pressure (i.e. fluid height), the pump was connected to a 50 cm long silicone tube with inner diameter of 1/32" and the mass of 1x PBS displaced over a one minute duration (n=3) was measured using a laboratory grade scale. The Poiseuille equation was used to calculate the expected flow rate given a hydrostatic pressure difference $\Delta P=8\mu LQ/(\pi R^4)$, where $\Delta P$ is the hydrostatic pressure difference, L is the length of the tubing, $\mu$ is the dynamic viscosity of PBS which is assumed to be close to that of water ($8.9\times10^{-4}$ Pa.s at 25 °C), Q is the volumetric flow rate and R is the inner radius of the tubing. Additionally, the hydrostatic pressure was calculated as $\Delta P = pgh$, where p is the density of PBS which is approximately that of water (1 kg/m$^3$), g is gravity and h is the total fluid height above the outlet feeding to the collection tubes used for weight measurement. In order to achieve high flow rates, an additional 53 mm offset was added to the fluid column by placing the collection tubes below the gravity driven pump outlet. The obtained flow-rate measurements (in triplicate) were compared to expected values using Poiseuille's equation as shown in Figure 53C.

*Characterization of fluid-type dependency*

The gain of the capacitive sensor design is expected to change depending on the charge distribution resulting from the conductivity and the electrical permittivity of the target fluid. Therefore, changes in charged solute concentration can affect these readings significantly. To characterize such effect, a titration experiment was conducted in triplicates using 1x, 0.5x, 0.25x, 0.125x and 0.0625x PBS, with deionized (DI) water and a steel inserts of known lengths as controls. Two inserts were made by cutting 10- and 30-mm sections from a tight-tolerance multipurpose O1 tool steel rod with 0.1750" diameter (McMaster Carr, Robbinsville, USA). The associated capacitance value from all fluid conditions and materials was recorded at two fixed fluid heights: lower Bound (H0=10 mm) and upper bound (H1=30 mm) as shown in Figure 54A. To confirm experimental results, the electric displacement flux density (D) around relevant target fluids (i.e. 1xPBS solution, DI-water) was computed, via finite element analysis (FEA) using the AC conduction field solver of ANSYS® Maxwell software (ANSYS, Inc., Canonsburg, USA). In these simulation, the two middle electrodes were fixed to zero voltage, and the four electrodes on the side were excited with a sinusoidal voltage of 5V amplitude at an excitation frequency $f_{EXC}$ = 32 kHz. These conditions were analogous to the settings used by the AD7746 24-Bit $\Sigma$-$\Delta$ capacitance-to-digital converter in the developed sensor.

*Flow-rate tracking and calibration of external pneumatic micropumps*

A series of proof-of-concept experiments were conducted to demonstrate flow-rate extraction and open-loop pumping calibration based on continuous fluid-level monitoring. The same previously described fluid-height tracking methodology was used, except that in these experiments, the calibrated syringe pump was programmed to impose specific flow-rate profiles (instead of volumetric changes) ranging from 100 nL/min to 1 mL/min These conditions included constant, ramp and intermittent flow-rates in input and output mode. Fluid-height changes over time were measured in triplicates and compared to theoretical flow estimates using the pre-programmed flow-rate parameters imposed by the calibrated syringe pump. Extraction of flow-rate was approximated by generating a linear fit of fluid volume change over time every 1000 samples (t=10 s). Experiments were limited to a maximum input/output volume ($V_{max}$=0.5 mL) and a maximum experimental period (T < 400s).

In a subsequent experiment, this height-based flow-rate tracking methodology was used to characterize and calibrate two presumably identical open-loop pneumatic diaphragm micropumps. These micropumps were set to supply and extract fluid from the same monitored reservoir at 1 $\mu$L/s. These pumps were fabricated in acrylic using a CNC mill and a thin polyurethane membrane according to a previously reported protocol (Inman et al., Journal of Micromechanics and Microengineering, 17(5):891 (2007)). Both pumps were actuated for 40 min at 1 Hz (stroke volume=1 $\mu$L) under 37 °C and 95% humidity to observe fluid-height drift (in triplicate). After confirming adequate operation of both pumps, any observed volume drift in the monitored reservoir was assumed to be caused by small differences in input/output pumping performance attributable to fabrication variations or head pressure effects. After combined drift characterization, flow-rates were independently tracked in triplicate for each pump to recalibrate their actuation frequency and adjust for errors. After calibration, fluid-height drift was again characterized for 40 min and compared against uncalibrated behavior (n=3) as shown in Figures 55A-55C.

*Validation of closed-loop control of gravity driven pump*

The performance, robustness and dynamic range of the proposed closed-loop feedback control system was characterized using the entire monitored setup. A single-channel microfluidic device made in a PDMS (length=50 mm, width=1 mm and height=0.2mm) was connected to the outlet of the hydrostatic chamber with capacitive sensing. After the chip was connected to the setup, sensor calibration and flow testing was performed to assess the emptying time constant of the hydrostatic chamber given the fluidic resistance from the connected microfluidic chip. After this, a closed-loop feedback control mode was activated to automatically control fluid input/output from the secondary piezoelectric pumps to maintain a target height ($\Delta$H=30 mm). For this experiment, the hydrostatic chamber was monitored over a period of 48 hours inside an incubator (at 37 °C, 5% $CO_2$ and 95% humidity). Presence of flow through the microfluidic device was confirmed by observing dripping fluid within the secondary recirculation container. This experiment was conducted in triplicate and the fluid height was also verified using video recordings of the fluid front to assess drift.

After verifying adequate closed-loop performance for a constant fluid-level set-point, a dynamic fluid-level target experiment was conducted under similar experimental conditions. However, in this case the target was pre-programmed to be a dynamic fluid-level profile stored in non-volatile memory of the MCU. This profile was a 40-min sequence including constant, sine, triangular, saw tooth and step waveforms (two periods each). This experiment was also conducted in triplicate within an incubator (37 °C at 95% humidity) using video recordings to verify location of the fluid front.

*Cell culture experiments*

In order to show biocompatibility of the setup, a 24-hour cell culture experiment was performed using iPSC-derived vascular endothelial cells as an example cell type. In this test, an IBIDI® µ-Slide VI 0.4 channel slide (IBIDI, Martinsried, Germany) was coated with human fibronectin (Life Technologies, Woburn, USA) at a concentration of 30 µg/mL for 1 hour at room temperature. Induced Pluripotent Stem Cell (iPSC) derived endothelial cells (CDI, Madison, WI) were seeded in all the channels, allowed to adhere for 3 hours then excess cells were washed away using two successive media changes. The device was cultured for 24 hours in the supplier-recommended media under standard incubator conditions. After assembly, the gravity-driven setup was sterilized by circulating 70% ethanol through the entire fluidic circuit for 5 min. Ethanol was removed by air drying within a sterile hood and then flushing with two cycles of sterile DI water. After sterilization, the seeded IBIDI® µ-Slide VI microfluidic channels were connected to the gravity-driven setup within a sterile hood. The hydrostatic chamber was then programmed to maintain a constant 40 mm fluid-height (392 Pa inlet pressure) to drive flow through the chip which had its outlet at atmospheric pressure through the dripping recirculation circuit. Laminar flow was assumed given the imposed pressure gradient and the rectangular design of the IBIDI® device channel (length=17 mm, width=3.8 mm and height=0.4 mm). The Poiseuille equation was used to calculate the expected flow rate given a hydrostatic pressure difference Q = $\Delta$P/R; where Q is the volumetric flow rate, $\Delta$P is the hydrostatic pressure difference (392 Pa for a 40-mm media height) and R is the total resistance of the fluidic circuit ($R_{channel}$ + $R_{tubing}$). For the IBIDI® channel, which has a rectangular cross section, the fluidic resistance can be calculated as

$$R_{channel} = \frac{12\mu L}{wh^3(1-0.63\frac{h}{w})} = 6.21 x 10^8 \, Pa \cdot s / m^3$$

where $\mu$ is the dynamic viscosity of media which was assumed to be close to that of water (6.94×10$^{-4}$ Pa.s at 37 °C), *L* is the length of the channel, w is the width and *h* is the height.

For the tubing, which has a circular cross-sectional area with inner diameter 1/32 inch and total length of 20 cm, the fluidic resistance can be calculated as:

$$R_{tubing} = \frac{8\mu L}{\pi r^4} = 1.48 x 10^{10} \, Pa \cdot s / m^3$$

where *r* is the inner radius of the tubing, L is the length of the tubing, $\mu$ is the dynamic viscosity of media which was assumed to be close to that of water (6.94×10$^{-4}$ Pa·s at 37 °C). Using this information, the flow rate Q was calculated to be $2.64 \times 10^{-8}$ m$^3$/s or 1.59 mL/min at this specific height. The wall shear stress, $\tau$ imposed on the endothelial cells under this flow rate can be calculated as:

$$\tau = \frac{6\mu Q}{h^2 w} = 1.8 dyne/cm^2$$

.

Here $\tau$ is in dyne/cm$^2$, $\mu$ is in poise, $Q$ is in cm$^3$/s and $h$ and w are in cm and are the height and width of the IBIDI® channel respectively. After 24 hours of culture, cells were fixed with 4% PFA, stained with DAPI and Rhodamine and imaged under an EVOS® inverted microscope (Life Technologies, Woburn MA, USA) with a 20x objective.

### *Statistical analysis*

All validation experiments were conducted in triplicates. Error bars represent standard deviation in all figures. Calculations and plots were generated with Graphpad Prism 7 (GraphPad Software Inc.; La Jolla, USA).

### Results

### *Capacitance readings depend on fluid conductivity*

Figure 54A shows the results from the titration experiments conducted to characterize performance of the capacitive sensor based on fluid conductivity. Capacitance readings for upper (H1=30 mm) and lower (H0=10 mm) levels were smallest and closest together for deionized (DI) water and increased as electrolyte concentration increased to 1x PBS (conductivity @25°C = 1.6 S/m). Small steel rods of both lengths were also placed inside the monitored reservoir to assess signal in the presence of a known perfect conductor. Higher dilutions (0.5x to 0.0625x PBS) led to a range reduction and lower absolute capacitance, reaching a minimum in DI-water lacking solutes (conductivity @25°C = 0.055 $\mu$S/cm).

This behavior can be explained by the model shown in Figures 54B and 54C. In the case of water with dissolved solutes forming free ions (Figure 54B), a large number of sensing fringing paths become terminated near the fluid-reservoir boundary leading to higher capacitance measurements. Conversely, in the case of pure water (Figure 54C), fringing capacitance takes longer uninterrupted paths through the bulk of the fluid, generating smaller capacitance measurements. The flux density around the 1xPBS solution $\varepsilon_{r,PBS}$ = 80, $\sigma_{PBS}$ = 1.45 S/m). The charge relaxation time of the 1xPBS solution is $\tau_{PBS}$ = $\varepsilon_{r,PBS}$ $\varepsilon_0/\sigma_{PBS}$= 488 ps, which corresponds to a break frequency $f_{PBS}$ = 2.04 GHz. This break frequency is orders of magnitude higher than the 32 kHz sensor excitation frequency used for the sensor.

Since $f_{PBS}$ » $f_{EXT}$, the 1xPBS solution behaves like a conductor, accumulating free charges on its surface and increasing the apparent capacitance seen from the sensor electrodes. The induced surface charges terminate the flux density vectors. For the case of DI-water ($\varepsilon_{r,DI}$ = 80, $\sigma_{DI}$ = 5.5 $\mu$S/m), Figure 54C shows the field lines schematically. The charge relaxation time of the DI-water is $\tau_{DI}$ = $\varepsilon_{r,DI}$ $\varepsilon_0/\sigma_{DI}$ = 129 $\mu$s, which corresponds to a break frequency $f_{DI}$ = 7.76 kHz. Since $f_{DI}$ < $f_{EXT}$, the DI-water behaves like an insulator, which explains why the flux density vectors induced inside the DI-water follows the pattern shown in Figures 54C. These results show that calibration is required to adjust for potential differences in fluid conductivity. Further sections of this work make use of sensors calibrated for 1x PBS and culture media.

### *Accurate flow-rate tracking and calibration of external pneumatic micropumps*

The results of an extended series of experiments carried out to verify if accurate flow-rate calculations were achievable from analyzing fluid-height changes over time using the capacitive sensor were obtained. Inflow and outflow was tested for constant 100 $\mu$l/min flow-rate, as well as for increasing and decreasing ramps (ranging from 100 nl/min to 1 mL/min). Inflow and outflow experiments were also conducted for a step-like profile with 100 $\mu$l/min amplitude. Flow rates were calculated every 10s and compared with flow rates know from simulations. Constant and dynamic additions or extractions of fluid generated linear fluid height increase and decrease profiles that were similar for both simulations and experimental results. Increasing and decreasing ramp profiles generated second order profiles similar to those predicted by simulations. Similar results were found for the step flow profiles. From these experiments, it was confirmed that a variety of flow-rate conditions can be inferred based on this sensor's output and that these values correspond to the programmed settings in the calibrated syringe pump used to impose flow.

Figures 55A-55C show the results of the characterization and calibration experiment of two open-loop pneumatic diaphragm micropumps feeding and extracting fluid from the same monitored reservoir at a nominal rate of 1 μL/s. Figure 55A is a diagram of a testing block setup **4100** with supply reservoir **4110** fluidically connected to a six pump block **4114** containing pneumatic diaphragm micropumps and pneumatic lines **4116**. The fluidic connection is achieved with tubing **4112a** and **4112b**. The pump block **4114** is fluidically connected to a monitored reservoir **4130** containing a capacitive sensor **4132**. Fluid enters the monitored reservoir **4130** via the output pump **4122** and leaves the monitored reservoir **4130** via the input pump **4120**.

Over the course of 40 min, a 0.2 mL decrease in fluid volume was observed in the monitored reservoir (Figure 55B), showing that the output pump flow-rate was slightly greater than the input pump flow rate despite being actuated at the same frequency.

Independent flow-rate characterization of each of these micropumps using the capacitive sensor revealed an 8.3% mismatch between the output and input micropump flow rates. This difference explains the observed decreasing height drift and is well within the 10-15% expected error usually reported for these type of pumping systems (Domansky et al., Lab on a chip, 10(1):51-58 (2010)). After recalibration of the actuation frequency for both micropumps based on these readings, fluid-height drift appeared to be corrected for the same 40-min period as compared to the uncalibrated behavior. In general, long-term drift is expected to be present for a wide range of open-loop micropumps, due to back pressure, use-induced stress or solute deposition may sporadically change stroke volume. Thus, the sensor is a valuable addition to open-channel microfluidic systems requiring accurate flow control, or as a new way to assess flow-rates and perform pump calibrations on demand.

### *Reliable closed-loop control of gravity driven pump*

From available approaches for fluid handling in micro- and mesofluidic devices, gravity-driven systems have historically been considered among the most robust, simple and convenient to use. Reservoirs acting as gravity-driven pumps are relatively low-cost, can achieve a wide range of flow-rates, rarely lead to bubble stagnation and usually do not require external power to impose flow (Chang et al, Biomicrofluidics, 8(4):044116 (2014)). However, traditional gravity-driven pumps (with vertically positioned reservoirs) can only produce unidirectional transient flows as the liquid level in the reservoir decreases. This situation leads to a time-dependent reduction in achievable flow rate proportional to the decline in hydrostatic pressure. This transient mode of operation is a key limitation of most gravity-driven systems, especially in long-term cell culture applications.

Recent modifications of gravity-driven systems have been reported to provide nearly constant flow rates either through the use of horizontal reservoirs (setting a deterministic internal fluid height) or through the use of a large vertical reservoir (maintaining fluid heights nearly constant during limited operation times) (U.S. Patent No. 7,704,728; Kim et al., Microtechnology for Cell Manipulation and Sorting, 175-192 (2017); Lee et al., Biotechnology Progress, 28(6):1466-1471 (2012)).

Despite the advantages that these modifications may provide, most currently reported gravity-driven microfluidic devices still remain open-loop in nature, are cumbersome to continuously monitor and cannot deliver bidirectional, smooth, reconfigurable flow over long periods of time. Without closed-loop feedback control, the emptying time for an initial 30 mm fluid column at the hydrostatic chamber in connection with the used single-channel microfluidic chip was approximately 265 sec.

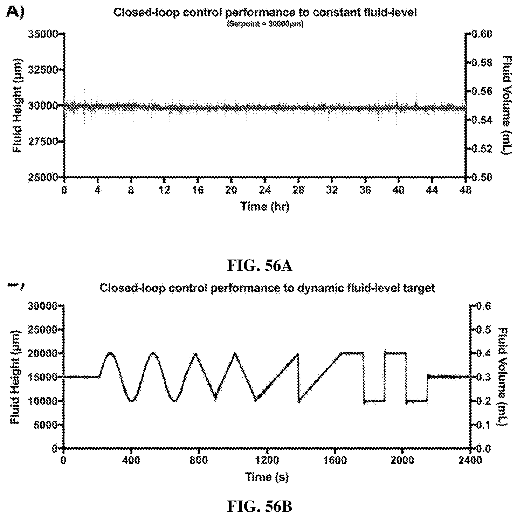

The data were obtained for the typical closed-loop response provided by the gravity-driven microfluidic setup to a static and dynamic target. Figures 56A and 56B show the typical closed-loop response provided by the gravity-driven microfluidic setup to a static (Figure 56A) and dynamic (Figure 56B) target. Both graphs show the aggregated height measurements for three experimental replicates as a function of time. Capacitive readouts were acquired every 10 ms, but were continuously averaged over 10 samples (T=100 ms) in both cases to feed the control algorithm. The maximum standard deviation across the entire 48-hr testing period for the constant target set point ($\Delta H$=30 mm) was 0.65 mm. The results were obtained over a 40-min period using a dynamic pre-programmed set point. The constant, sine, triangular, saw tooth and step waveforms were all recognizable and accurately followed with less than 5% error. Overshooting decaying oscillations were observable at the high-frequency transitions for both step-like cycles, which is characteristic of many second order systems using closed-loop feedback control as it reflects the control loop dynamics. In the augmented gravity-driven setup, fluid height appears to be a useful target variable allowing to accurately control both pressure and flow-rate in this system. While these variables are commonly measured and used to control large-scale gravity-dominated fluidic systems (volumes > 10 L) (Stillinger et al., Journal of Fluid Mechanics, 131:73-89 (1983); Ali and Foss, Experiments in fluids, 19(4):250-254 (1995)), this is the first time that capacitive sensing has been adapted to control such parameters in an openwell microfluidic system.

### *Biocompatibility for continuous cell culture*

The assembled and sterilized gravity driven setup was used for cell culture proof-of-concept. Optical analysis of both static and gravity-driven cultures confirmed cell viability. No differences in endothelial growth was observed in the test samples compared to the control, which suggests adequate biocompatibility of the designed fluidic circuit, as expected due to the use of inert materials in the system's fluidic pathways. Given the low shear stress the cells were exposed to (1.8 dyn/cm$^2$), no alignment of cells in the direction of flow was expected as such a response for endothelial cells typically requires a shear >10 dyne/cm$^2$ and in previous characterization of iPSC-derived endothelial cells a 20 dyne/cm$^2$ shear stress was used.

**Claims**

1. A fluidic multiwell device with an on-board pumping system comprising:

   (a) a first plate (100), the first plate comprising:

      two or more wells (103) comprising

         a three-dimensional space (1103) in each well defined by a bottom surface and a circumferential wall; and
         an inlet and an outlet in each well;

      a spillway conduit (121) positioned between the at least two wells (103i, 103j), having geometries that allow unidirectional fluid connectivity from above the bottom surface of a first well to a second well;
      a network of fluid paths (101) providing fluid connectivity between at least two of the wells through the inlet and the outlet of each of the two wells;

   (b) a detachable second plate (200), the detachable second plate comprising:

      a plurality of internal channels, each with an inlet opening (210) and an outlet opening (210) on opposing sides of the second plate,
      and one or more holes on the surface of the second plate in connection with each of the internal channels; and

   (c) a barrier membrane (300) positioned between the fluid paths of the first plate and the one or more holes on the surface of the second plate wherein the barrier membrane is at least partially flexible, such that applying a pressure to the internal channels of the second plate causes the membrane to move, thereby obstructing or clearing a portion of the fluid paths of the first plate, and
   (d) the device further comprising an apical insert (407) for culturing cells and an o-ring (409) arranged for sealing around the apical insert in a state in which the apical insert is suspended in a well of the fluidic plate.

2. The device of claim 1 wherein the detachable second plate and the barrier membrane form one or more pump units with at least a portion of the fluid paths of the first plate.

3. The device of claim 2, wherein each of the pump units comprises a pump chamber in the center and at least two valve chambers configured to be fluidically connected with the pump chamber when the barrier membrane is flexed.

4. The device of any one of claims 1 to 3 wherein the cells are selected from the group consisting of bacteria, fungi, yeast and combinations thereof.

5. The device of any one of claims 1 to 4 wherein the cells are from a microbiome present in the gastrointestinal tract, oral cavity, nasal cavity, vagina, or combination thereof.

6. The device of any one of claims 1 to 5 in a system comprising more than one device, each device creating one or more organ equivalents.

7. The device of any one of claims 1 to 6, wherein the apical insert includes an inlet point (375) and an outlet point (377).

8. The device of any one of claims 1 to 7, wherein the apical insert is configured to provide fluid and the cells to at least one of the two or more wells and to remove the fluid and the cells from the at least one of the two or more wells.

9. The device of any one of claims 1 to 7, wherein the apical insert further comprises an inlet oxygen sensor and/or an outlet oxygen sensor.

10. A meso- and/or microfluidic system with closed-loop feedback control, comprising:

   a) at least one open reservoir (4001) for fluid;
   b) the fluidic multiwell device according to any one of claims 1 to 9, whereby at least one meso- or microfluidic channel of said fluidic multiwell device is in communication with the reservoir; and
   c) an automatable sensor (4007) to detect fluid height in the reservoir and provide corresponding signal as feedback;

   wherein the signal corresponding to dynamic changes of the fluid height in the reservoir compared to a reference input indicates dynamic flow rate of fluid through the mesoor microfluidic channel.

11. A meso- and/or microfluidic device, comprising:

   a) a gravity-driven pump comprising at least one gravity-dominated fluid supply reservoir (4001);
   b) a sensor (4007) to detect fluid level in the fluid supply reservoir and provide corresponding signal as feedback and capable of adjusting fluid level in the supply reservoir to form a closed-loop feedback control system with the gravity-driven pump;
   c) the fluidic multiwell device according to any one of claims 1 to 9, whereby at least one meso- and/or microfluidic channel of said fluidic multiwell device is in communication with the fluid supply reservoir;

   wherein the fluid level in the supply reservoir drives gravity-dominated flow through the meso and/or microfluidic channel according to a reference input.

12. The fluidic device of claim 11, comprising a plurality of gravity-driven pumps and a closed-loop feedback control system, wherein the flow through the meso and/or microfluidic channel has a flow rate decoupled from changes in hydrostatic pressure in the fluid supply reservoir.

13. The fluidic device of claim 11, wherein the gravity-driven pump comprises two gravity-dominated fluid supply reservoirs (4001, 4003) in fluidic communication through the at least one meso- and/or microfluidic channel, and changes in the heights of fluid in the two fluid supply reservoirs drive bidirectional constant and/or dynamic flows through the meso- and/or microfluidic channel.

14. The fluidic device of claim 11, further comprising a recirculation connection, wherein fluid exiting the mesoand/or microfluidic channel is recirculated to the gravity-driven pump.

15. The fluidic device of any one of claims 10 to 14, wherein the fluid supply reservoir comprises a defined hollow structure with a constant cross section for at least the depth detectable by the sensor.

16. The fluidic device of any one of claims claim 10 to 15, wherein the sensor comprises a non-contact capacitive fluid sensing system.

17. The fluidic device of any one of claims 10 to 16, wherein the sensor is connected to at least one computing processing unit and/or a microcontroller unit (4010), directing fluid to be supplied to or extracted from the reservoir based on the feedback and/or a reference input.


**Patentansprüche**

1. Fluidische Multiwell-Vorrichtung mit einem Bordpumpsystem, die umfasst:

   (a) eine erste Platte (100), wobei die erste Platte umfasst:

      zwei oder mehr Näpfchen (103), die umfassen:

         einen dreidimensionalen Raum (1103), in dem jedes Näpfchen von einer Bodenoberfläche und einer Umfangswand definiert ist; und
einen Einlass und einen Auslass in jedem Näpfchen;

eine Überlaufleitung (121), die zwischen den mindestens zwei Näpfchen (103i, 103j) positioniert ist, die Geometrien aufweist, die unidirektionale Fluidverbindungsfähigkeit von oberhalb der Bodenoberfläche eines ersten Näpfchens zu einem zweiten Näpfchen aufweist;
ein Netzwerk aus Fluidpfaden (101), das Fluidverbindungsfähigkeit zwischen mindestens zwei der Näpfchen durch den Einlass und den Auslass jedes der zwei Näpfchen bereitstellt;

(b) eine abnehmbare zweite Platte (200), wobei die abnehmbare zweite Platte umfasst:

eine Vielzahl innerer Kanäle, jeweils mit einer Einlassöffnung (210) und einer Auslassöffnung (210) auf entgegengesetzten Seiten der zweiten Platte,
und ein oder mehr Löcher auf der Oberfläche der zweiten Platte in Verbindung mit jedem der inneren Kanäle; und

(c) eine Sperrmembran (300), die zwischen den Fluidpfaden der ersten Platte und dem einen oder den mehreren Löchern auf der Oberfläche der zweiten Platte positioniert ist, wobei die Sperrmembran mindestens teilweise derart biegsam ist, dass das Anlegen eines Drucks an die inneren Kanäle der zweiten Platte die Membran veranlasst, sich zu bewegen, wodurch ein Abschnitt der Fluidpfade der ersten Platte verlegt oder freigelegt wird, und
(d) wobei die Vorrichtung weiter einen apikalen Einsatz (407) für Kulturzellen und einen O-Ring (409) umfasst, der zum Abdichten um den apikalen Einsatz in einem Zustand, in dem der apikale Einsatz in einem Näpfchen der fluidischen Platte eingehängt ist, eingerichtet ist.

2. Vorrichtung nach Anspruch 1, wobei die abnehmbare zweite Platte und die Sperrmembran eine oder mehrere Pumpeinheiten mit mindestens einem Abschnitt der Fluidpfade der ersten Platte bilden.

3. Vorrichtung nach Anspruch 2, wobei jede der Pumpeinheiten eine Pumpkammer in der Mitte und mindestens zwei Ventilkammern umfasst, die dazu ausgelegt sind, fluidisch mit der Pumpkammer verbunden zu sein, wenn die Sperrmembran gebogen ist.

4. Vorrichtung nach einem der Ansprüche 1 bis 3, wobei die Zellen aus der Gruppe ausgewählt sind, die aus Bakterien, Pilzen, Hefe und Kombinationen davon besteht.

5. Vorrichtung nach einem der Ansprüche 1 bis 4, wobei die Zellen von einem Mikrobiom stammen, das in dem Magen-Darmtrakt, der Mundhöhle, Nasenhöhle, Vagina oder Kombinationen davon anwesend ist.

6. Vorrichtung nach einem der Ansprüche 1 bis 5 in einem System, das mehr als eine Vorrichtung umfasst, wobei jede Vorrichtung ein oder mehrere äquivalente Organe schafft.

7. Vorrichtung nach einem der Ansprüche 1 bis 6, wobei der apikale Einsatz eine Einlassstelle (375) und eine Auslassstelle (377) beinhaltet.

8. Vorrichtung nach einem der Ansprüche 1 bis 7, wobei der apikale Einsatz dazu ausgelegt ist, Fluid und die Zellen zu mindestens einem oder mehr der zwei oder mehr Näpfchen bereitzustellen, und das Fluid und die Zellen aus dem mindestens einen der zwei oder mehr Näpfchen zu entfernen.

9. Vorrichtung nach einem der Ansprüche 1 bis 7, wobei der apikale Einsatz weiter einen Einlasssauerstoffsensor und/oder einen Auslasssauerstoffsensor umfasst.

10. Meso- und/oder mikrofluidisches System mit Regelkreis-Feedbacksteuerung, das umfasst:

a) mindestens einen offenen Behälter (4001) für Fluid;
b) die fluidische Multiwell-Vorrichtung nach einem der Ansprüche 1 bis 9, wobei mindestens ein meso- oder mikrofluidischer Kanal der fluidischen Multiwell-Vorrichtung mit dem Behälter in Kommunikation steht; und
c) einen automatisierbaren Sensor (4007), um eine Fluidhöhe in dem Behälter zu erfassen und ein entsprechendes Signal als Feedback bereitzustellen;

wobei das Signal entsprechend dynamischen Änderungen der Fluidhöhe in dem Behälter im Vergleich zu einer Referenzeingabe eine dynamische Flussrate des Fluids durch den meso- oder mikrofluidischen Kanal angibt.

11. Meso- und/oder mikrofluidische Vorrichtung, die umfasst:

a) eine schwerkraftbetriebene Pumpe, die mindestens einen schwerkraftdominierten Fluidversorgungsbehälter (4001) umfasst;
b) einen Sensor (4007), um einen Fluidpegel in dem Fluidversorgungbehälter zu erfassen und ein entsprechendes Signal als Feedback bereitzustellen, und fähig, den Fluidpegel in dem Versorgungbehälter einzustellen, um ein Regelkreis-Feedback-Steuersystem mit der schwerkraftbetriebenen Pumpe zu bilden;
c) die fluidische Multiwell-Vorrichtung nach einem der Ansprüche 1 bis 9, wodurch
mindestens ein meso- und/oder mikrofluidischer Kanal der fluidischen Multiwell-Vorrichtung mit dem Fluidversorgungsbehälter in Kommunikation steht;
wobei der Fluidpegel in dem Versorgungsbehälter einen schwerkraftdominierten Fluss durch den meso- und/oder mikrofluidischen Kanal gemäß einer Referenzeingabe treibt.

12. Fluidische Vorrichtung nach Anspruch 11, die eine Vielzahl schwerkraftbetriebener Pumpen und ein Regelkreis-Feedback-Steuersystem umfasst, wobei der Fluss durch den meso- und/oder mikrofluidischen Kanal eine Flussrate aufweist, die von Änderungen des hydrostatischen Drucks in dem Fluidversorgungsbehälter abgekoppelt ist.

13. Fluidische Vorrichtung nach Anspruch 11, wobei die schwerkraftgetriebene Pumpe zwei schwerkraftdominierte Fluidversorgungsbehälter (4001, 4003) in fluidischer Kommunikation durch den mindestens einen meso- und/oder mikrofluidischen Kanal umfasst, und Änderungen der Fluidhöhen in den zwei Fluidversorgungsbehältern bidirektionale konstante und/oder dynamische Flüsse durch den meso- und/oder mikrofluidischen Kanal treiben.

14. Fluidische Vorrichtung nach Anspruch 11, die weiter eine Rückführungsverbindung umfasst, wobei das Fluid, das aus dem meso- und/oder mikrofluidischen Kanal austritt, zu der schwerkraftgetriebenen Pumpe zurückgeführt wird.

15. Fluidische Vorrichtung nach einem der Ansprüche 10 bis 14, wobei der Fluidversorgungsbehälter eine definierte Hohlstruktur mit einem konstanten Querschnitt für mindestens die Tiefe, die von dem Sensor erfassbar ist, umfasst.

16. Fluidische Vorrichtung nach einem der Ansprüche 10 bis 15, wobei der Sensor ein kontaktloses kapazitives Fluidabtastsystem umfasst.

17. Fluidische Vorrichtung nach einem der Ansprüche 10 bis 16, wobei der Sensor mit mindestens einer Rechenverarbeitungseinheit und/oder einer Mikrocontrollereinheit (4010), die Fluid lenkt, das zu dem Behälter basierend auf dem Feedback und/oder einer Referenzeingabe geliefert oder extrahiert werden soll, verbunden ist.

**Revendications**

1. Dispositif multipuits fluidique avec un système de pompage embarqué comprenant :

(a) une première plaque (100), la première plaque comprenant :

deux puits (103) ou plus comprenant
un espace tridimensionnel (1103) dans chaque puits défini par une surface de fond et une paroi circonférentielle ; et
une entrée et une sortie dans chaque puits ;
un conduit d'évacuation (121) positionné entre les au moins deux puits (103i, 103j), présentant des géométries permettant une connectivité de fluide unidirectionnelle depuis le dessus de la surface de fond d'un premier puits jusqu'à un second puits ;
un réseau de voies de fluide (101) fournissant une connectivité de fluide entre au moins deux des puits à travers l'entrée et la sortie de chacun des deux puits ;

(b) une seconde plaque détachable (200), la seconde plaque détachable comprenant :

une pluralité de canaux internes, chacun avec une ouverture d'entrée (210) et une ouverture de sortie (210) sur des côtés opposés de la seconde plaque,
et un ou plusieurs trous sur la surface de la seconde plaque en liaison avec chacun des canaux internes ; et

(c) une membrane barrière (300) positionnée entre les voies de fluide de la première plaque et les un ou plusieurs trous sur la surface de la seconde plaque, dans lequel la membrane barrière est au moins partiellement souple, de sorte que l'application d'une pression sur les canaux internes de la seconde plaque amène la membrane à se déplacer, de ce fait en obstruant ou en dégageant une portion des voies de fluide de la première plaque, et
(d) le dispositif comprenant en outre un insert apical (407) pour cultiver des cellules et un joint torique (409) agencé pour sceller autour de l'insert apical dans un état dans lequel l'insert apical est suspendu dans un puits de la plaque fluidique.

2. Dispositif selon la revendication 1 dans lequel la seconde plaque détachable et la membrane barrière forment une ou plusieurs unités de pompe avec au moins une portion des voies de fluide de la première plaque.

3. Dispositif selon la revendication 2, dans lequel chacune des unités de pompe comprend une chambre de pompe au centre et au moins deux chambres de vanne configurées pour être reliées fluidiquement à la chambre de pompe lorsque la membrane barrière est fléchie.

4. Dispositif selon l'une quelconque des revendications 1 à 3 dans lequel les cellules sont sélectionnées dans le groupe consistant en des bactéries, des champignons, des levures et des combinaisons de ceux-ci.

5. Dispositif selon l'une quelconque des revendications 1 à 4, dans lequel les cellules proviennent d'un microbiome présent dans le tube digestif, la cavité orale, la cavité nasale, le vagin ou une combinaison de ceux-ci.

6. Dispositif selon l'une quelconque des revendications 1 à 5 dans un système comprenant plus d'un dispositif, chaque dispositif créant un ou plusieurs équivalents d'organe.

7. Dispositif selon l'une quelconque des revendications 1 à 6, dans lequel l'insert apical inclut un point d'entrée (375) et un point de sortie (377).

8. Dispositif selon l'une quelconque des revendications 1 à 7, dans lequel l'insert apical est configuré pour fournir un fluide et les cellules à au moins l'un parmi les deux puits ou plus et pour retirer le fluide et les cellules de l'au moins un parmi les deux puits ou plus.

9. Dispositif selon l'une quelconque des revendications 1 à 7, dans lequel l'insert apical comprend en outre un capteur d'oxygène d'entrée et/ou un capteur d'oxygène de sortie.

10. Système mésofluidique et/ou microfluidique avec commande à rétroaction en boucle fermée, comprenant :

a) au moins un réservoir ouvert (4001) pour un fluide ;
b) le dispositif multipuits fluidique selon l'une quelconque des revendications 1 à 9, selon lequel au moins un canal mésofluidique ou microfluidique dudit dispositif multipuits fluidique est en communication avec le réservoir ; et
c) un capteur automatisable (4007) pour détecter une hauteur de fluide dans le réservoir et fournir un signal correspondant en tant que rétroaction ;

dans lequel le signal correspondant à des changements dynamiques de la hauteur de fluide dans le réservoir par rapport à une entrée de référence indique un débit dynamique de fluide à travers le canal mésofluidique ou microfluidique.

11. Dispositif mésofluidique et/ou microfluidique, comprenant :

a) une pompe entraînée par la gravité comprenant au moins un réservoir d'alimentation en fluide dominé par la gravité (4001) ;
b) un capteur (4007) pour détecter un niveau de fluide dans le réservoir d'alimentation en fluide et fournir un signal correspondant en tant que rétroaction et capable d'ajuster un niveau de fluide dans le réservoir d'alimentation pour former un système de commande à rétroaction en boucle fermée avec la pompe entraînée par la gravité ;

c) le dispositif multipuits fluidique selon l'une quelconque des revendications 1 à 9, selon lequel au moins un canal mésofluidique ou microfluidique dudit dispositif multipuits fluidique est en communication avec le réservoir d'alimentation en fluide ;

dans lequel le niveau de fluide dans le réservoir d'alimentation entraîne un écoulement entraîné par la gravité à travers le canal mésofluidique et/ou microfluidique en fonction d'une entrée de référence.

12. Dispositif fluidique selon la revendication 11, comprenant une pluralité de pompes entraînées par la gravité et un système de commande à rétroaction en boucle fermée, dans lequel l'écoulement à travers le canal mésofluidique et/ou microfluidique présente un débit découplé de changements de pression hydrostatique dans le réservoir d'alimentation en fluide.

13. Dispositif fluidique selon la revendication 11, dans lequel la pompe entraînée par la gravité comprend deux réservoirs d'alimentation en fluide dominés par la gravité (4001, 4003) en communication fluidique à travers l'au moins un canal mésofluidique et/ou microfluidique, et des changements de hauteurs de fluide dans les deux réservoirs d'alimentation en fluide entraînent des écoulements constants et/ou dynamiques bidirectionnels à travers le canal mésofluidique et/ou microfluidique.

14. Dispositif fluidique selon la revendication 11, comprenant en outre une liaison de recirculation, dans lequel un fluide sortant du canal mésofluidique et/ou microfluidique est recirculé dans la pompe entraînée par la gravité.

15. Dispositif fluidique selon l'une quelconque des revendications 10 à 14, dans lequel le réservoir d'alimentation en fluide comprend une structure creuse définie avec une coupe transversale constante pour au moins la profondeur détectable par le capteur.

16. Dispositif fluidique selon l'une quelconque des revendications 10 à 15, dans lequel le capteur comprend un système de détection de fluide capacitif sans contact.

17. Dispositif fluidique selon l'une quelconque des revendications 10 à 16, dans lequel le capteur est relié à au moins une unité de traitement informatique et/ou une unité de microcontrôleur (4010) dirigeant un fluide à alimenter dans ou à extraire du réservoir sur la base de la rétroaction et/ou d'une entrée de référence.

FIG. 1

**FIG. 2**

**FIG. 3A**

**FIG. 3B**

**FIG. 4**

**FIG. 5**

**FIG. 6**

**FIG. 7**

**FIG. 8**

**FIG. 9**

*FIG. 11*

*FIG. 10*

**FIG. 12**

111a    103i    111b    121    103j

125

124    123

103i    111b    **FIG. 13**

121

130

131

103j

**FIG. 14**

Spill out

1102

1103

Recirculation    Flow in

**FIG. 15**

Fluid film

ΔH₁    H₂    — Fluid Profile    ΔH₃

H₁    H₃

SOURCE    SINK

*FIG. 16A*

Fluid Film Breaks!    Conduit drying and / or Syphoning

Sink drying

SOURCE    SINK

*FIG. 16B*

Fluid Meniscus becomes stable in entry geometry blocking flow

Dry Conduit (no fluid film)    Sink drying until empty

Fluid Accumulates

SOURCE    SINK

*FIG. 16C*

TIME

Entry    — Fluid Profile

ΔH₁    H₂    ΔH₃

H₁    H₃

SOURCE    SINK

*FIG. 17*

FIG.18

FIG. 19

FIG. 20

*FIG. 21*

*FIG. 22A*

*FIG. 22B*

*FIG. 22C*

**FIG. 23**

**FIG. 24**

**FIG. 25**

Rounded

Knife Edge

V-cut

Inlet

*FIG. 26A*

*FIG. 27A*

*FIG. 28A*

Diagonal

*FIG. 26B*

*FIG. 27B*

*FIG. 28B*

Section

*FIG. 26C*

*FIG. 27C*

*FIG. 28C*

Outlet

*FIG. 26D*

*FIG. 27D*

*FIG. 28D*

125

*FIG. 29*

FIG. 30A

FIG. 30B

FIG. 30C

FIG. 31

*FIG. 32*

*FIG. 33*

*FIG. 34*

**FIG. 35**

*FIG. 36*

*FIG. 37A*

*FIG. 37B*

221    222    223    201    300    101

*FIG. 38*

A1    A2    A3    A4

Set-1

Set-2

A1    A2    A3    A4

FIG. 39

FIG. 40

FIG. 41

Compression force

d$_2$

253   252a   251a   251b   252b

*FIG. 42*

350

351   252c   252d

*FIG. 43*

**FIG. 44A**

**FIG. 44B**

**FIG. 45**

**FIG. 46**

FIG. 47

FIG. 48

4047a

4046a    4046b    4048

z
y
x

4044

4045

**FIG. 49**

4054

4051

4052

CAPDAC (-)

$C_{REF}$ (-)

CDC

DATA OUT

$C_{SENSE}$ (+)

CAPDAC (+)

4049    4050

EXC

4053

**FIG. 50**

4062

4058

4060

4055

4056

4057

4059

4061

**FIG. 51**

4066

4065a

4065b

4064

4063

4067

4068

**FIG. 52**

**Validation of capacitance sensing output**

**FIG. 53A**

**Basic fluid-height challenge**

**FIG. 53B**

**Gravity Drive Flow Rate Validation**

**FIG. 53C**

**Capacitance dependance on Fluid Type**

**FIG. 54A**

**FIG. 54B**

**FIG. 54C**

**FIG. 55A**

**Fluid height tracking of system with uncalibrated open-loop pneumatic pumps**

$dH/dt \approx -4.01\,um/s$
$dV/dt \approx -0.079\,uL/s$

**FIG. 55B**

**Input and output behaviour of uncalibrated open-loop pneumatic pumps**

1
2

**FIG. 55C**

**A)**

**Closed-loop control performance to constant fluid-level**
(Setpoint = 30000µm)

**FIG. 56A**

**Closed-loop control performance to dynamic fluid-level target**

**FIG. 56B**

FIG. 57A

FIG. 57B

FIG. 57C

FIG. 57D

EP 3 600 668 B1

FIG. 58A

FIG. 58B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2005260745 A1
- US 2010230613 A1
- US 2013068310 A1
- US 6197575 B, Griffith
- US 8318479 B, Inman
- US 5042299 A
- US 62556595
- US 7129714 B
- US 3963380 A
- US 3803424 A
- US 5482447 A
- US 6213739 B
- US 20140079571 A
- US 20160051935 A
- US 62556595 B
- US 20170227525 A
- US 7704728 B

### Non-patent literature cited in the description

- **HUH D et al.** *Lab Chip,* 2012, vol. 12 (12), 2156-2164
- **SUNG JH et al.** *Lab Chip,* 2013, vol. 13 (7), 1201-1212
- **WIKSWO JP et al.** *Exp Biol Med (Maywood),* 2014, vol. 239 (9), 1061-1072
- **LIVINGSTON CA et al.** *Computational and Structural Biotechnology Journal,* 2016, vol. 14, 207-210
- **YU J et al.** *Drug Discovery Today,* 2014, vol. 19 (10), 1587-1594
- **ZHU L et al.** *Lab Chip,* 2016, vol. 16 (20), 3898-3908
- **ROTH A et al.** *Adv Drug Deliver Rev,* 2014, vol. 69-70, 179-189
- **HUEBSCH N et al.** *Scientific Reports,* 2016, vol. 6, 24726
- **DOMANSKY K et al.** *Lab Chip,* 2010, vol. 10 (1), 51-58
- **OLEAGA C et al.** *Sci Rep,* 2016, vol. 6, 20030
- **ESCH MB et al.** *Lab Chip,* 2014, vol. 14 (16), 3081-3092
- **MASCHMEYER I et al.** *Lab Chip,* 2015, vol. 15 (12), 2688-2699
- **MATERNE EM et al.** *J Biotechnol,* 2015, vol. 205, 36-46
- **LOSKILL P et al.** *Plos One,* 2015, vol. 10 (10), e0139587
- **KUBINYI H.** *Nat Rev Drug Discov,* 2003, vol. 2 (8), 665-668
- **COOK D et al.** *Nat Rev Drug Discov,* 2014, vol. 13 (6), 419-431
- **DENAYER T et al.** *New Horizons in Translational Medicine,* 2014, vol. 2 (1), 5-11
- **SWEENEY LM et al.** *Toxicol. Vitr.,* 1995, vol. 9, 307-316
- **SUNG JH et al.** *Lab Chip,* 2009, vol. 9, 1385
- **SUNG JH et al.** *Lab Chip,* 2010, vol. 10, 446-455
- **ESCH MB et al.** *Lab Chip,* 2014, vol. 14, 3081
- **ANNA SL.** *Annu. Rev. Fluid Mech.,* 2016, vol. 48, 285-309
- **HALLDORSSON S et al.** *Biosens. Bioelectron.,* 2015, vol. 63, 218-231
- **WIJS et al.** *Separations: Materials, Devices and Processes,* 2016, vol. 62 (12), 4453-4465
- **MATERNE EM et al.** *J. Vis. Exp.,* 2015, 1-11
- **OLEAGA C. et al.** *Sci. Rep.,* 2016, vol. 6, 20030
- **BERTHIER J et al.** *AIMS Biophysics,* 2014, vol. 1 (1), 31-48
- **BRAKKE et al.** *Exp Math,* 1992, vol. 1 (2), 141-165
- **POWERS M J et al.** *Biotechnol Bioeng,* 2002, vol. 78, 257-69
- **DOMANSKY K et al.** *Lab on a Chip,* 2010, vol. 10, 51-58
- **EBRAHIMKHANI MR et al.** *Advanced Drug Delivery Reviews,* April 2014, 132-57
- **YATES C et al.** *Adv. Cancer Res.,* 2007, vol. 97, 225-246
- **WALKER I et al.** *Journal of Micromechanics and Microengineering,* 2007, vol. 17 (5), 891
- **BAXTER, L.K.** Capacitive Sensors: Design and Applications. IEEE Press Series on Electronics Technology, 1996, vol. 1
- **LI, X.B.** *IEEE Sensors Journal,* 2006, vol. 6 (2), 434-440
- **TOEPKE MW et al.** *Lab Chip,* 2006, vol. 6, 1484-1486
- **NG SF et al.** *Pharmaceutics,* 2010, vol. 2, 209-223

- **LOZUPONE et al.** *Nature,* 2012, vol. 489 (7415), 220-230
- **HAKANSSON ; MOLIN.** *Nutrients,* 2011, vol. 3 (6), 637-682
- Microbiology of the Gastrointestinal Tract. **GORBACH.** Medical Microbiology. 1996
- **LINSKENS et al.** *Scand J Gastroenterol Suppl,* 2001, vol. 234, 29-40
- **LEY et al.** *Nature,* 21 December 2006, vol. 444 (7122), 1022-3
- **CANI et al.** *Pathol Biol (Paris),* July 2008, vol. 56 (5), 305-9
- **IVANOV et al.** *Cell,* 2009, vol. 139 (3), 485-98
- **HOLLAND et al.** *Br J Dermatol.,* 1977, vol. 96 (6), 623-6
- **THOMSEN et al.** *Arch. Dermatol.,* 1980, vol. 116, 1031-1034
- **TILL et al.** *Br. J. Dermatol.,* 2000, vol. 142, 885-892
- **PAULINO et al.** *J. Clin. Microbiol.,* 2006, vol. 44, 2933-2941
- **ROTH ; JAMES.** *Annu. Rev. Microbiol.,* 1988, vol. 42, 441-464
- **HAY et al.** *Br. Med. J.,* 1994, vol. 308, 295-298
- **CANI et al.** *Molecular Metabolism,* 2016, vol. 5, 743-752
- **SAMPSON et al.** *Cell,* 2016, vol. 167, 1469-1480
- **BRAVO et al.** *Proc Natl Acad Sci U S A.,* 2011, vol. 108 (38), 16050-5
- **LONG T et al.** *Drug Metab Dispos,* 2016, vol. 44, 1940-1948
- **CHITCHOLTAN et al.** *Exp Cell Research,* 2013
- **MARSHALL JC.** Host Defense Dysfunction in Trauma, Shock and Sepsis: Mechanisms and Therapeutic Approaches. Springer, 1993, 243-252
- **MORGAN ET.** *Drug Metab Dispos,* 2001, vol. 29 (3), 207-212
- **DENG X et al.** *Pharmacological Reviews,* 2009, vol. 61 (3), 262-282
- **LONG TJ et al.** *Drug Metabolism and Disposition,* 2016, vol. 44 (12), 1940-1948
- **MESTAS J et al.** *The Journal of Immunology,* 2004, vol. 172 (5), 2731-2738
- **GIESE C et al.** *Adv Drug Deliver Rev,* 2014, vol. 69, 103-122
- **SEOK J et al.** *Proc Natl Acad Sci U S A,* 2013, vol. 110 (9), 3507-3512
- **FINK MP.** *Virulence,* 2014, vol. 5 (1), 143-153
- **ROWLANDS BJ et al.** *British Medical Bulletin,* 1999, vol. 55 (1), 196-211
- **NESSELER N et al.** *Crit Care,* 2012, vol. 16 (5), 235
- **PIERRAKOS C et al.** *Crit Care,* 2010, vol. 14 (1), R15
- **LIASKOU E et al.** *Mediators Inflamm,* 2012, vol. 2012, 949157
- **BROWN RP et al.** *Toxicol Ind Health,* 1997, vol. 13 (4), 407-484
- **SARKAR U et al.** *Drug Metabolism and Disposition,* 2015, vol. 43 (7), 1091-1099
- **PILLAI VC et al.** *J Pharm Biomed Anal,* 2013, vol. 74, 126-132
- **KIM D et al.** *Genome Biol,* 2013, vol. 14 (4), R36
- **ANDERS S et al.** *Bioinformatics,* 2015, vol. 31 (2), 166-169
- **LOVE MI et al.** *Genome Biol,* 2014, vol. 15 (12), 550
- **YOUNG MD et al.** *Genome Biol,* 2010, vol. 11 (2), R14
- **SUBRAMANIAN A et al.** *Proceedings of the National Academy of Sciences,* 2005, vol. 102 (43), 15545-15550
- **DING L et al.** *Acta Pharm Sin B,* 2015, vol. 5 (2), 135-144
- **MIDWOUD PM et al.** *Lab Chip,* 2010, vol. 10 (20), 2778-2786
- **LEBLOND F et al.** *Am J Physiol Gastrointest Liver Physiol,* 2009, vol. 296 (4), G805-815
- **COSGROVE BD et al.** *Toxicology and Applied Pharmacology,* 2009, vol. 237 (3), 317-330
- **GUO S et al.** *Am J Pathol,* 2013, vol. 182 (2), 375-387
- **YAMAOKA T et al.** *Proc Natl Acad Sci U S A,* 2008, vol. 105 (33), 11772-11777
- **ZHANG Y et al.** *Lab Invest,* 2012, vol. 92 (3), 437-450
- **FREY MR et al.** *Gastroenterology,* 2009, vol. 136 (1), 217-226
- **JACOB A et al.** *Int J Clin Exp Med,* 2009, vol. 2 (3), 203-211
- **EBRAHIMKHANI MR et al.** *Adv Drug Deliv Rev,* 2014, vol. 69-70, 132-157
- **HUANG SM et al.** *Clin Pharmacol Ther,* 2010, vol. 87 (4), 497-503
- **KHOVIDHUNKIT W et al.** *J Lipid Res,* 2004, vol. 45 (7), 1169-1196
- **VARGA T et al.** *Biochim Biophys Acta,* 2011, vol. 1812 (8), 1007-1022
- **KIM TH et al.** *Febs J,* 2011, vol. 278 (13), 2307-2317
- **GROOM JR et al.** *Immunol Cell Biol,* 2011, vol. 89 (2), 207-215
- **SINGH UP et al.** *Endocr Metab Immune Disord Drug Targets,* 2007, vol. 7 (2), 111-123
- **NGUYEN TV et al.** *Drug Metab Dispos,* 2015, vol. 43 (5), 774-785
- **YERUVA S et al.** *Int J Colorectal Dis,* 2008, vol. 23 (3), 305-317
- **DWINELL MB et al.** *Gastroenterology,* 2001, vol. 120 (1), 49-59
- **HASSANSHAHI G et al.** *Iran J Allergy Asthma Immunol,* 2007, vol. 6 (3), 115-121

- **DANESE S et al.** *Gut,* 2004, vol. 53 (7), 1035-1043
- **DAVIES NM et al.** *Clin. Pharmacokinet.,* 1997, vol. 33, 184-213
- **WALKER, C.S.** Capacitance, inductance, and crosstalk analysis. *Artech House,* 1990
- **INMAN et al.** *Journal of Micromechanics and Micro-engineering,* 2007, vol. 17 (5), 891
- **DOMANSKY et al.** *Lab on a chip,* 2010, vol. 10 (1), 51-58
- **CHANG et al.** *Biomicrofluidics,* 2014, vol. 8 (4), 044116
- **KIM et al.** *Microtechnology for Cell Manipulation and Sorting,* 2017, 175-192
- **LEE et al.** *Biotechnology Progress,* 2012, vol. 28 (6), 1466-1471
- **STILLINGER et al.** *Journal of Fluid Mechanics,* 1983, vol. 131, 73-89
- **ALI ; FOSS.** *Experiments in fluids,* 1995, vol. 19 (4), 250-254